# EUROPEAN PATENT SPECIFICATION

(11) **EP 0 757 677 B1**
(45) Date of publication and mention of the grant of the patent: **18.06.2003**
(21) Application number: 95916449.2
(22) Date of filing: 25.04.1995
(51) Int. Cl.: C07D 233/84, A61K 31/415, A61K 31/41, C07D 233/88, C07D 233/90, C07D 249/12, C07D 249/14, C07D 401/06, C07D 401/12, C07D 403/04, C07D 403/10

(54) **BENZOCYCLOHEXYLIMIDAZOLETHIONE DERIVATIVES**
BENZOCYCLOHEXYLIMIDAZOLTHION-DERIVATE
DERIVES DES BENZOCYCLOHEXYLIMIDAZOLETHIONES

(30) Priority: 26.04.1994 US 233835; 26.04.1994 US 233655; 17.03.1995 US 403209
(43) Date of publication of application: 12.02.1997
(73) Proprietor: Syntex (U.S.A.) LLC, Palo Alto, California 94304 (US)
(72) Inventor: MARTINEZ, Gregory, R., Mountain View, CA 94043-2184 (US); REPKE, David, B., Milpitas, CA 95305 (US); TEITELBAUM, Philip, J., Boulder Creek, CA 95006 (US); WALKER, Keith, A., M., Los Altos Hills, CA 94022 (US); GOODING, Owen, W., Los Gatos, CA 95030 (US); WHITING, Roger, L., Los Altos, CA 94022 (US); BANSAL, Rekha, P., Santa Clara, CA 95050 (US); MUEHLDORF, Alexander, V., Sunnyvale, CA 94087 (US); O'YANG, Counde, Sunnyvale, CA 94087 (US)
(74) Representative: Wächter, Dieter Ernst, Dr.
(86) International application number: US9504783
(87) International publication number: WO95029165

(56) References cited:
- EP-A- 0 207 563
- US-A- 3 547 942
- CHEMICAL ABSTRACTS, vol. 75, no. 11, 13 September 1971, Columbus, Ohio, US; abstract no. 76683, H. SCHUBERT ET AL. 'Synthesis and ring closure reactions of imidazole 4,5-dialdehydes' page 457 ;column 1 ; & Z. CHEM., vol.11, no.5, 1971 pages 175 - 176
- CHEMICAL ABSTRACTS, vol. 110, no. 5, 30 January 1989, Columbus, Ohio, US; abstract no. 38996g, M.F.L. DE BRUYN ET AL. 'Preparation of 1H-imidazole-5-carboxylic acid derivatives as herbicides.' page 546 ;column 1 ; & EP,A,0 277 384 (JANSSEN PHARMACEUTICA N.V.) 10 August 1988
- JOURNAL OF MEDICINAL CHEMISTRY, vol.29, no.12, December 1986, WASHINGTON US pages 2465 - 2472 L.I. KRUSE ET AL. 'Multisubstrate Inhibitors of Dopamine beta-Hydroxylase. 1. Some 1-Phenyl and 1-Phenyl-Bridged Derivatives of Imidazole-2-thione'
- JOURNAL OF MEDICINAL CHEMISTRY, vol.30, no.3, March 1987, WASHINGTON US pages 486 - 494 L.I. KRUSE ET AL. 'Multisubstrate Inhibitors of Dopamine beta-Hydroxylase. 2. Structure-Activity Relationship at the Phenethylamine Binding Site'
- JOURNAL OF MEDICINAL CHEMISTRY, vol.33, no.2, February 1990, WASHINGTON US pages 781 - 789 L.I. KRUSE ET AL. 'Some Benzyl-Substituted Imidazoles, Triazoles, Tetrazoles, Pyridinethiones, and Structural Relatives as Multisubstrate Inhibitors of Dopamine beta-Hydroxylase. 4. Structure-Activity Relationships at the Copper Binding Site'
- JOURNAL OF MEDICINAL CHEMISTRY, vol.32, no.4, April 1989, WASHINGTON US pages 779 - 783 L. BJÖRK ET AL. 'Resolved N,N-Dialkylated 2-Amino-8-hydroxytetralins: Stereoselective Interactions with 5-HT1A Receptors in the Brain'
- JOURNAL OF MEDICINAL CHEMISTRY, vol.33, no.6, June 1990, WASHINGTON US pages 1541 - 1544 S.-E. HILLVER ET AL. '(S)-5-Fluoro-8-hydro xy-2-(dipropylamino)tetralin: A Putative 5-HT1A-Receptor Antagonist'
- JOURNAL OF MEDICINAL CHEMISTRY, vol.36, no.17, 20 August 1993, WASHINGTON US pages 2542 - 2551 S.D. WYRICK ET AL. 'Synthesis and Pharmacological Evaluation of 1-Phenyl-3-a mino1,2,3,4-tetrahydronaphthalenes as Ligands for a Novel Receptor with sigma-like Neuromodulatory Activity'
- CHEMISTRY LETTERS, no.2, February 1984, TOKYO JP pages 239 - 242 M. KAWASAKI ET AL. 'Asymmetric Reduction of Prochiral Cyclic Ketones with Lithium Aluminium Hydride Partially Decomposed by (1R,2S)-(-)-N-Methylephedrine and 2-Alyklaminopyridine'

## Description

### BACKGROUND OF THE INVENTION

### Field of the Invention:

This invention relates to novel benzocyclohexylimidazolethione dopamine β-hydroxylase inhibitors and the methods of using and preparing such inhibitors.

### Description of the Field:

Dopamine is a catecholamine neurotransmitter found predominately, along with specific dopaminergic receptors, in the central nervous system. Norepinephrine is a circulating catecholamine, which acts at discrete adrenergic receptors in peripheral systems. Dopamine β-hydroxylase (DBH) catalyzes the conversion of dopamine to norepinephrine and is found in both central and peripheral sympathetic neurons. Inhibition of DBH concurrently elevates dopamine levels by blocking its metabolism and reduces norepinephrine levels by blocking its synthesis. Thus, drugs which inhibit DBH are useful for treating diseases associated with reduced dopamine levels (e.g., Parkinson's disease) and for treating diseases associated with elevated norepinephrine levels (e.g., hypertension, congestive heart failure, etc.). Fusaric acid, a DBH inhibitor, decreases the tremors and other abnormalities associated with Parkinson's disease. Fusaric acid also reduces blood pressure in hypertensive patients; however, release of norepinephrine from the adrenal gland and a resultant tachycardia is also observed. Other more selective DBH inhibitors are known but often possess disadvantageous effects.

EP-A-0 207 563, US-A-3 547 942, Chem. Abs. 75 (1971), 766838 RN 33457-39-5 and Chem. Abs. 110 (1989), 389969 & RN 118 240-10-1 disclose certain α-substituted tetrahydronaphthalene derivatives, mainly as weed controll agents or fungizidal agents.

J. Med. Chem. 29 (1986), 2465-2472, J. Med. Chem. 30 (1987), 486-494 and J. Med. Chem. 33 (1990), 781-789 disclose certain dopamine β-hydroxylase inhibitors.

The compounds consist of an optionally substituted phenyl residue and an imidazole-2-thione functional group which are connected via a flexible spacer (an optionally substituted alkyl chain of variable length).

J. Med. Chem. 32 (1989), 779-783, J. Med. Chem. 33 (1990) 1541-1575 and J. Med. Chem. 36 (1993), 2542-2551 disclose certain 2-aminotetralones which show interactions with the 5-HT₁₄ receptor or act as a ligand for a novel receptor with α-like neuromodulatory activity.

Chem. Lett. 2 (1984), 239-242 discloses a chiral hydride, which can reduce prochiral cyclic ketones such as β-tetralones.

### SUMMARY OF THE INVENTION

The present invention relates to a Compounds of Formula I, II or III 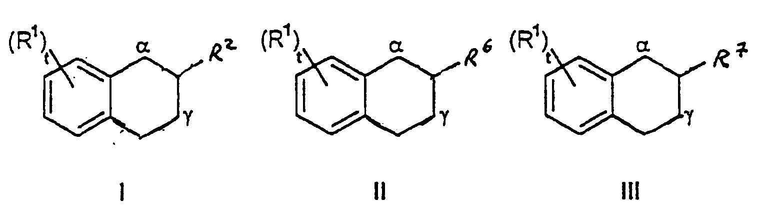 in which:
**t** is 0, 1, 2 or 3;
**R**^{**1**} is independently halo, hydroxy or (C₁₋₄)alkyloxy; and
**R**^{**2**} is a group of Formula (a) 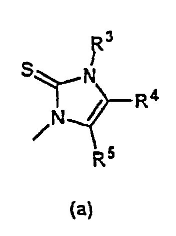 in which:
   **R**^{**3**} is hydro, **R**^{**4**} is hydro, and **R**^{**5**} is NHR¹⁰; or **R**^{**3**} is -(CH₂)_{q}R⁹, **R**^{**4**} is hydro and **R**^{**5**} is hydro; or **R**^{**3**} is -(CH₂)_{q}R⁹, **R**^{**4**} is hydro and **R**^{**5**} is NHR¹⁰; or
   **R**^{**3**} is -NHR¹⁰ and **R**^{**4**} and **R**^{**5**} are each hydro; or.
   **R**^{**3**} is hydro or -(CH₂)_{q}R⁹, **R**^{**4**} is (C₁₋₄)alkyl, di(C₁₋₄)alkylaminomethyl, piperidin-1-ylmethyl, morpholin-4-ylmethyl, formyl, 1-hydroxy(C₁₋₄)alkyl or -CH₂NHR¹³ and **R**^{**5**} is hydro; or
   **R**^{**3**} is hydro or -(CH₂)_{q}R⁹, **R**^{**4**} is hydro, (C₁₋₄)alkyl or -C(O)R¹⁴ and **R**^{**5**} is cyano, hydroxymethyl, 1H-tetrazol-5-yl, 4,5-dihydroimidazol-2-yl, pyrrolidin-1-ylmethyl, piperidin-1-ylmethyl, morpholin-4-ylmethyl, piperazin-1-ylmethyl, 4-(C₁₋₄)alkylpiperazin-1-ylmethyl, -C(O)R¹⁴, -C(NH)NR¹⁵R¹⁶ or -CH₂NR¹⁰R¹⁷; and
   **R**^{**3**} is hydro or -(CH₂)_{q}R⁹ and **R**^{**4**} and **R**^{**5**} are dependently di(C₁₋₄)alkylaminomethyl, piperidin-1-ylmethyl, morpholin-4-ylmethyl or hydroxymethyl;
**R**^{**6**} is a group of Formula (d): 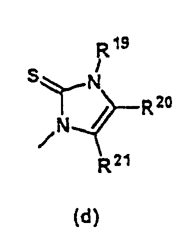 in which:
   **R**^{**19**} is hydro or -(CH₂)_{q}R⁹, **R**^{**20**} is hydro and **R**^{**21**} is -NR²⁵R²⁶; or
   **R**^{**19**} is -NR²⁵R²⁶, **R**^{**20**} and **R**^{**21**} are each hydro; or
   **R**^{**19**} is hydro or -(CH₂)_{q}R⁹, **R**^{**20**} is -CH₂NR²⁵R²⁶ and **R**^{**21**} is hydro; and
   **R**^{**19**} is hydro or -(CH₂)_{q}R⁹, **R**^{**20**} is hydro, (C₁₋₄)alkyl or -C(O)R¹⁴ and **R**^{**21**} is -CH₂NR²⁵R²⁶;
**R**^{**7**} is a group of Formula (g): 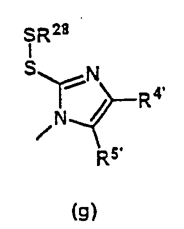 in which:
   **R**^{**4**}**'** is hydro and **R**^{**5**}**'** is -NHR¹⁰; or
   **R**^{**4**}**'** is (C₁₋₄)alkyl, di(C₁₋₄)alkylaminomethyl, piperidin-1-ylmethyl, morpholin-4-ylmethyl, 1-hydroxy(C₁₋₄)alkyl or -CH₂NHR¹³ and **R**^{**5**}**'** is hydro; or
   **R**^{**4**}**'** is hydro, (C₁₋₄)alkyl or -C(O)R¹⁴ and **R**^{**5**}**'** is hydroxymethyl, 1H-tetrazol-5-yl, 4,5-dihydroimidazol-2-yl, pyrrolidin-1-ylmethyl, piperidin-1-ylmethyl, morpholin-4-ylmethyl, piperazin-1-ylmethyl, 4-(C₁₋₄)alkylpiperazin-1-ylmethyl, -C(O)R¹⁴, -C(NH)NR¹⁵R¹⁶ or -CH₂NR¹⁰R¹⁷; or
   **R**^{**4**}**'** and **R**^{**5**}**'** are dependently di(C₁₋₄)alkylaminomethyl, piperidin-1-ylmethyl, morpholin-4-ylmethyl or hydroxymethyl;
      and
   **R**^{**28**} is (C₂₋₆)alkyl {which alkyl is further substituted by one to two substituents independently selected from -N(R²⁹)₂, -C(O)OR³⁰, -PO(OR³⁰)₂, -SO₃R³⁰, -SO₂NHR³⁰ and -OR³⁰};
**q** is 0, 1, 2, 3 or 4;
**R**^{**9**} is carboxy, (C₁₋₄)alkyloxycarbonyl, carbamoyl or a group selected from aryl and heteroaryl (which group is optionally further substituted with one to two substituents independently selected from hydroxy, (C₁₋₄)alkyloxy, cyano, 1H-tetrazo-5-yl, carboxy and (C₁₋₄)alkyloxycarbonyl);
**R**^{**10**} is hydro, (C₁₋₄)alkanoyl, trifluoro(C₁₋₄)alkanoyl, carbamoyl, (C₁₋₄)alkyloxycarbonyl, (C₁₋₄)alkylcarbamoyl, di(C₁₋₄)alkylcarbamoyl, amino(C₁₋₄)alkanoyl, (C₁₋₄)alkylamino(C₁₋₄)-alkanoyl, di(C₁₋₄)alkylamino(C₁₋₄)alkanoyl, a group selected from aroyl and heteroaroyl (which aroyl and heteroaroyl are optionally further substituted with one to two substituents independently selected from hydroxy, (C₁₋₄)alkyloxy, cyano, 1H-tetrazol-5-yl, carboxy and (C₁₋₄)alkyloxycarbonyl) or -C(NR¹¹)NHR¹²;
**R**^{**11**} and **R**^{**12**} are independently hydro, acetyl or tert-butoxycarbonyl;
**R**^{**13**} is hydro, (C₁₋₄)alkyl, (C₁₋₄)alkanoyl, trifluoro(C₁₋₄)alkanoyl, carbamoyl, (C₁₋₄)alkyloxycarbonyl, (C₁₋₄)alkylcarbamoyl, di(C₁₋₄)alkylcarbamoyl, amino(C₁₋₄)alkanoyl, (C₁₋₄)alkylamino(C₁₋₄)alkanoyl, di(C₁₋₄)alkylamino(C₁₋₄)alkanoyl, carboxy(C₁₋₄)alkyl, (C₁₋₄)alkyloxycarbonyl(C₁₋₄)alkyl, carbamoyl(C₁₋₄)alkyl, a group selected from aroyl, heteroaroyl, aryl(C₁₋₄)alkyl and heteroaryl(C₁₋₄)alkyl (which aroyl, heteroaroyl, aryl and heteroaryl are optionally further substituted with one to two substituents independently selected from hydroxy, (C₁₋₄)alkyloxy, cyano, 1H-tetrazol-5-yl, carboxy and (C₁₋₄)alkyloxycarbonyl) or -C(NR¹¹)NHR¹²);
**R**^{**14**} is amino, hydroxy, (C₁₋₄)alkyloxy, 2-(dimethylamino)ethylamino, 4-methylpiperazin-1-yl, 2-(dimethylamino)-ethylmercapto, 4-(methylsulfonylamino)anilino or 1H-tetrazol-5-ylamino;
**R**^{**15**} and **R**^{**16**} are independently hydro, (C₁₋₄)alkyl or trifluoro(C₁₋₄)alkyl;
**R**^{**17**} is hydro or (C₁₋₄)alkyl;
**R**^{**25**} is hydro or (C₁₋₄)alkyl;
**R**^{**26**} is L-alanyl, L-arginyl, L-asparaginyl, L-α-aspartyl; L-β-aspartyl, L-cysteinyl, L-glutaminyl, L-α-glutamyl, L-γ-glutamyl, N-(C₁₋₄)-alkanoyl-L-α-glutamyl, N-(C₁₋₄)alkanoyl-L-γ-glutamyl, glycyl, L-histidyl, L-isoleucyl, L-leucyl, L-lysyl, L-methionyl, L-ornithinyl, L-phenylalanyl, L-prolyl, L-seryl, L-threonyl, L-tryptophyl, L-tyrosyl, L-valyl, 1-amino-cyclopropylcarbonyl, 1-aminocyclobutylcarbonyl, 1-aminocyclo-pentylcarbonyl or 1-aminocyclohexylcarbonyl;
**R**^{**29**} is independently hydro, acetyl or trifluoroacetyl;
**R**^{**30**} is independently hydro or (C₁₋₅)alkyl;
**aryl** means an organic radical derived from an aromatic hydrocarbon containing 6 to 14 carbon atoms and includes monocyclic or condensed carbocyclic aromatic rings. The aryl residue is optionally further substituted with one to two substituents independently selected from halo and cyano;
**aroyl** means the radical -C(O)R, wherein R is aryl as defined above;
**heteroaryl** means an organic radical derived from an aromatic hydrocarbon containing 5 to 14 atoms, 1 to 5 of which are hetero atoms chosen from N, O, or S, and includes monocyclic, condensed heterocyclic and condensed carbocyclic and heterocyclic aromatic rings, is the heteroaryl residue is optionally further substituted with one to two substituents independently selected from halo and cyano;
**heteroaroyl** means the radical -C(O)R, wherein R is heteroaryl as defined above;
and the pharmaceutically acceptable salts, individual isomers, and mixtures of isomers thereof.

Preferred compounds are defined in the subclaims.

Another aspect of this invention is a pharmaceutical composition comprising a therapeutically effective amount of a compound of Formula I,II or III or an individual isomer, a mixture of isomers, or the pharmaceutically acceptable salt or salts thereof, in combination with one or more pharmaceutically acceptable excipients.

Another aspect of this invention is the use of a compound of formula I, II or III or of an individual isomer, mixture of isomers, or the pharmaceutically acceptable salt or salts thereof for the preparation of a medicament for treating a condition capable of amelioration by inhibition of dopamine β-hydroxylase in an animal in need thereof, namely for treating Parkinson's disease, hypertension or congestive heart failure.

Another aspect of this invention is the processes for preparing compounds of Formula I, II or III and is set forth in "Detailed Description of the Invention".

Another aspect of this invention is a compound of the formula: 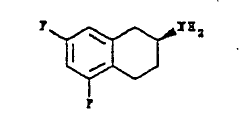 namely (*S*)-5,7-difluoro-1,2,3,4-tetrahydronaphthalen-2-ylamine.

Another aspect of this invention is the processes for preparing (*S*)-5,7-difluoro-1,2,3,4-tetrahydronaphthalen-2-ylamine (see "Detailed Description of the Invention").

### DETAILED DESCRIPTION OF THE INVENTION

### Definitions:

As used herein:
"C₁₋₄-Alkyl" means a straight or branched saturated hydrocarbon radical having from one to 4 carbon atoms (e.g., methyl, ethyl, prop-1-yl, prop-2-yl, but-1-yl, but-2-yl, 2-methylpropyl and 1,1-dimethylethyl).
"Trifluoro(C₁₋₄)alkyl" means a radical alkyl as defined above having from one to the number of carbon atoms designated wherein is contained a trifluoromethyl group (e.g., trifluoro(C₁₋₄)alkyl includes trifluoromethyl, 2,2,2-trifluoroethyl, 3,3,3-trifluoroprop-1-yl, 1,1,1-trifluoroprop-2-yl, etc.)
"C₁₋₄-Alkyloxy" means the radical -OR, wherein R is alkyl having from one to 4 carbon atoms designated (e.g., methoxy, ethoxy, prop-1-yloxy, prop-2-yloxy, but-1-yloxy, but-2-yloxy, 2-methylprop-1-yloxy and 2-methylprop-2-yloxy).
"Aryl", as in aryl or aryl(C₁₋₄)alkyl, means an organic radical derived from an aromatic hydrocarbon containing 6 to 14 carbon atoms and includes monocyclic or condensed carbocyclic aromatic rings (e.g., phenyl, naphthyl, anthracenyl, phenanthrenyl,) optionally further substituted with one to two substituents independently selected from halo and cyano.
"Aroyl" means the radical -C(O)R, wherein R is aryl as defined above (e.g., benzoyl,).
"Heteroaryl", as in heteroaryl or heteroaryl(C₁₋₄)alkyl, means an organic radical derived from an aromatic hydrocarbon containing 5 to 14 atoms, 1 to 5 of which are hetero atoms chosen from N, O, or S, and includes monocyclic, condensed heterocyclic and condensed carbocyclic and heterocyclic aromatic rings (e.g., thienyl, furyl, pyrrolyl, pyrimidinyl, isoxazolyl, oxazolyl, indolyl, benzo[b]thienyl, isobenzofuranyl, purinyl, isoquinolyl, pterdinyl, perimidinyl, imidazolyl, pyridyl, pyrazolyl, pyrazinyl) optionally further substituted with one to two substituents independently selected from halo and cyano.
"Heteroaroyl" means the radical -C(O)R, wherein R is heteroaryl as defined above (e.g., nicotinoyl, 2-furanoyl, picolinoyl).
"Carbamoyl", as in carbamoyl, (C₁₋₄)alkylcarbamoyl, di(C₁₋₄)alkylcarbamoyl or carbamoyl(C₁₋₄)alkyl, means aminocarbonyl.
"C₁₋₄-Alkanoyl" means the radical -C(O)R having from one to 4 carbon atoms designated (e.g., formyl, acetyl, propionyl, butyryl,)
"Halo" means fluoro, chloro or bromo.
"Leaving group" has the meaning conventionally associated with it in synthetic organic chemistry, i.e., an atom or group displaceable under alkylating conditions, and includes halo and alkane- or arenesulfonyloxy, such as mesyloxy, ethanesulfonyloxy, benzenesulfonyloxy, trifluoromethanesufonyloxy and tosyloxy.
"Animal" includes humans, non-human mammals, e.g., dogs, cats, rabbits, cattle, horses, sheep, goats, swine, and deer, and non-mammals, e.g., birds.
Insofar as the term "Disease" is used in this specification it includes any unhealthy condition of an animal or part thereof and includes an unhealthy condition which may be caused by, or incident to, medical or veterinary therapy applied to that animal, i.e., the "side effects" of such therapy.
"Pharmaceutically acceptable" means that which is useful in preparing a pharmaceutical composition that is generally safe, non-toxic and neither biologically nor otherwise undesirable and includes that which is acceptable for veterinary use as well as human pharmaceutical use.
"Pharmaceutically acceptable salts" means salts which are pharmaceutically acceptable, as defined above, and which possess the desired pharmacological activity. Such salts include acid addition salts formed with inorganic acids such as hydrochloric acid, hydrobromic acid, sulfuric acid, nitric acid and phosphoric acid; or with organic acids such as formic acid, acetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, *o*-(4-hydroxybenzoyl)benzoic acid, cinnamic acid, mandelic acid, methanesulfonic acid, ethanesulfonic acid, 1,2,-ethanedisulfonic acid, 2-hydroxyethanesulfonic acid, benzenesulfonic acid, *p*-chlorobenzenesulfonic acid, 2-naphthalenesulfonic acid, *p*-toluenesulfonic acid, camphorsulfonic acid, 4-methylbicyclo[2.2.2]oct-2-ene-1-carboxylic acid, glucoheptonic acid, 4,4'-methylenebis(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid and muconic acid.

Pharmaceutically acceptable salts also include base addition salts which may be formed when acidic protons present are capable of reacting with inorganic or organic bases. Acceptable inorganic bases include sodium hydroxide, sodium carbonate, potassium hydroxide, aluminum hydroxide and calcium hydroxide. Acceptable organic bases include ethanolamine, diethanolamine, triethanolamine, tromethamine and, *N*-methylglucamine

"Therapeutically effective amount" means that amount which, when administered to an animal for treating a disease, is sufficient to effect such treatment for the disease.

'Treating" or "treatment" of a disease includes:
(1) preventing the disease from occurring in an animal which may be predisposed to the disease but does not yet experience or display symptoms of the disease,
(2) inhibiting the disease, i.e., arresting its development, or
(3) relieving the disease, i.e., causing regression of the disease.

Compounds of Formula I in which R³ or R⁵ is amino or in which R⁴ or R⁵ is aminomethyl can react with amino acids to give compounds of Formula II. Suitable amino acids include L-alanine, L-arginine, L-asparagine, L-aspartic acid, L-cysteine, L-glutamine, L-glutamic acid, glycine, L-histidine, L-isoleucine, L-leucine, L-lysine, L-methionine, L-ornithine, L-phenylalanine, L-proline, L-serine, L-threonine, L-tryptophan, L-tyrosine or L-valine and achiral cyclo amino acids such as 1-amino-1-cyclopropanecarboxylic acid, 1-amino-1-cyclobutanecarboxylic acid, 1-amino-1-cyclopentanecarboxylic (cycloleucine) or 1-amino-1-cyclohexanecarboxylic acid. The amino acid group imparts hydrophilic properties to the molecule. The hydrophilicity promotes the water solubility of the molecule. The amide bond is subsequently cleaved *in vivo* by proteolysis. Thus, compounds of Formula II are water soluble prodrugs for compounds of Formula I in which R³ or R⁵ is amino or in which R⁴ or R⁵ is aminomethyl.

Compounds of Formula I in which R³ is hydro can react to form a dithio linkage with a (C₂₋₆)alkyl group which is substituted by one to two substituents independentyl selected from -N(R²⁹)₂, -C(O)OR³⁰, -PO(OR³⁰)₂, -SO₃R³⁰, -SO₂NHR³⁰ and -OR³⁰, in which each R²⁹ is independently hydro, acetyl or trifluoroacetyl and each R³⁰ is independently hydro or (C₁₋₅)alkyl. The substituted alkyl group imparts hydrophilic properties to the molecule. The hydrophilicity promotes the water solubility of the molecule. The dithio linkage is subsequently cleaved *in vivo* by chemical, enzymatic or metabolic transformation. Thus, compounds of Formula III are water soluble prodrugs for compounds of Formula I in which R³ is hydro.

The compounds of Formulae I, II and III are benzocyclohexylimidazolethione derivatives referred to as optionally substituted 1,2,3,4-tetrahydronaphthalen-2-yl.

The monovalent carbon of the benzocyclohexyl group may be a chiral center. Thus, compounds of Formula I and certain compounds used in the synthesis thereof may exist as either one of a pair of enantiomers of opposite chirality or as a mixture of such enantiomers. Compounds of Formulae II and III each contain one or two chiral centers. Compounds of Formulae II and III containing two chiral centers have two pairs of enantiomers (i.e., four diastereomers) and may exist as any one of the enantiomers or as a mixture thereof. The enantiomers are characterized by the absolute configuration of their chiral centers and described by the *R*and *S*-sequencing rules of Cahn, Ingold and Prelog. When two chiral centers are present, the configuration of each chiral center is assigned an *R* or *S* descriptor as appropriate. Conventions for stereochemical nomenclature, methods for the determination of stereochemistry and the separation of stereoisomers are well-known in the art (e.g., see "Advanced Organic Chemistry", 3rd edition, March, Jerry, John Wiley & Sons, New York, 1985). Unless indicated otherwise, the illustration, description or naming of a particular chiral compound of Formulae I, II, III, 1-6, 8-32, 44-46 and 48 in the specification or in the claims is intended to include both individual enantiomers and the mixtures, racemic or otherwise, thereof.

The imidazolethione portion R² of the molecule is according to the group of compounds of Formula I, specifically defined as a group of Formula (a): 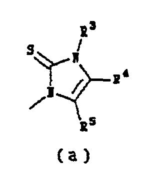 referred to as 1,3-dihydroimidazole-2-thione when forming the parent name or 2-thioxo-2,3-dihydro-1*H*-imidazolyl when forming a prefix to the parent name.

Certain R² and R⁶ groups exist in tautomeric equilibrium between thioxo and mercapto tautomers (e.g., a group of Formula (a) in which R³ is hydro). Compounds of Formula I or II which contain groups that can exist as either tautomer are named, illustrated or otherwise described in this application as thiones or thioxo substituted derivatives. However, it is to be understood that the mercapto tautomers are encompassed by such names, illustrations and descriptions as well.

The compounds of Formulae I, II and III are named by AUTONOM Version 1.0 by Beilstein-Institut and Springer-Verlag Berlin Heidelberg, a fully automatic computerized system for assigning IUPAC systematic nomenclature directly from the structural diagrams of organic compounds. For example, a compound of Formula I in which t is 0 and is a group of Formula (a), i.e., of the formula: 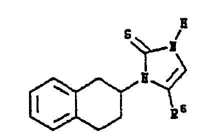 is named 5-aminomethyl-1-(1,2,3,4-tetrahydronaphthalen-2-yl)-1,3-dihydroimidazole-2-thione when R⁵ is aminomethyl and is named 3-(1,2,3,4-tetrahydro-naphthalen-2-yl)-2-thioxo-2,3-dihydro-1*H*-imidazole-4-carboxylic acid when R⁵ is carboxy.

A compound of Formula II in which t is 0 and R⁶ is a group of Formula (d), wherein R²¹ is L-α-aspartylaminomethyl, i.e., of the formula: 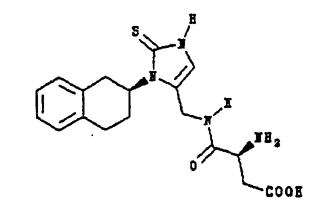 is named 3*S*-amino-*N*-[3-(1,2,3,4-tetrahydronaphthalen-2*S*-yl)-2-thioxo-2,3-dihydro-1*H*-imidazol-4-ylmethyl]succinamic acid.

A compound of Formula III in which t is 0 and R⁷ is a group of Formula (g), wherein R^{5'} is aminomethyl and R²⁸ is 2-amino-2-carboxyethyl, i.e., of the formula: 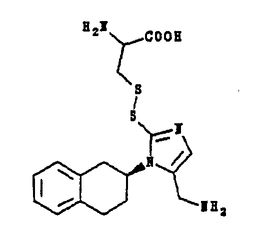 is named 2-amino-3-[5-aminomethyl-1-(1,2,3,4-tetrahydronaphthalen-2-yl)-1*H*-imidazol-2-yldisulfanyl]propionic acid.

### Presently Preferred Embodiments:

While the breadth of compounds which are intended by the invention is as set forth in the Summary of the Invention, certain compounds of Formulae I, II and III are preferred. Compounds of Formula I in which R² is a group of Formula (a) are designated as compounds of Formula I(a). Preferred compounds of Formula I(a) are those in which R⁵ is di(C₁₋₄)alkylaminomethyl, pyrrolidin-1-ylmethyl, piperidin-1-ylmethyl, morpholin-4-ylmethyl, piperazin-1-ylmethyl, 4-(C₁₋₄)alkylpiperazin-1-ylmethyl or -CH₂NHR¹⁰. Most preferred compounds of Formula I(a) are those in which t is 2, each R¹ is fluoro, preferably at the 5- and 7-position, and R⁵ is -CH₂NHR¹⁰, in which R¹⁰ is hydro, carbamoyl or (C₁₋₄)alkanoyl, preferably acetyl, particularly the *S*-enantiomer thereof.

Preferred compounds of Formula II are those in which R²¹ is -CH₂NHR²⁶. Most preferred are compounds of Formula II in which t is 2, each R¹ is fluoro, preferably at the 5- and 7-position, and R²¹ is -CH₂NHR²⁶, preferably wherein R²⁶ is arginyl, α-aspartyl, β-aspartyl, histidyl or ornithinyl.

Preferred compounds of Formula III are those in which R^{5'} is di (C₁₋₄)alkylaminomethyl, pyrrolidin-1-ylmethyl, piperidin-1-ylmethyl, morpholin-4-ylmethyl, piperazin-1-ylmethyl, 4-(C₁₋₄)alkylpiperazin-1-ylmethyl or -CH₂NHR¹⁰. Most preferred are compounds of Formula III in which t is 2, each R¹ is fluoro, preferably at the 5-and 7-position, R^{5'} is di(C₁₋₄)alkylaminomethyl, pyrrolidin-1-ylmethyl, piperidin-1-ylmethyl, morpholin-4-ylmethyl, piperazin-1-ylmethyl, 4- (C₁₋₄)alkylpiperazin-1-ylmethyl or -CH₂NHR¹⁰ and R²⁸ is a group selected from ethyl, 1,1-dimethylethyl and propyl (which group is further substituted with one to two substituente independently selected from carboxy, methoxycarbonyl, amino and trifluoroacetylamino, preferably wherein R²⁸ is (*R*)-2-amino-2-methoxycarbonylethyl, (*R*) -2-amino-2-carboxyethyl, (*R*)-2-trifluoroacetylamino-2-methoxycarbonylethyl, 2-aminoethyl, (*S*)-2-amino-2-carboxy-1,1-dimethylethyl or 3-amino-3-carboxyprop-1-yl.

### Pharmacology and Utility:

The compounds of the invention are inhibitors of dopamine β-hydroxylase. Accordingly, the compounds of the invention are useful in treating diseases capable of amelioration by inhibition of dopamine β-hydroxylase. For example, in as much as the compounds of the invention block norepinephrine biosynthesis, they are useful in treating diseases caused or exacerbated by a hypersympathetic condition. In particular, because the compounds of the invention are peripheral vasodilators, they are useful as afterload reducing agents in treating congestive heart failure. Furthermore, because the compounds of the invention reduce norepinephrine levels, they alleviate the damaging effects to the myocardium that hypersympatheic activity produces in congestive heart failure. Thus, the compounds of the invention are particularly useful for treating congestive heart failure because they produce an initial improvement in cardiac output by reducing afterload and a sustained improvement in cardiac function by reducing norepinephrine levels in the myocardial tissue.

The dopamine β-hydroxylase inhibitor properties of test compounds can be determined by an art-recognized *in vitro* assay which relies upon the DBH-catalyzed conversion of tyramine to octopamine and the inhibition of DBH activity by test compounds and is described specifically in Example 48. Dopamine β-hydroxylase inhibitor properties of test compounds also can be determined by an art-recognized *in vivo* assay which relies upon dopamine and norepinephrine tissue concentrations and the effect of the test compounds thereon (e.g., see author: B.A. Berkowitz et al., 1988 *J. Pharm. Exp Ther*. **245**, 850-857) and is described specifically in Example 49. The blood pressure lowering properties of test compounds can be determined by an *in vivo* assay utilizing spontaneously hypertensive rats which is described specifically in Example 50.

### Administration and Pharmaceutical Composition:

In general, compounds of the invention will be administered in therapeutically effective amounts via any of the usual and acceptable modes known in the art, either singly or in combination with another compound of the invention or with another therapeutic agent. A therapeutically effective amount may vary widely depending on the severity of the disease, the age and relative health of the subject, the potency of the compound used and other factors. A therapeutically effective amount may range from 0.1 milligram per Kg (mg/Kg) body weight per day to 30 mg/Kg body weight per day. Preferably the amount will be 1.0 to 10 mg/Kg/day. Therefore, a therapeutically effective amount for a 70 Kg human may range from 7.0 to 2100 mg/day, preferably 70 to 700 mg/day.

One of ordinary skill in the art of treating such diseases will be able to ascertain a therapeutically effective amount of a compound of the invention for a given disease without undue experimentation and in reliance upon personal knowledge and the disclosure of this application. In general, compounds of the invention will be administered as pharmaceutical compositions by one of the following routes: oral, systemic (e.g., transdermal, intranasal or by suppository) or parenteral (e.g., intramuscular, intravenous or subcutaneous). Compositions can take the form of tablets, pills, capsules, semisolids, powders, sustained release formulations, solutions, suspensions, elixirs, aerosols, or any other appropriate composition and are comprised of, in general, a compound of the invention in combination with at least one pharmaceutically acceptable excipient. Acceptable excipients are non-toxic, aid administration, and do not adversely affect the therapeutic benefit of the compound of Formula I. Such excipient may be any solid, liquid, semisolid or, in the case of an aerosol composition, gaseous excipient that is generally available to one of skill in the art.

Solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk, and the like. Liquid and semisolid excipients may be selected from water, ethanol, glycerol, propylene glycol and various oils, including those of petroleum, animal, vegetable or synthetic origin (e.g., peanut oil, soybean oil, mineral oil, sesame oil, etc.). Preferred liquid carriers, particularly for injectable solutions, include water, saline, aqueous dextrose and glycols.

Compressed gases may be used to disperse the compound of the invention in aerosol form. Inert gases suitable for this purpose are nitrogen, carbon dioxide, nitrous oxide, etc. Other suitable pharmaceutical carriers and their formulations are described in A.R. Alfonso *Remington's Pharmaceutical Sciences* 1985, 17th ed. Easton, Pa.: Mack Publishing Company.

The amount of a compound of the invention in the composition may vary widely depending upon the type of formulation, size of a unit dosage, kind of excipients and other factors known to those of skill in the art of pharmaceutical sciences. In general, the final composition will comprise from 10%w to 90%w of the compound, preferably 25%w to 75%w, with the remainder being the excipient or excipients.

Preferably the pharmaceutical composition is administered in a single unit dosage form for continuous treatment or in a single unit dosage form ad libitum when relief of symptoms is specifically required.
Representative pharmaceutical formulations containing a compound of Formula I are described in Example 52.

### CHEMISTRY:

### Compounds of Formula I(a) :

A method for making reference compounds of Formula I(a) in which R³, R⁴ and R⁵ are each hydro is depicted by the following Reaction Scheme I: 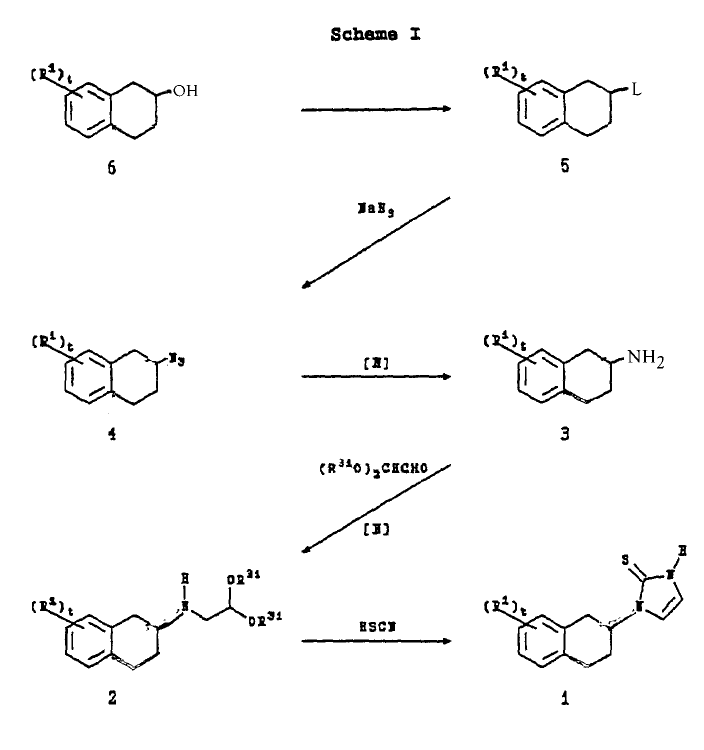 in which L is a leaving group, R³¹ is alkyl, preferably methyl or ethyl, and each n, t and R¹ are as defined in the Summary of the Invention with respect to Formula I.

Reference compounds of Formula I (a) in which R³, R⁴ and R⁵ are each hydro (Formula 1) can be prepared by reacting a compound of Formula 2 with thiocyanic acid in a suitable solvent, typically an alcohol (e.g., methanol, ethanol, any appropriate mixture of suitable alcohols, etc.) and preferably methanol. The reaction is carried out with potassium thiocyanate in the presence of aqueous acid (e.g., dilute hydrochloric acid, phosphoric acid or sulfuric acid, etc.) at 50 to 100°C, typically at 70 to 90°C and preferably at approximately 80°C, and requires 1 to 5 hours.

Compounds of Formula 2 can be prepared by reductive amination of a dialkyloxyacetaldehyde, preferably dimethoxyacetaldehyde or diethoxyacetaldehyde, with a compound of Formula 3. The reductive amination is carried out in the presence of a chemical reducing agent (e.g., sodium cyanoborohydride, sodium borohydride, etc.) or catalytic hydrogenation (e.g., H₂, palladium on carbon, H₂, Raney® nickel, etc.) in a suitable solvent (e.g., methanol, ethanol, ethyl acetate, any appropriate mixture of suitable solvents, etc.). Optionally water is removed from the reaction mixture by standard methods (e.g., with drying agents such as molecular seives or by azeotroping). Further details of the reaction steps set forth in this and the preceding paragraph are provided in Reference Example 3, infra.. Alternatively, compounds of Formula 2 can be prepared by reductive amination of a compound of Formula 7: 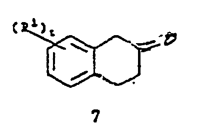 with a 2,2-dialkyloxyethylamine, preferably 2,2-dimethoxyethylamine or 2,2-diethoxyethylamine.

Compounds of Formula 3 can be obtained commercially or can be prepared by reacting a compound of Formula 5 with an appropriate azide salt (e.g., sodium azide, lithium azide, etc.) in a suitable solvent (e.g., dimethyl sulfoxide (DMSO), *N,N*-dimethylformamide (DMF) to give an azide of Formula 4 and then reducing. The reaction with the azide salt is carried out at 50 to 90°C, typically at 50 to 60°C and preferably at approximately 50°C, and requires 12 to 18 hours. Reduction of the compound of Formula 4 can be effected by catalytic hydrogenation (e.g., H₂, 10% palladium on carbon; or H₂, platinum on carbon, etc.) in a suitable solvent (e.g., ethyl acetate, ethanol). Further details of the reaction steps set forth in this paragraph are provided in Example 8, infra..

Compounds of Formula 5 are prepared by treating a compound of Formula 6 with an appropriate agent to create leaving group L. For example, compounds of Formula 5 in which L is mesyloxy can be prepared by reacting a compound of Formula 6 with methanesulfonyl chloride in a suitable solvent (e.g., diethyl ether, tetrahydrofuran (THF), methylene chloride, any appropriate mixture of suitable solvents, etc.). The reaction is carried out in the presence of triethylamine at -20 to 5°C, typically at -15 to -5°C and preferably at approximately -10°C, and requires 3 to 15 hours (for further details see Example 5, infra.).

An alternative method for making reference compounds of Formula I (a) in which R³, R⁴ and R⁵ are each hydro is depicted by the following Reaction Scheme II: 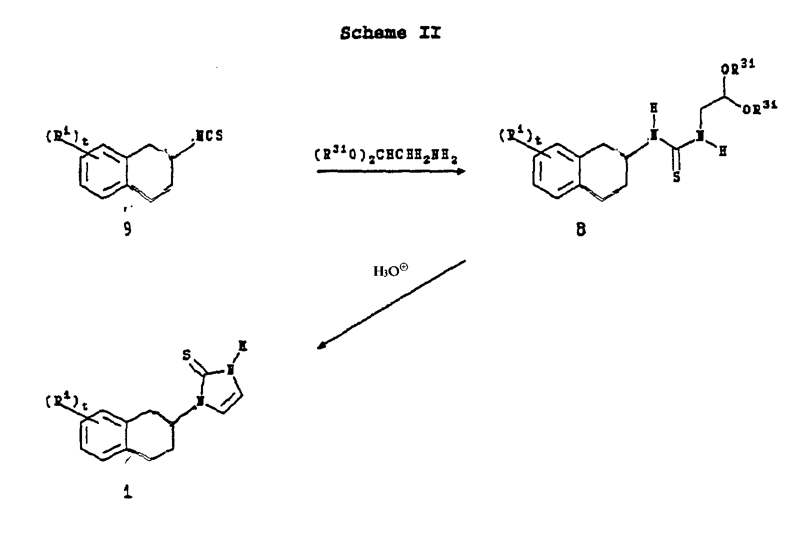 in which R³¹ is alkyl, preferably methyl or ethyl, and each n, t and R¹ are as defined in the Summary of the Invention with respect to Formula I.

Reference compounds of Formula I (a) in which R³, R⁴ and R⁵ are each hydro can be prepared by reacting a compound of Formula 9 with a 2,2-dialkyloxyethylamine, preferably 2,2-dimethoxyethylamine or 2,2-diethoxyethylamine, in a suitable solvent (e.g., DMF, DMSO, 1,3-dimethyl-3,4,5,6-tetrahydro-2(1*H*)-pyrimidinone (DMPU)) to give a compound of Formula 8 and then treating the compound of Formula 8 with acid (e.g., hydrochloric acid) to effect ring closure. The reaction with the amine is carried out at 20 to 90°C, typically at 70 to 90°C and preferably at approximately 85°C, and requires 1 to 2.5 hours. The treatment with acid and resultant ring closure is carried out at 20 to 90°C, typically at 70 to 85°C and preferably at approximately 80°C, and requires 3 to 72 hours. Further details of the reaction steps set forth in this paragraph are provided in Example 11, infra..

Compounds of Formula 9 can be prepared by reacting a compound of Formula 3 with 1,1'-thiocarbonyldiimidazole in a suitable solvent (e.g., ethyl acetate, acetonitrile, acetone, methylene chloride, any appropriate mixture of suitable solvents). The reaction is carried out at 0 to 50°C, typically at 10 to 30° and preferably at approximately 20°C, and requires 3 to 18 hours (for further details see Example 7, infra.).

A method for making compounds of Formula I(a) in which R³ is -(CH₂)_{q}R⁹ is depicted by the following Reaction Scheme III: 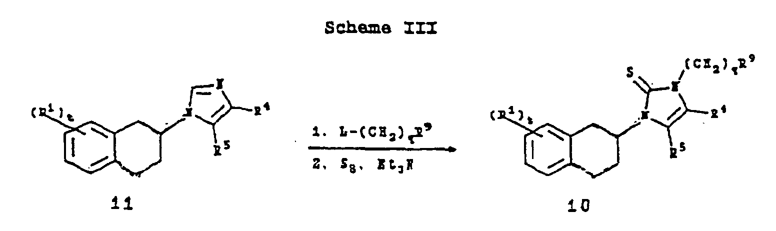 in which L is a leaving group and each n, t, R¹, R⁴, R⁵ and R⁹ are as defined in the Summary of the Invention with respect to Formula I.

Compounds of Formula I(a) in which R³ is -(CH₂)_{q}R⁹ (Formula 10) can be prepared by alkylating a compound of Formula 11 with an alkylating agent of the formula L-(CH₂)_{q}R⁹ to give the corresponding imidazolium salt and then sulfurizing. The alkylation is carried out in a suitable solvent (e.g., acetonitrile, DMF, THF, any appropriate mixture of suitable solvents), preferably acetonitrile or DMF, at 0 to 160°C, typically at approximately 25°C to reflux, and requires 1 to 16 hours. The sulfurization is carried out with lac sulfur in a suitable mild base (e.g., triethylamine, pyridine, any appropriate mixture of mild bases, preferably a mixture of triethylamine and pyridine) at 50 to 125°C, typically 80 to 100°C and preferably at approximately 90°C, and requires 1 to 8 hours. In a similar fashion, compounds of Formula I (a) in which R³ is amino can be prepared by reacting a compound of Formula 11 with an amino aryl or alkylsulfonate (e.g., *O*-mesitylenesulfonyl-hydroxylamine, *O*-methanesulfonylhydroxylamine or *O*-hydroxylaminesulfonic acid) to give the corresponding 3-aminoimidazolium salt and then sulfurizing. The reaction with the sulfonate is carried out in a suitable solvent (e.g., acetonitrile, methylene chloride, THF, any appropriate mixture of suitable solvents, preferably acetonitrile) at 0 to 40°C, typically at 10 to 30°C and preferably at approximately 20°C, and requires 0.5 to 18 hours. Further details of the reaction steps set forth in this paragraph are provided in Example 10, infra..

Compounds of Formula 11 can be prepared by reacting the corresponding compound of Formula 5 with an appropriately substituted imidazole in a suitable solvent (e.g., DMF, DMSO, acetonitrile). The reaction is carried out at 50 to 100°C, preferably at approximately 85°C, and requires 8 to 24 hours (for further details see Example 8, infra.). In a similar fashion, compounds of Formula 11 can be prepared by reacting an appropriately substituted 2-bromo-1,2,3,4-tetrahydronapththalen-1-one with an appropriately substituted imidazole and then reducing. The reduction can be carried out by catalytic hydrogenation (e.g., H₂, palladium hydroxide) in a suitable acidic solvent (e.g., sulfuric acid, acetic acid, any appropriate mixture of acids at 21,7 to 145,0 Pa (15 to 100 psi), typically at 58,0 to 87,0 Pa (40 to 60 psi) and preferably at approximately 72,5 Pa (50 psi), and requires 1 to 24 hours.

Alternatively, compounds of Formula 11 can be prepared by treating a corresponding of Formula I with Raney® nickel. The treatment with Raney® nickel is carried out in a suitable solvent (e.g., ethanol, methanol, acetic acid, water, any appropriate mixture of suitable solvents preferably ethanol) at 0 to 100°C, typically at 25 to 80°C and preferably at approximately 80°, and requires 0.25 to 4 hours (for further details see Example 14, infra.).

Compounds of Formula I(a) in which R³ and R⁴ are hydro and R⁵ is di(C₁₋₄)alkylaminomethyl, piperidin-1-ylmethyl or morpholin-4-ylmethyl can be prepared by alkylating a compound of Formula 11 in which R⁴ and R⁵ are both hydro with an appropriately *N,N*-disubstituted methyleneammonium salt (e.g., a *N,N*-di(C₁₋₄)alkylmethyleneammonium salt such as *N,N*-dimethylmethyleneammonium chloride, *N,N-*diethylmethyleneammonium chloride and the like, 1-methylenepiperidinium chloride, a 4-methylenemorpholinium chloride) and then sulfurizing. The alkylation is carried out in a suitable solvent (e.g., DMF, DMPU, acetonitrile, any appropriate mixture of suitable solvents, preferably DMF) at 50 to 100°C, typically at 80 to 100°C and preferably at approximately 95°C, and requires 4 to 18 hours.

Compounds of Formula I(a) in which R³ is hydro can be prepared by reacting a compound of Formula 11 with a strong base (e.g., *n*-butyllithium, lithium diisopropylamide (LDA)) in a suitable solvent (e.g., 1,2-dimethoxyethane, THF, 2-methoxyethyl ether.) to give the corresponding 2-imidazolide and then sulfurizing. The reaction with the base is carried out by cooling a solution of a compound of Formula 12 to between 0 and -78°C, typically to between -50 and -78° and preferably to approximately -78°C, adding the base and then allowing the reaction to proceed for 0.25 to 3 hours. The sulfurization is carried out at 0 to -78°C, typically at -50 and -78° and preferably at approximately -78°C, and requires 2 to 18 hours. Further details of the reaction steps set forth in this paragraph are provided in Reference Example 3a, infra..

A method for making compounds of Formula I(a) in which R³ and R⁴ are hydro and R⁵ is amino is depicted by the following Reaction Scheme IV: 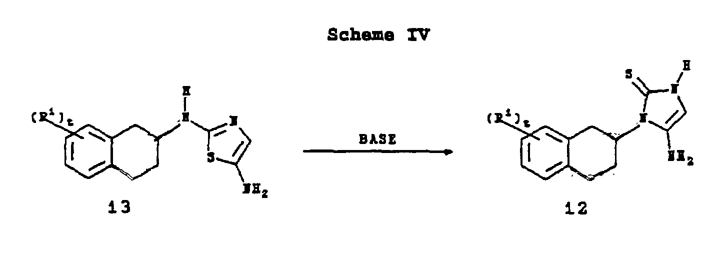 in which each t and R¹ are as defined in the Summary of the Invention with respect to Formula I.

Compounds of Formula I(a) in which R³ and R⁴ are hydro and R³ is amino (Formula 12) can be prepared by treating a compound of Formula 13 with an alkali base (e.g., potassium hydroxide or sodium hydroxide) to effect rearrangement. The treatment with base and attendant rearrangement is carried out at 0 to 40°C, typically at 10 to 30°C and preferably at approximately 20°C, and requires 0.1 to 2 hours.

Compounds of Formula 13 can be prepared by reacting a compound of Formula 9 with aminoacetonitrile hydrochloride in a suitable solvent (e.g., triethylamine, triethylamine in acetonitrile, dimethylformamide, or dimethylsulfoxide). The reaction is carried out at 20 to 100°C, typically at 10 to 30°C and preferably at approximately 20°C, and requires 12 to 24 hours. Further details of the reaction steps set forth in this paragraph and the preceding paragraph are provided in Example 11, infra..

A method for making compounds of Formula I(a) in which R³ is hydro, R⁴ is hydro, (C₁₋₄)alkyl or (C₁₋₄)alkyloxycarbonyl and R⁵ is cyano or (C₁₋₄)alkyloxycarbonyl is depicted by the following Reaction Scheme V: 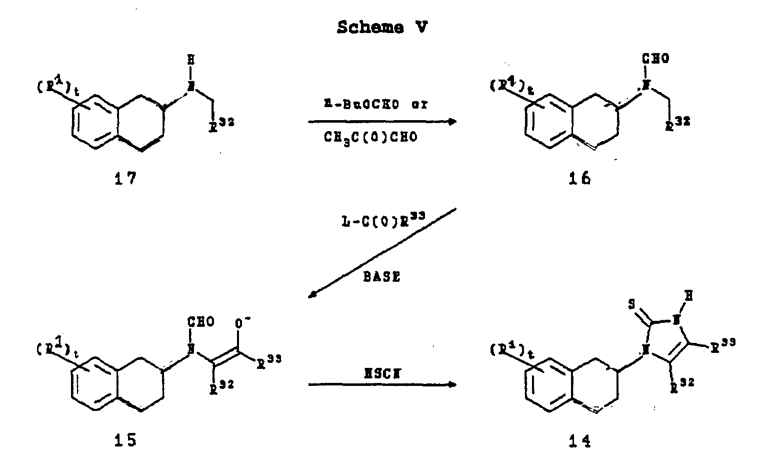 in which L is a leaving group (e.g., halo, alkyloxy, acyloxy, aryloxy, etc.), R³² is cyano or (C₁₋₄)alkyloxycarbonyl), R³³ is hydro, (C₁₋₄)alkyl or (C₁₋₄)alkyloxycarbonyl and each t and R¹ are as defined in the Summary of the Invention with respect to Formula I.

Compounds of Formula I(a) in which R³ is hydro, R⁴ is hydro, (C₁₋₄)alkyl or (C₁₋₄)alkyloxycarbonyl and R⁵ is cyano or (C₁₋₄)alkyloxycarbonyl (Formula 14) can be prepared by reacting a compound of Formula 16 with a compound of the formula R³³C(O)L to give a compound of Formula 15 and then reacting the compound of Formula 15 with thiocyanic acid. The reaction with the compound of the formula R³³C(O)L is carried out in the presence of base (e.g., potassium tert-butoxide, LDA) and in a suitable solvent (e.g., THF, 1,2-diethoxyethane, diethyl ether, any appropriate mixture of suitable solvents).

For example, a compound of Formula 15 in which R⁴ is hydro can be prepared by reacting a compound of Formula 16 with an alkyl or arylformate (e.g., ethyl formate, phenyl formate) in the presence of potassium *tert*-butoxide. The reaction with the formate is carried out at -40 to 65°C, typically at -30 to 0°C and preferably at approximately -15°C, and requires 3 to 24 hours (for further details see Example 24, infra.). A compound of Formula 15 in which R⁴ is (C₁₋₄)alkyl can be prepared by reacting a compound of Formula 16 with an (C₂₋₅)alkanoic acid chloride or anhydride (e.g., acetyl chloride, propionyl chloride; acetic anhydride) in the presence of LDA. The reaction with the acid chloride or anhydride is carried out at -78 to -15°C, typically at -50 to -78°C and preferably at approximately -78°C, and requires 1 to 24 hours (for further details see Example 25, infra.). The reaction with the thiocyanic acid is carried out with potassium thiocyanate in the presence of aqueous acid (e.g, aqueous hydrochloric acid, aqueous sulfuric acid, aqueous phosphoric acid.) at 50 to 100°C, typically at 75 to 95°C and preferably at approximately 85°C, and requires 1 to 5 hours.

Compounds of Formula 16 can be prepared by heating a compound of Formula 17 in *n*-butyl formate at temperatures of 70 to 105°C, preferably at approximately 105°C, for 3 to 24 hours. Alternatively, compounds of Formula 16 can be prepared by reacting a compound of Formula 17 with acetic formic anhydride in a suitable solvent (e.g., methylene chloride, dichloromethane, THF). The reaction with acetic formic anhydride is carried out at -15 to 25°C, preferably at approximately 0°C, and requires 0.5 to 3 hours.

Compounds of Formula 17 in which R³² is ethoxycarbonyl can be prepared by the reductive amination of ethyl glyoxalate with a compound of Formula 3. The reductive amination is carried out in the presence of a chemical reducing agent (e.g., sodium cyanoborohydride, sodium borohydride, etc.) or catalytic hydrogenation (e.g., H₂, palladium on carbon; H₂, nickel; etc.) in a suitable solvent (e.g., methanol, ethanol, ethyl acetate, any appropriate mixture of suitable solvents). Optionally water is removed from the reaction mixture by standard methods (e.g., with drying agents such as molecular seives or by azeotroping). For further details of the reaction steps set forth in this and the preceding paragraph see Example 17, infra..

Compounds of Formula 17 in which R³² is cyano can be prepared by reacting a compound of Formula 3 with formaldehyde sodium bisulfite complex and potassium cyanide in a suitable solvent (e.g., water, aqueous ethanol, etc.). The reaction is carried out at 50 to 80°C, typically at 50 to 60°C and preferably at approximately 50°C, and requires 0.5 to 2 hours (for further details see Example 13, infra.).

A method for making compounds of Formula I (a) in which R³ and R⁵ are hydro and R⁴ is formyl is depicted by the following Reaction Scheme VI: 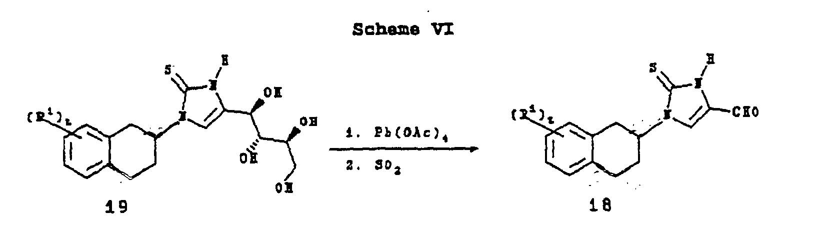 in which each t and R¹ are as defined in the Summary of the Invention with respect to Formula I.

Compounds of Formula I(a) in which R³ and R⁵ are hydro and R⁴ is formyl (Formula 18) are prepared by oxidizing a compound of Formula 19. The oxidization is effected with an appropriate oxidizing agent (e.g., lead tetraacetate, periodic acid) in a suitable solvent (e.g., acetic acid-benzene, acetic acid-toluene, acetic acid, any appropriate mixture of suitable solvents) at 0 to 80°C, typically at 20 to 40°C and preferably at approximately 25°C, and requires 0.25 to 4 hours.

Compounds of Formula 19 are prepared by reacting a compound of Formula 9 with D-(+)-glucosamine. The reaction is carried out in a suitable solvent, typically an alcohol (e.g., ethanol, methanol, any appropriate mixture of alcohols) and preferably ethanol, at 50 to 80°C, typically at 70 to 80°C and preferably at approximately 80°C, and requires 1 to 2 hours. Further details of the reaction steps set forth in this and the preceding paragraph are provided in Example 16, infra..

Compounds of Formula I in which R³ and R⁴ are hydro and R⁵ is hydroxymethyl can be prepared by reacting a compound of Formula 3, or the acid addition salt thereof, with thiocyanic acid and dihydroxyacetone in a suitable solvent (e.g., ethyl acetate, THF, dioxane, any appropriate mixture of suitable solvents, preferably ethyl acetate) and then optionally treating the reaction mixture with sulfuric acid (treating with sulfuric acid enhances purity). The reaction is carried out with potassium thiocyanate in the presence of acid (e.g., glacial acetic acid, propionic acid, preferably glacial acetic acid) under nitrogen at 20 to 50°C for 0.5 to 3 hours (for further details see Example 12, infra.).

### Additional Processes for Making Compounds of Formula I:

Compounds of Formula I in which R⁵ is 1*H*-tetrazol-5-yl can be prepared by reacting a compound of Formula I in which R⁵ is cyano with a hydrazoic acid derivative (e.g., tributyltin azide, triphenylsilyl azide, t-butyldiphenylsilyl azide). The reaction is carried out at 100 to 150°C, typically at 120 to 140°C and preferably at approximately 130°C, and requires 2 to 18 hours (for further details see Example 15, infra.).

Compounds of Formula I in which R⁵ is carbamoyl can be prepared by hydrolyzing compound of Formula I in which R⁵ is cyano. The hydrolysis can be carried out with aqueous hydrochloric acid at 100 to 140°C, typically at 125 to 135°C and preferably at approximately 130°C, and requires 2 to 18 hours. Proceeding as described above, 5,6-difluoroindan-2-yl-2-thioxo-2,3-dihydro-1*H*-imidazol-4-yl-carboxamide, m.p. >280°C, was prepared.

Compounds of Formula I in which R⁵ is -C(NH)NR¹⁵R¹⁶ can be prepared by reacting a compound of Formula I in which R⁵ is cyano with a reagent of the formula (CH₃)₂AlNR¹⁵R¹⁶, in a suitable solvent (e.g., toluene, benzene, methylene chloride, tetrachloroethane, any appropriate mixture of suitable solvents). The reaction is carried out at 20 to 130°C, typically at 60 to 100°C and preferably at approximately 80°C, and requires 1 to 18 hours. The reagent of the formula (CH₃)₂AlNR¹⁵R¹⁶ is prepared by reacting an amine of the formula NHR¹⁵R¹⁶ with trimethylaluminum. Proceeding as described above 5-(aminoiminomethyl)-1-(5,6-difluoroindanyl-2-yl)-1,3-dihydroimidazole-2-thione and 1-(5,6-difluoroiindan-2-yl)-5-[imino(2,2,2-trifluoroethylamino)methyl]-1,3-dihydroimidazole-2-thione, m.p. 199-200°C, were prepared.

Compounds of Formula I in which R⁵ is 4,5-dihydroimidazol-2-yl can be prepared by reacting compound of Formula I in which R⁵ is cyano with ethylenediamine. The reaction can be carried out by heating the reactants in the presence *p*-toluenesulfonic acid at 100 to 250°C, typically at 180 to 220°C and preferably at approximately 200°C, for 1 to 3 hours. Proceeding as described above, 1,2,3,4-tetrahydronaphthalen-2-yl-5-(4,5-dihydroimidazol-2-yl)-1,3-dihydroimidazole-2-thione, m.p. 130-145°C, was prepared.

Compounds of Formula I in which R³ and R⁴ are hydro and R⁵ is aminomethyl can be prepared by reducing compound of Formula I in which R⁵ is cyano. The reduction can be effected with a chemical reducing agent (e.g., lithium aluminum hydride, borane in THF, aluminum hydride, etc.) in a suitable solvent (e.g., THF, 1,2-dimethoxyethane, 2,2-dimethoxyethyl ether, any appropriate mixture of suitable solvents) at 0 to 65°C, typically at 0 to 20°C and preferably at approximately 0°C and requires 1 to 5 hours (for further details see Example 20, infra.).

A preferred method for making compounds of Formula I in which R³ and R⁴ are hydro and R⁵ is aminomethyl is depicted by the following Reaction Scheme IX: 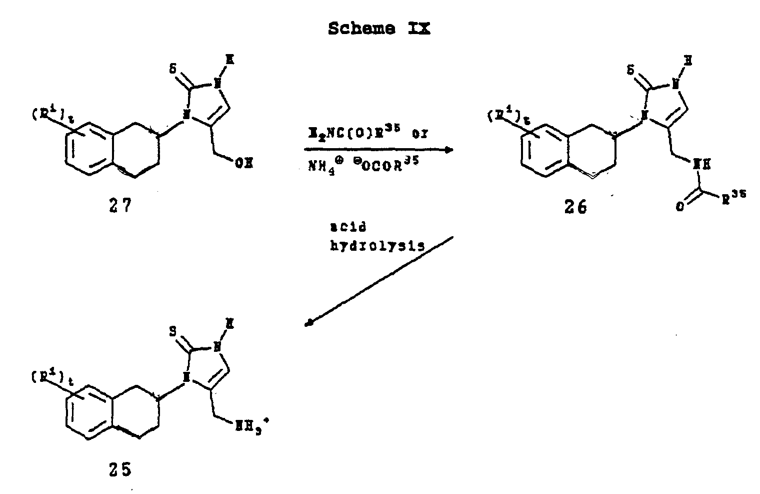 in which R³⁵ is hydro, (C₁₋₄)alkyl or trifluoro(C₁₋₄)alkyl and each t and R¹ are as defined in the Summary of the Invention with respect to Formula I.

The acid addition salt of a compound of Formula I in which R³ and R⁴ are each hydro and R⁵ is aminomethyl (Formula 25) can be prepared by acid catalyzed hydrolysis of the corresponding compound of Formula I in which R⁵ is formylaminomethyl, (C₁₋₄)alkylcarbonylaminomethyl or trifluoro(C₁₋₄)alkylcarbonyl-aminomethyl (Formula 26). The hydrolysis is carried out in a suitable solvent, typically an alcohol (e.g., isopropanol, ethanol, methanol, any appropriate mixture of alcohols) and preferably isopropanol, and under nitrogen at 65 to 82°C, preferably at reflux, and requires 0.5 to 4 hours.

Pharmaceutically acceptable acid addition salts of compounds of Formula I in which R³ and R⁴ are each hydro and R⁵ is aminomethyl can be prepared by performing the hydrolysis with a pharmaceutically acceptable acid (e.g., 2 to 8 equivalents of concentrated hydrochloric acid, preferably approximately 5 equivalents). Alternatively, any acid addition salt form of a compound of Formula 25 can be converted to the corresponding free base form by reacting with an acceptable inorganic or organic base and then converted to a pharmaceutically acceptable acid addition salt by reacting with an appropriate pharmaceutically acceptable acid. Further details of the reaction steps set forth in this paragraph and the preceding paragraph are provided in Example 2, infra..

Compounds of Formula I in which R⁵ is formylaminomethyl, (C₁₋₄)alkylcarbonylaminomethyl or trifluoro (C₁₋₄)alkylcarbonylaminomethyl can be prepared by reacting the corresponding compound of Formula I in which R⁵ is hydroxymethyl (Formula 27) with a primary amide of the formula H₂NC(O)R³⁵ (e.g., formamide, acetamide, trifluoroacetamide). The reaction is carried out by adding the compound of Formula 27 to the amide and then heating the mixture under a stream of nitrogen for 0.5 to 2 hours at 150 to 190°C. Preferably the amide is formamide and the reaction is carried out by heating at 170 to 175° for approximately 1 hour. Proceeding similarly but substituting urea for the primary amide, compounds of Formula I in which R⁵ is ureidomethyl can be prepared. Further details of the reaction steps set forth in this paragraph are provided in Example 20, infra..

A preferred process for preparing compounds of Formula 26 comprises reacting a compound of Formula 27 with an ammonium salt of the formula NH₄^{+ -}OC(O)R³⁵ (e.g., ammonium formate, ammonium acetate, ammonium trifluoroacetate, preferably ammonium formate). For example, a compound of Formula 27 can be reacted with ammonium formate to give a compound of Formula 24 wherein R³⁵ is hydro. The reaction is carried out neat or in formamide, preferably formamide at 100 to 180°C, preferably at 120 to 150°C, and requires 1 to 2 hours (for further details see Example 21, infra.).

Compounds of Formula I in which R³ and R⁴ are hydro and R⁵ is aminomethyl, (C₁₋₄)alkylaminomethyl, di (C₁₋₄)alkylaminomethyl, pyrrolidin-1-ylmethyl, piperidin-1-ylmethyl, morpholin-4-ylmethyl, piperazin-1-ylmethyl or 4-(C₁₋₄)alkylpiperazin-1-ylmethyl can be prepared by converting a compound of Formula I in which R⁵ is hydroxymethyl to a compound of Formula 28: 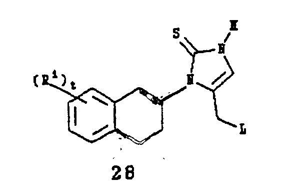 in which L is a leaving group and t and R¹ are as defined in the Summary of the Invention with respect to Formula I, and reacting the compound of Formula 28 with an amine of the formula HNR³⁶R³⁷, in which R³⁶ and R³⁷ are independently (C₁₋₄)alkyl or together are -(CH₂)₄-, -(CH₂)₅-, -(CH₂)₂O(CH₂)₂- or -(CH₂)₂NR³⁸(CH₂)₂- (in which R³⁸ is hydro or (C₁₋₄)alkyl). The conversion to the compound of Formula 28 is effected with an appropriate agent for forming a suitable leaving group (e.g., methanesulfonyl chloride, thionyl chloride, phosphorous pentachloride, phosphorous oxychloride,) in a suitable solvent (e.g, methylene chloride, chloroform, THF, any appropriate mixture of suitable solvents,) The reaction with the amine is carried out in a suitable solvent (e.g., THF, 1,2-dimethoxyethane, acetonitrile, any appropriate mixture of suitable solvents) at -10 to 20°C and requires 1 to 4 hours. Further details of the reaction steps set forth in this paragraph are provide in Example 23, infra..

Compounds of Formula I in which R⁴ is aminomethyl can be prepared by reacting a corresponding compound of Formula I in which R⁴ is formyl with hydroxylamine hydrochloride to give the corresponding oxime and then reducing. The reaction with the hydroxylamine hydrochloride is carried out in the presence of a suitable base (e.g., sodium hydroxide, sodium acetate, etc.) and in a suitable solvent, typically an alcohol (e.g., ethanol, methanol, any appropriate mixture of alcohols) or a mixture of alcohol and water (e.g., ethanol/water (1:1)) at 20 to 100°C, typically at 50 to 70°C and preferably at approximately 60°C, and requires 1 to 8 hours. The reduction of the oxime can be effected with a chemical reducing agent (e.g., lithium aluminum hydride) in a suitable solvent (e.g., THF, any mixture of suitable solvents) at -50 to 50°C, typically at -20 to 20°C and preferably at approximately 0°C. For further details of the reaction steps set forth in this paragraph see Example 38, infra..

Compounds of Formula I in which R⁴ is -CH₂NHR¹⁰, wherein R¹⁰ is as defined in the Summary of the Invention with respect to Formula I, can be prepared by reductive amination of a compound of Formula I in which R⁴ is formyl with an amine of the formula NH₂R¹⁰ (e.g., glycine *tert*-butyl ester hydrochloride, glycinamide hydrochloride, phenethylamine, methyl 4-(2-amino-ethyl)benzoate). The reductive amination is carried out in the presence of a chemical reducing agent (e.g., sodium cyanoborohydride, sodium borohydride) or catalytic hydrogenation (e.g., H₂, palladium on carbon, H₂, Raney® nickel) in a suitable solvent (e.g., THF, water, ethyl acetate, alcohol, any appropriate mixture of solvents), typically an alcohol (e.g., ethanol, methanol, any appropriate mixture of alcohols, etc.) at 20 to 100°C, preferably at approximately 50°C, and requires 1 to 8 hours (for further details see Example 39, infra.).

Compounds of Formula I in which R⁴ is 1-hydroxy(C₁₋₄)alkyl can be prepared by reacting a corresponding compound of Formula I in which R⁴ is formyl with an appropriate alkylating agent (e.g., methylmagnesium chloride, ethylmagnesium chloride, *n*-propyimagnesium chloride.). The alkylation is carried out neat at -20 to 60°C, typically at 0 to 25°C and preferably at approximately 0°C (for further details see Example 31, infra.).

Compounds of Formula I in which R³ or R⁵ is -NHC(NR¹¹)NHR¹² or compounds of Formula I in which R⁴ or R⁵ is -CH₂NHC(NR¹¹)NHR¹² in which R¹¹ is hydro, acetyl or *tert*-butoxycarbonyl and R¹² is acetyl or *tert*-butoxycarbonyl can be prepared by reacting a compound of Formula I in which R³, R⁴, or R⁵, is amino or aminomethyl with an appropriately substituted amidine (e.g., *N*¹-(*tert*-butoxy-carbonyl)methylthioamidine, *N*¹,*N*²-di(*tert*-butoxycarbonyl)methylthioamidine, *N*¹,*N*²-di(acetyl)methylthioamidine). The reaction is carried out in a suitable solvent (e.g., THF, methanol, ethanol, DMF, water, any appropriate mixture of suitable solvents, preferably THF) at 0°C to reflux, typically at 20°C to reflux and preferably at approximately 50°C, under an inert atmosphere, and requires 1 to 24 hours (for further details see Example 40, infra.).

Compounds of Formula I in which R³, or R⁵ is -NHC(NR¹¹)NHR¹² or compounds of Formula I in which R⁴, or R⁵ is -CH₂NHC (NR¹¹) NHR¹², wherein R¹¹ and R¹² are hydro, can be prepared by treating the corresponding compound of Formula I wherein R¹¹ and/or R¹² are acetyl or tert-butoxycarbonyl with a suitable acid (e.g., trifluoroacetic acid (TFA), hydrochloric acid, hydrobromic acid, sulfuric acid, preferably TFA) and optionally with a suitable cosolvent (e.g., ethanol). The acid treatment is carried out at 0 to 120°C, typically at 0 to 80°C and preferably at approximately 25°C, and requires 0.5 to 12 hours (for further details see Example 41, infra.).

Compounds of Formula I in which R³ and R⁵ are hydro and R⁴ is di(C₁₋₄)alkylaminomethyl, piperidin-1-ylmethyl or morpholin-4-ylmethyl can be prepared by alkylating a compound of Formula I in which R³, R⁴ and R⁵ are each hydro with an appropriately *N,N*-disubstituted methyleneammonium salt. The alkylation is carried out in a suitable solvent (e.g., DMF, acetonitrile, any appropriate mixture of suitable solvents, preferably DMF) at 50 to 130°C, typically at 80 to 110°C and preferably at approximately 95°C, and requires 1 to 24 hours.

Compounds of Formula I in which R⁴ is hydro, R⁵ is di(C₁₋₄)alkylaminomethyl, piperidin-1-ylmethyl or morpholin-4-ylmethyl and R³ is other than hydro can be prepared by alkylating a corresponding compound of Formula I in which R⁵ is hydro with about 1 molar equivalent of an appropriately *N,N*-disubstituted methyleneammonium salt. The alkylation is carried out in a suitable solvent (e.g., DMF, DMPU, acetonitrile, any appropriate mixture of suitable solvents, preferably DMF) at 0°C to reflux, typically at 25 to 100°C and preferably at approximately 80°C, and requires 1 to 24 hours (for further details see Example 26, infra.).

Compounds of Formula I in which R³ and R⁴ are hydro and R⁵ is di(C₁₋₄)alkylaminomethyl, piperidin-1-ylmethyl or morpholin-4-ylmethyl can be prepared by alkylating a thio protected derivative of a corresponding compound of Formula I in which R⁵ is hydro (e.g., the 3-(imidazol-2-ylthio)propionate derivative thereof) with about 1 molar equivalent of an appropriately *N,N*-disubstituted methyleneammonium salt and then deprotecting. The alkylation is carried out in a suitable solvent (e.g., DMF, DMPU, acetonitrile, any appropriate mixture of suitable solvents, preferably DMF) at 50 to 130°C, preferably at 80 to 100°C, and requires 1 to 24 hours. The deprotection can be effected with a suitable base (e.g., a sodium alkoxide such as sodium ethoxide, sodium hydroxid, potassium hydroxide, preferably sodium ethoxide) at 0 to 50°C, preferably at approximately 25°C.

A suitable thio protected derivative can be prepared by reacting a compound of Formula I in which R³ is hydro with ethyl acrylate to give a 3-(imidazol-2-ylthio)propionate derivative. The protection step is carried out in the presence of an acid (e.g., anhydrous hydrochloric acid) in a suitable solvent typically an alcohol (e.g., methanol, ethanol, any appropriate mixture of suitable alcohols,) and preferably ethanol, at 0°C to reflux, typically at 50°C to reflux and preferably at approximately 80°C.

Compounds of Formula I in which R³ is hydro and R⁴ and R⁵ are each di(C₁₋₄)alkylaminomethyl, piperidin-1-ylmethyl or morpholin-4-ylmethyl can be prepared by alkylating a protected derivative of a corresponding compound of Formula I in which R⁴ and R⁵ are each hydro with 2 to 15 molar equivalents of an appropriately *N,N*-disubstituted methyleneammonium salt, typically 5 to 10 molar equivalents and preferably approximately 7 molar equivalents, and then deprotecting. The alkylation is carried out in a suitable solvent (e.g., DMF, DMPU, acetonitrile, any appropriate mixture of suitable solvents, preferably DMF) at 50 to 130°C, typically at 90 to 110°C and preferably at approximately 100°C, and requires 1 to 24 hours (for further details see Example 27, infra.).

Compounds of Formula I in which R⁴ and/or R⁵ are hydroxymethyl can be prepared by reducing a compound of Formula I in which R⁴ and/or R⁵ is ethoxycarbonyl. The reduction can be effected with a chemical reducing agent (e.g., sodium borohydride, calcium borohydride, lithium borohydride, lithium aluminum hydride, preferably sodium borohydride in the presence of calcium chloride) in a suitable solvent (e.g., THF, diglyme, dioxane, any appropriate mixture of suitable solvents, etc., preferably THF) at -20°C to reflux, typically at 0 to 80°C and preferably at approximately 50°C, requiring 1 to 72 hours (for further details see Example 24, infra.).

Compounds of Formula I in which R³, R⁴ or R⁵ is a group selected from aroyl, heteroaroyl, aryl (C₁₋₄) alkyl and heteroaryl (C₁₋₄)alkyl (which aroyl, heteroaroyl, aryl and heteroaryl are further substituted by a 1*H*-tetrazol-5-yl substituent can be prepared by reacting a corresponding of Formula I in which the aroyl, heteroaroyl, aryl and heteroaryl substituent which are further substituted by a cyano substituent with a hydrazoic acid derivative (e.g., tributyltin azide). The reaction is carried out neat or in a suitable solvent (e.g., xylene, toluene, benzene, any appropriate mixture of suitable solvents, etc., preferably xylene) at 80 to 150°C, typically at 80 to 130°C and preferably at reflux, and requires 4 to 24 hours (for further details see Example 30, infra.).

Compounds of Formula I in which R⁵ or both R⁴ and R⁵ are 1*H*-tetrazol-5-yl-aminocarbamoyl, 2-(dimethylamino)ethylcarbamoyl, 4-methylpiperazin-1-ylcarbonyl, methylsulfonylanilinocarbonyl or 2-(dimethylamino)ethylmercaptocarbonyl can be prepared by reacting a compound of Formula I in which R⁵ or both R⁴ and R⁵ are carboxy, or an acid derivative thereof, with an appropriate amine or thiol (i.e., 5-amino-1*H*-tetrazole, 2-(dimethylamino)ethylamine, 4-methylpiperizine, 4-methylsulfonylaminoaniline or 2-dimethylaminoethanethiol hydrochloride). For example, compounds of Formula I in which R⁵ is 1*H*-tetrazol-5-ylcarbamoyl can be prepared by converting the corresponding carboxylic acid to an acid halide and then reacting the acid halide with 5-amino-1*H*-tetrazole. The reaction with the 5-amino-1*H*-tetrazole is carried out in a suitable solvent (e.g., pyridine, DMF, any appropriate mixture of suitable solvents) at 0 to 40°C, typically at 20 to 30°C and preferably at approximately 25°C, and requires 1 to 24 hours (for further details see Example 32, infra.).

Compounds of Formula I in which R⁵ is 2-(dimethylamino)ethylcarbamoyl, 4-methylpiperazin-1-ylcarbonyl or 2-(dimethylamino)ethylmercaptocarbonyl can be prepared by treating a corresponding carboxylic acid with a coupling agent (e.g., 1,1'-carbonyldiimidazole, dicyclohexylcarbodiimide, benzotriazol-1-yloxytripyrrolidinophosphonium hexafluorophosphate (PyBOP), etc.) in a suitable solvent (e.g., THF, methylene chloride, DMF, any appropriate mixture of suitable solvent) and then reacting with the appropriate amine or thiol. The reaction with the amine or thiol is carried out at 0 to 80°C, typically at 20 to 30°C and preferably at approximately 25°C, and requires 1 to 24 hours (for further details see Example 35 infra.).

Compounds of Formula I in which R³ is 2-(C₁₋₄)alkyloxycarbonylethyl can be prepared by reacting a compound of Formula I in which R³ is hydro with (C₁₋₄)alkyl acrylate (e.g, methyl acrylate, ethyl acrylate ). The reaction is carried out in the presence of base (e.g., sodium ethoxide, benzyltrimethylammonium hydroxide, sodium hydride.) and in a suitable solvent (e.g., ethanol, *N,N*-dimethylformamide, *N,N*-dimethylacetamide, acetonitrile, .) at 50 to 100°C, preferably approximately at 80°C, and requires 1 to 6 hours (for further details see Example 25, infra.).

Compounds of Formula I in which a R³, R⁴ or R⁵, substituent is carboxy or a group which is further substituted by a carboxy substituent can be prepared by hydrolysis of a corresponding compound of Formula I in which a R³, R⁴ or R⁵ substituent is (C₁₋₄)alkyloxycarbonyl or a group which is further substituted by a (C₁₋₄)alkyloxycarbonyl substituent. The hydrolysis can be carried out with an aqueous base (e.g., potassium hydroxide, sodium hydroxide, lithium hydroxide) in a suitable solvent, typically an alcohol (e.g., ethanol, methanol, isopropanol, any appropriate mixture of alcohols) and preferably ethanol, or an aqueous acid (e.g., hydrochloric acid, trifluoroacetic acid, sulfuric acid, hydrochloric acid, hydrobromic acid) in a suitable solvent (e.g., methylene chloride, ethyl acetate, dioxane, DMF, THF) at 20 to 120°C, typically at 90 to 110°C and preferably at approximately 100°C, and requires 4 to 24 hours (for further details see Example 28, infra.).

Compounds of Formula I in which a R³, R⁴ or R⁵ is carbamoyl or a group which is further substituted by a carbamoyl substituent can be prepared by amination of a corresponding compound of Formula I in which a R³, R⁴ or R⁵ is carboxy or a group which is further substituent by a carboxy substituent. The amination can be carried out by converting the carboxylic acid to the corresponding acid chloride and then reacting the acid chloride with aqueous ammonium hydroxide. Converting the acid to the acid chloride is carried out with an appropriate chlorinating agent (e.g., thionyl chloride, oxalyl chloride, phosphorus pentachloride) and in a suitable solvent (e.g., DMF, methylene chloride, dichloroethane, any appropriate mixture of suitable solvents, preferably DMF) at 10 to 40°C, typically at 15 to 30°C and preferably at approximately 20°C, and requires 2 to 18 hours. The reaction between the acid chloride and the aqueous ammonium hydroxide is carried out at 0 to 50°C, typically at 20 to 30°C and preferably at approximately 25°C, and requires 0.5 to 24 hours. Further details of the reaction steps set forth in this paragraph are provided in Example 29, infra..

Compounds of Formula I in which R³, R⁴ or R⁵ is (C₁₋₄)alkyloxycarbonyl or a group that is further substituted by a (C₁₋₄)alkyloxycarbonyl group can be prepared by reacting a compound of Formula I in which R³, R⁴ or R⁵ is carboxy or a group which is further substituted by a carboxy group with a (C₁₋₄)alcohol. The reaction is carried out at 20 to 110°C, preferably approximately 85°C, and requires 8 to 72 hours.

Compounds of Formula I in which R¹⁰ is (C₁₋₄)alkanoyl, trifluoro(C₁₋₄)alkanoyl, carbamoyl, (C₁₋₄)alkyloxycarbonyl, (C₁₋₄)alkylcarbamoyl, di(C₁₋₄)alkylcarbamoyl, amino (C₁₋₄)alkanoyl, (C₁₋₄)alkylamino(C₁₋₄)alkanoyl, di(C₁₋₄) alkylamino (C₁₋₄) alkanoyl, aroyl or heteroaroyl can be prepared by reacting a corresponding compound of Formula I in which R³, R⁴ or R⁵ is amino or aminomethyl with an appropriate acylating agent (e.g., acyl halides such as dimethylcarbamyl chloride, benzoyl chloride, nicotinoyl chloride, anhydrides such as acetic anhydride, activated esters such as methyl chloroformate) or a protected derivative thereof. The reaction is carried out in a suitable solvent (e.g., methylene chloride, THF, pyridine, water, any appropriate mixture of suitable solvents, preferably pyridine) at -10 to 40°C, typically at 15 to 35°C and preferably at approximately 25°C, and requires 0.5 to 8 hours (for further details see Example 35, infra.).

Alternatively, compounds of formula I in which R¹⁰ is carbamoyl, (C₁₋₄)alkyloxycarbonyl, (C₁₋₄)alkylcarbamoyl, di(C₁₋₄) alkylcarbamoyl, amino(C₁₋₄)alkanoyl, (C₁₋₄) alkylamino (C₁₋₄)alkanoyl, di(C₁₋₄)alkylamino (C₁₋₄)alkanoyl, aroyl or heteroaroyl can be prepared by reacting a corresponding compound of Formula I in which R³, R⁴ or R⁵ is amino or aminomethyl with an appropriate acid (e.g., picolinic acid and the like) or a protected derivative thereof (e.g., *N-*(*tert*-butoxycarbonyl)glycine). The reaction is carried out in the presence of a non-nucleophilic base (e.g., *N,N*-diisopropylethylamine (DIEA), *N,N*-dicyclohexylmethylamine, preferably DIEA) and a suitable coupling agent (e.g., PyBOP, 1,1'-carbonyldiimidazole, dicyclohexylcarbodiimide, preferably PyBOP) in a suitable solvent (e.g., DMF, DMPU, acetonitrile, THF, methylene chloride, any appropriate mixture of suitable solvents, preferably DMF) at -20 to 80°C, typically at 0 to 30°C and preferably at approximately 25°C (for further details see Example 36, infra.).

Alternatively, compounds of Formula I in which R³, or R⁵ is -NHR¹⁰ or compounds of Formula I in which R⁴ or R⁵ is -CH₂NHR¹⁰, wherein R¹⁰ is (C₁₋₄)alkylcarbamoyl, can be prepared by reacting a corresponding compound of Formula I in which R³, R⁴ or R⁵ is amino or aminomethyl with (C₁₋₄)alkyl isocyanate. The reaction is carried out optionally in the presence of a base (e.g., triethylamine, pyridine) in a suitable solvent (e.g., THF, benzene, methylene chloride, any appropriate mixture of suitable solvents, etc., preferably THF) at 0°C to reflux, typically at 25 to 80°C and preferably at approximately 50°C (for further details see Example 37, infra.).

Compounds of Formula I can be prepared as their individual stereoisomers by reacting a racemic mixture thereof with an optically active resolving agent to form a pair of diastereomeric compounds, separating the diastereomers and recovering the optically pure enantiomer. While resolution of enantiomers can be carried out using covalent diastereomeric derivatives of compounds of Formula I, dissociable complexes are preferred (e.g., crystalline diastereoisomeric salts). In that compounds of Formula I contain a basic amine group, such crystalline diastereoisomeric salts can be prepared by using a suitable optically active acid as the resolving agent (e.g., tartaric acid, mandelic acid, malic acid, the 2-arylpropionic acids in general, camphorsulfonic acid).

Diastereomers have distinct physical properties (e.g., melting points, boiling points, solubilities, reactivity) and can be readily separated by taking advantage of these dissimilarities. The diastereomers can be separated by chromatography or, preferably, by separation/resolution techniques based upon differences in solubility. The optically pure enantiomer is then recovered, along with the resolving agent, by any practical means that would not result in racemization. A more detailed description of the techniques applicable to the resolution of stereoisomers of compounds from their racemic mixtures can be found in Jean Jacques, Andre Collet, Samuel H. Wilen, Enantiomers, Racemates and Resolutions, John Wiley & Sons, Inc. (1981).

In summary, an aspect of this invention is a process for the preparation of a compound of Formula I: 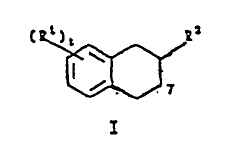 as defined above, and the pharmaceutically acceptable salts, individual isomers, and mixtures of isomers thereof; which process comprises:
(a) reacting a compound of Formula 3: 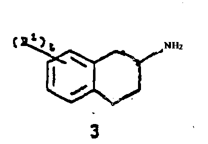 or an acid addition salt thereof in which each n, t and R¹ are as defined with respect to Formula I, with thiocyanic acid and dihydroxyacetone, followed by optionally treating the reaction mixture with sulfuric acid to give a compound of Formula I in which R³ and R⁴ are hydrogen and R⁵ is hydroxymethyl; or
(b) reacting a compound of Formula 9: 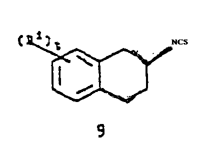 in which each t and R¹ are as defined above with respect to Formula I with aminoacetonitrile hydrochloride and then treating with base to give a compound of Formula I in which R¹ is a group of Formula (a) wherein R³ and R⁴ are each hydro and R⁵ is amino; or
(c) reacting a compound of Formula 9 with D-(+)-glucosamine and then oxidizing to give a compound of Formula I in which R² is a group of Formula (a) wherein R³ and R⁵ are each hydro and R⁴ is foxmyl; or
(d) reacting a compound of Formula 11: 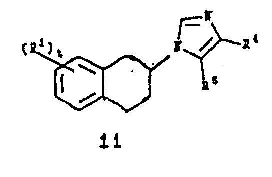 in which each t, R¹, R⁴ and R⁵ are as defined above with respect to Formula I with a strong base and then sulfurizing to give a compound of Formula I in which R³ is hydro; or
(e) reacting a compound of Formula 11 in which each t, R¹, R⁴ and R⁵ are as defined above with respect to Formula I with a compound of the formula L- (CH₂)_{q}R⁹ in which L is a leaving group and each q and R⁹ are as defined above with respect to Formula I and then sulfurizing to give a compound of Formula I in which R² is a group of Formula (a) wherein R³ is -(CH₂)_{q}R⁹; or
(f) reacting a compound of Formula 11 in which R⁴ and R⁵ are each hydro and each t, and R¹ are as defined above with respect to Formula I, with an amino aryl- or alkylsulfonate to give a compound of Formula I in which R² is a group of Formula (a) wherein R³ is amino; or
(g) alkylating a compound of Formula 11 in which R⁴ and R⁵ are each hydro and each t, and R¹ are as defined above with respect to Formula I, with an appropriately *N,N*-disubstituted methyleneaamonium salt and then sulfurizing to give a compound of Formula I in which R² is a group of Formula (a) wherein R⁵ is di(C₁₋₄)alkylaminomethyl, piperidin-1-ylmethyl or morpholin-4-ylmethyl; or
(h) reacting a compound of Formula 16: 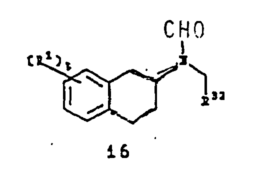 in which R³² is cyano or (C₁₋₄)alkyloxycarbonyl and each t and R¹ are as defined above with respect to Formula I with a compound of the formula R³³C(O)L and then treating with thiocyanic acid to give a compound of Formula I in which R² is a group of Formula (a) wherein R³ is hydro, R⁵ is cyano or (C₁₋₄)alkyloxycarbonyl and R⁴ is hydro, (C₁₋₄)alkyloxycarbonyl or (C₁₋₄)alkyl; or
(i) reacting a compound of Formula I in which R⁵ is hydroxymethyl with a compound of the formula H₂N(O)R³¹ or an ammonium salt of the formula NH₄⁺⁻OC(O)R³¹ (in which R³¹ is hydrogen, C₁₋₄ alkyl or trifluoro(C₁₋₄)alkyl) to give a compound of Formula I in which R⁵ is formylaminomethyl, C₁₋₄alkylcarbonyl-aminomethyl or trifluoro(C₁₋₄)alkylcarbonylaminomethyl, which compound can be further optionally hydrolized to give an acid addition salt of a compound of Formula (I) in which R³ and R⁴ are each hydro and R⁵ is aminomethyl; or
(j) reacting a compound of Formula I in which R⁵ is hydroxymethyl with urea to give a compound of Formula I in which R⁵ is ureidomethyl; or
(k) reacting a compound of Formula I in which R⁵ is cyano with a hydrazoic acid derivative to give the compound of Formula I in which R⁵ is 1*H*-tetrazol-5-yl; or
(l) reducing a compound of Formula I in which R⁵ is cyano to give a compound of Formula I in which R⁵ is aminomethyl; or
(m) hydrolyzing a compound of Formula I in which R⁵ is cyano to give the compound of Formula I in which R⁵ is carbamoyl; or
(n) reacting a compound of Formula I in which R⁵ is cyano with ethylenediamine to give the compound of Formula I in which R⁵ is 4,5-dihydroimidazol-2-yl; or
(o) reacting a compound of Formula I in which R⁵ is cyano with a compound of the formula (CH₃)₂AlNR¹⁵R¹⁶ to give the compound of Formula I in which R⁵ is -C(NH)NR¹⁵R¹⁶, wherein R¹⁵ and R¹⁶ are as defined above with respect to Formula I; or
(p) reducing a compound of Formula I in which R⁵ or both R⁴ and R⁵ are ethoxycarbonyl to give a compound of Formula I in which R⁵ or both R⁴ and R⁵ are hydroxymethyl; or
(q) converting a compound of Formula I in which R⁵ is hydroxymethyl to a compound of Formula 28: 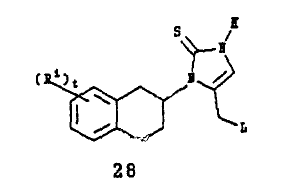 in which L is a leaving group and t and R¹ are as defined in the Summary of the Invention with respect to Formula I and reacting the compound of Formula 28 with an amine of the formula HNR³⁶R³⁷ in which R³⁶ and R³⁷ are independently (C₁₋₄)alkyl or together are -(CH₂)₄-, -(CH₂)₅-, -(CH₂)₂O(CH₂)₂- or -(CH₂)₂NR³⁸(CH₂)₂-, wherein R³⁸ is hydro or (C₁₋₄)alkyl, to give a compound of Formula I in which R⁵ is aminomethyl, (C₁₋₄)alkylaminomethyl, di(C₁₋₄)alkylaminomethyl, pyrrolidin-1-ylmethyl, piperidin-1-ylmethyl, morpholin-4-ylmethyl, piperazin-1-ylmethyl or 4-(C₁₋₄)alkylpiperazin-1-ylmethyl; or
(r) reacting a compound of Formula I in which R⁴ is formyl with hydroxylamine hydrochloride and then reducing to give a compound of Formula I in which R⁴ is aminomethyl; or
(s) reacting a compound of Formula I in which R⁴ is formyl with an amine of the formula NH₂R¹⁰ in the presence of a chemical reducing agent or catalytic hydrogenation to give a compound of Formula I in which R⁴ is -CH₂NHR¹⁰, wherein R¹⁰ is as defined above with respect to Formula I; or
(t) alkylating a compound of Formula I in which R⁴ is formyl to give a compound of Formula I in which R⁴ is 1-hydroxy(C₁₋₄)alkyl; or
(u) reacting a compound of Formula I in which R³ or R⁵ is amino or R⁴ or R⁵ is aminomethyl with an appropriately substituted amidine to give a corresponding compound of Formula I in which R³ or R⁵ is -NHC(NR¹¹)NHR¹² or R⁴ or R⁵ is -CH₂NHC(NR¹¹)NHR¹² wherein R¹¹ is hydro, acetyl or *tert*-butoxycarbonyl and R¹² is acetyl or *tert*-butoxycarbonyl; or
(v) treating a compound of Formula I in which R³ or R⁵ is -NHC(NR¹¹)NHR¹² or R⁴ or R⁵ is -CH₂NHC(NR¹¹)NHR¹², wherein R¹¹ is hydro, acetyl or *tert*-butoxycarbonyl and R¹² is acetyl or *tert*-butoxycarbonyl, with acid to give a compound of Formula I in which R³ or R⁵ is -NHC(NH)NH₂ or R⁴ or R⁵ is -CH₂NHC(NH)NH₂; or
(w) acylating a compound of Formula I in which R³ or R⁵ is amino or R⁴ or R⁵ is aminomethyl with an appropriate acylating agent, or a protected derivative thereof, and then deprotecting when necessary to give a corresponding compound of Formula I in which R³ or R⁵ is -NHR¹⁰ or R⁴ or R⁵ is -CH₂NHR¹⁰, wherein R¹⁰ is (C₁₋₄)alkanoyl, trifluoro(C₁₋₄)alkanoyl, carbamoyl, (C₁₋₄)alkyloxycarbonyl, (C₁₋₄)alkylcarbamoyl, di(C₁₋₄)alkylcarbamoyl, amino(C₁₋₄)alkanoyl, (C₁₋₄)alkylamino (C₁₋₄)alkanoyl, di(C₁₋₄)alkylamino(C₁₋₄)alkanoyl or a group selected from aroyl and heteroaroyl, (which aroyl and heteroaroyl are optionally further substituted with one to two substituents independently selected from (C₁₋₄)alkyloxy, cyano, carboxy and (C₁₋₄)alkyloxycarbonyl); or
(x) reacting a compound of Formula I in which R³ or R⁵ is amino or R⁴ or R⁵ is aminomethyl with (C₁₋₄)alkyl isocyanate to give a compound of Formula I in which R³ or R⁵ is -NHR¹⁰ or R⁴ or R⁵ is -CH₂NHR¹⁰, wherein R¹⁰ is (C₁₋₄)alkylcarbamoyl; or
(y) alkylating a compound of Formula I in which R³, R⁴ and R⁵ are each hydro with an appropriately *N,N*-disubstituted methyleneammonium salt to give a compound of Formula I in which R³ and R⁵ are each hydro and R⁴ is di(C₁₋₄)alkylaminomethyl, piperidin-1-ylmethyl or morpholin-4-ylmethyl; or
(z) alkylating a compound of Formula I in which R⁴ is hydro and R³ is other than hydro with an appropriately *N,N*-disubstituted methyleneammonium salt to give a corresponding compound of Formula I in which R⁵ is di(C₁₋₄)alkylaminomethyl, piperidin-1-ylmethyl or morpholin-4-ylmethyl; or
(aa) protecting a compound of Formula I in which R³ is hydro with a thiol protective group, alkylating with an appropriately *N,N*-disubstituted methyleneammonium salt and then deprotecting to give a compound of Formula I in which R³ and R⁴ are each hydro and R⁵ is di(C₁₋₄)alkylaminomethyl, piperidin-1-ylmethyl or morpholin-4-ylmethyl or in which R³ is hydro and both R⁴ and R⁵ are di(C₁₋₄)alkylaminomethyl, piperidin-1-ylmethyl or morpholin-4-ylmethyl; or
(bb) reacting a compound of Formula I in which R⁵ or both R⁴ and R⁵ are carboxy, or an acid derivative thereof, with an appropriate amine or thiol to give a compound of Formula I in which R⁵ or both R⁴ and R⁵ are 1*H*-tetrazol-5-ylcarbamoyl, 2-(dimethylamino)ethylcarbamoyl, 4-methylpiperazin-1-ylcarbonyl or 2-(dimethylamino)ethylmercapto; or
(cc) reacting a compound of Formula I in which R³ is hydro with (C₁₋₄)alkyl acrylate to give a compound of Formula I in which R³ is 2-(C₁₋₄)alkyloxycarbonylethyl; or
(dd) hydrolyzing a compound of Formula I in which R³, R⁴ or R⁵ is (C₁₋₄)alkyloxycarbonyl or a group which is further substituted by a (C₁₋₄)alkyloxycarbonyl substituent to give a compound of Formula I in which R³, R⁴ or R⁵ is carboxy or group which is further substituted by a carboxy substituent; or
(ee) aminating a compound of Formula I in which R³, R⁴ or R⁵ is carboxy or a group which is further substituted by a carboxy substituent to give a compound of Formula I in which R³, R⁴ or R⁵ is carbamoyl or a group which is further substituted by a carbamoyl substituent; or
(ff) reacting a compound of Formula I in which R³, R⁴ or R⁵ is carboxy or a group which is further substituted by a carboxy group with a (C₁₋₄) alcohol to give a compound of Formula I in which R³, R⁴ or R⁵ is (C₁₋₄) alkyloxycarbonyl or a group that is further substituted by a (C₁₋₄)alkylaxycarbonyl group; or
(gg) de-methylating a compound of Formula I in which R¹ is methoxy and/or in which R³, R⁴ or R⁵ is a group selected from aroyl, heteraroyl, aryl(C₁₋₄)alkyl and heteroaryl(C₁₋₄)alkyl (which aroyl, heteroaroyl, aryl and heteroaryl are further substituted with one to two methoxy substituents) to give a compound of Formula I in which R¹ is hydroxy and/or R³, R⁴ or R⁵ is a group selected from aroyl, heteraroyl, aryl(C₁₋₄)alkyl and heteroaryl(C₁₋₄)alkyl (which aroyl, heteroaroyl, aryl and heteroaryl are further substituted with one to two hydroxy substituents); or
(hh) reacting a compound of Formula I in which R³ or R⁵ is -NHR¹⁰ or in which R⁴ or R⁵ is -CH₂NHR¹⁰, wherein R¹⁰ is a group selected from aroyl, heteroaroyl, aryl(C₁₋₄)alkyl and heteroaryl(C₁₋₄)alkyl (which aroyl, heteroaroyl, aryl and heteroaryl are further substituted with a cyano substituent) with a hydrazoic acid derivative to give a compound of Formula I in which R¹⁰ is a group selected from aroyl, heteroaroyl, aryl(C₁₋₄)alkyl and heteroaryl(C₁₋₄)alkyl (which aroyl, heteroaroyl, aryl and heteroaryl are further substituted with a 1*H*-tetrazol-5-yl substituent; or
(ii) reacting the corresponding non-salt form of a compound of Formula I with a pharmaceutically acceptable inorganic or organic acid or base to give a pharmaceutically acceptable salt; or
(jj) reacting the corresponding acid addition salt or base addition salt form of a compound of Formula I with a suitable base or acid, respectively, to give the free acid or free base; or
(kk) separating a mixture of stereoisomers of a compound of Formula I to give a single stereoisomer.

### Compounds of Formula 3:

A preferred method for making a compound of Formula 3 as the individual (*S*)-enantiomer is depicted by the following Reaction Scheme X: 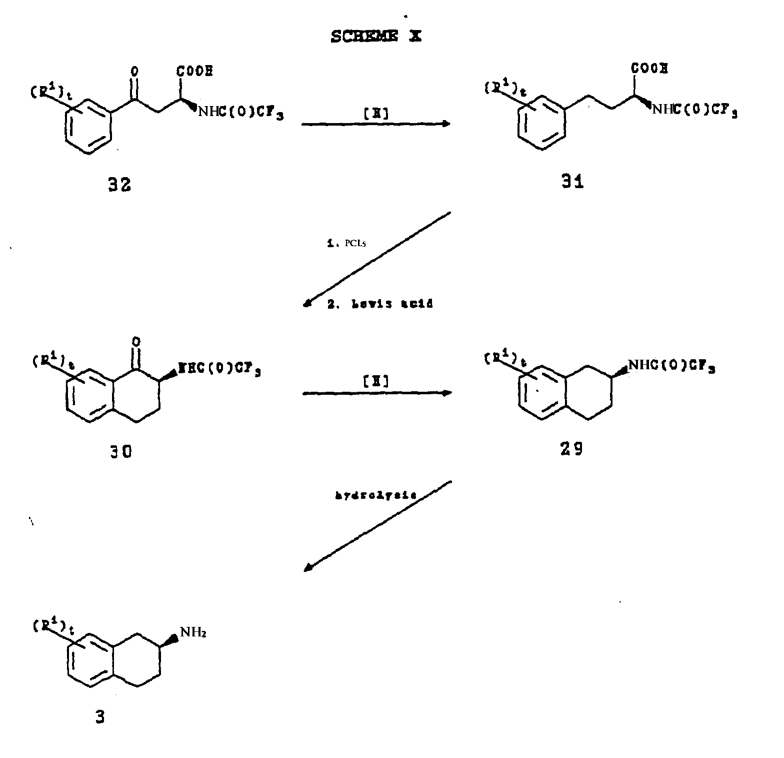 in which each t and R¹ are as defined in the Summary of the Invention with respect to Formula I.

Compounds of Formula 3 can be prepared as the individual (*S*)-enantiomer (Formula 3(a)) by hydrolyzing a compound of Formula 29. The hydrolysis can be carried out with an aqueous base (e.g., lithium hydroxide monohydrate, sodium hydroxide, potassium hydroxide, potassium carbonate, preferably lithium hydroxide monohydrate) in a suitable solvent, typically an alcohol (e.g., methanol, ethanol, isopropanol) and preferably methanol, at 25 to 100°C, preferably reflux, and requires 0.5 to 5 hours.

Compounds of Formula 29 are prepared by hydrogenolysis of a compound of Formula 30. The hydrogenolysis can be accomplished by a two step process comprising (i) hydrogenating a compound of Formula 30 until conversion to the corresponding 1-naphthol is complete and (ii) adding sulfuric acid and continuing hydrogenation to give the compound of Formula 29. Hydrogenation of the compound of Formula 30 to the 1-naphthol is carried out in the presence of an appropriate catalyst (e.g., Pearlman's catalyst, palladium on carbon, etc., preferably Pearlman's catalyst) in acetic acid or trifluoroacetic acid (TFA), preferably acetic acid, at 1 to 130 psig and 10 to 30°C and requires 0.5 to 4 hours. Hydrogenation of the 1-naphthol is carried out by adding 1 to 10 equivalents, preferably 4 to 6 equivalents, of sulfuric acid or perchloric acid and continuing the hydrogenation under the same conditions for 3 to 120 hours. Alternatively, the hydrogenolysis is effected by a single step process comprising hydrogenating the compound 30 in the presence of an appropriate catalyst and either sulfuric acid or perchloric acid in acetic acid. The single step hydrogenolysis is carried out at 1 to 130 psig and 10 to 30°C and requires 3 to 120 hours.

Compounds of Formula 30 are prepared from compounds of Formula 31 by an intramolecular Friedel-Crafts reaction. The reaction is effected by converting a compound of Formula 31 to the corresponding acid chloride and then treating the acid chloride with an appropriate Lewis acid (e.g. aluminum chloride, hydrogen fluoride, etc., preferably aluminum chloride) to give the compound of Formula 30. Conversion to the acid chloride can be carried out with an appropriate chlorinating agent (e.g., phosphorus pentachloride, thionyl chloride, oxalyl chloride, preferably phosphorus pentachloride) in methylene chloride at 0 to 10°C, preferably at 5 to 10°C, and requires 0.5 to 2 hours. The treatment with Lewis acid and the resultant ring closure is carried out at 0 to 10°C, preferably at 5 to 10°C, and requires 1 to 3 hours. Preferably, the crude product is isolated by crystallization from a methanol/water or isopropanol/water mixture; and then, if necessary, recrystallization from a toluene/heptane mixture.

Compounds of Formula 31 are prepared by hydrogenolysis of a compound of Formula 32. The hydrogenolysis is carried out by hydrogenating in the presence of an appropriate catalyst (e.g., 20% palladium hydroxide on carbon (Pearlman's catalyst), palladium on carbon, preferably Pearlman's catalyst) and 1 to 5 equivalents, preferably 1.5 to 2 equivalents, of sulfuric acid, in a glacial acetic acid at 1 to 60 psig and 5 to 30°C and requires 2 to 48 hours.

Compounds of Formula 32 are prepared via a Friedel-Crafts alkylation of optionally substituted benzene with *N*-(trifluoroacetyl)-L-aspartic anhydride. The alkylation is carried out in the presence of a Lewis acid (e.g., aluminum chloride, tin chloride, hydrogen fluoride, preferably aluminum chloride) and in a suitable solvent (e.g., methylene chloride, preferably methylene chloride) at 25 to 40°C, preferably at reflux, and requires 2 to 5 hours.

*N*-(Trifluoroacetyl)-L-aspartic anhydride is prepared by reacting L-aspartic acid with trifluoroacetic anhydride (TFAA). The reaction is highly exothermic and when more than 100 g amounts of reactants are employed, it must be conducted under conditions such that the liberation of heat is controlled. For example, a convenient method for carrying out the reaction is by heating 2 to 4 equivalents, preferably 2.3 to 2.5 equivalents, of TFAA to between 30 and 40°C, preferably to reflux, and then adding 1 equivalent of L-aspartic acid at a rate such that the reaction readily proceeds but the heat generated by the reaction can be dissipated by reflux. Preferably the L-aspartic acid is added to the TFAA as a solution in TFA over 30 to 60 minutes.

Proceeding as described in Reaction Scheme XIII but substituting D-aspartic acid for L-aspartic acid, the (*R*)-enantiomer of the compound of Formula 3(a) can be prepared. Further details of the reaction steps set forth above for Reaction Scheme XII are provided in Example 4.

### Compounds of Formula 6:

Compounds of Formula 6 can be prepared by reducing a corresponding compound of Formula 7. The reduction is carried out with a chemical reducing agent such as sodium borohydride in a suitable solvent, typically an alcohol (e.g., ethanol, methanol, isopropanol, any appropriate mixture of suitable alcohols) or lithium aluminum hydride (LAH) in a suitable solvent (e.g., diethyl ether, THF, 1,2-dimethoxyethane, any appropriate mixture of suitable solvents) at 0 to 80°C and requires 1 to 2 hours (for further details see Reference Example 1, infra.).

An alternative method for making compounds of Formula 6 is depicted by the following Reaction Scheme XI: 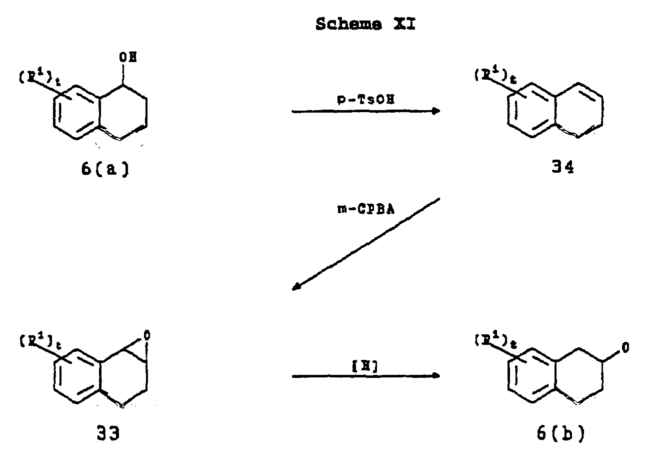 in which each t and R¹ is as defined in the Summary of the Invention with respect to Formula I.

Compounds of Formula 6(b) can be prepared by reacting a compound of Formula 34 with 3-chloroperoxybenzoic acid (*m*-CPBA) to give an epoxide of Formula 33 and then reducing the epoxide to give the corresponding β-alcohol. The reaction with *m*-CPBA is carried out in a suitable solvent (e.g., benzene, methylene chloride, chloroform) at 0 to 20°C, preferably approximately 0°C, and requires 0.5 to 5 hours. Reduction of the epoxide can be effected by catalytic hydrogenation (e.g., H₂, 10% palladium on carbon; ) in a suitable solvent (e.g., ethyl acetate, ethanol, isopropanol).

Compounds of Formula 34 can be prepared by reacting an α-alcohol of Formula 6(a) with *p*-toluenesulfonic acid in a suitable solvent (e.g., benzene, toluene, dichloroethane, methylene chloride) at 20 to 110°C, typically at 60 to 100°C and preferably at approximately 80°C, for 1 to 5 hours. Further details of the reaction steps set forth in this and the preceding paragraph are provided in Example 2.

Compounds of Formula I can be prepared as their individual stereoisomers from the individual stereoisomers of starting material. The starting material can be prepared as individual stereoisomers by any of the resolution techniques described above or by any method known to one of ordinary skill in the art. For example, compounds of Formula 6 can be prepared as their individual stereoisomers by kinetic enzymatic resolution with a suitable enzyme (e.g., porcine pancreatic lipase, candida cylindracea, pancreatin).

Alternatively, certain starting materials in the process for preparing compounds of Formula I can be prepared as their individual stereoisomers by chiral synthesis. For example, compounds of Formula 6 can be prepared as their individual stereoisomers by chiral reduction of the corresponding compound of Formula 7.

Compounds of Formula 6, can be prepared as the (*R*)-enantiomer by reducing the corresponding compound of Formula 7 with lithium aluminum hydride in the presence of (1*R*,2*S*)-*N*-methylephedrine and 2-ethylaminopyridine. The reaction is carried out in diethyl ether at -78 to -65°C, preferably approximately -78°C, and requires 2 to 3 hours (for further details see Example 3, infra.). Similarly, the (*S*)-enantiomer can be prepared by reducing the compound of Formula 7 in the presence of (1*S*,2*R*)-*N*-methylephedrine and 2-ethylaminopyridine.

### Compounds of Formula 7:

Compounds of Formula 7 are available commercially or can be readily made by those of ordinary skill in the art. For example, suitable compounds of Formula 7 can be obtained can be prepared by oxidizing an available compound of Formula 6. The oxidation of the compound of Formula 6 can be effected with an appropriate oxidizing agent (e.g., Dess-Martin reagent) in a suitable solvent (e.g., THF, methylene chloride) at 20 to 50°C.

A method for making compounds of Formula 7 is depicted by the following Reaction Scheme XIII: 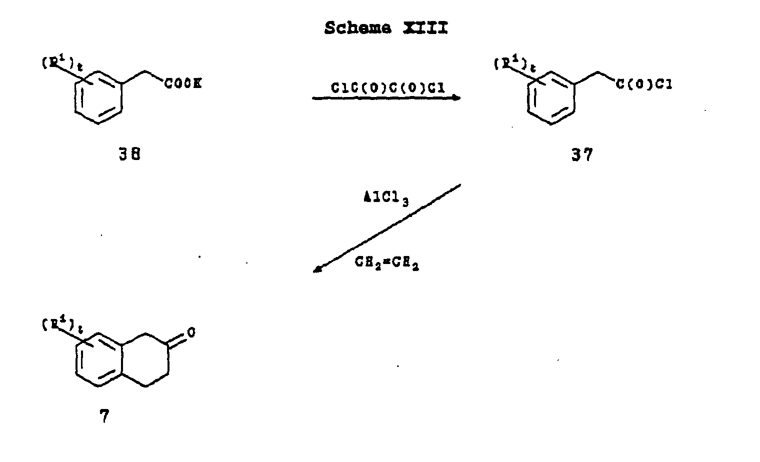 in which each t and R¹ is as defined in the Summary of the Invention with respect to Formula I.

Compounds of Formula 7 can be prepared by converting a compound of Formula 38 to the corresponding acid chloride (Formula 37) and then reacting the acid chloride with ethylene in the presence of a Lewis acid (e.g., aluminum chloride, boron trifluoride, aluminum bromide). The conversion to the acid chloride is carried out with an appropriate chlorinating agent (e.g., thionyl chloride, oxalyl chloride, phosphorus pentachloride) and in a suitable solvent (e.g., methylene chloride, dichloroethane, any appropriate mixture of suitable solvents) at 20 to 40°C, typically at 20 to 30°C and preferably at approximately 20°C, and requires 2 to 18 hours. The reaction with ethylene is carried out in a suitable solvent (e.g., methylene chloride, carbon disulfide, any appropriate mixture of suitable solvents) and by adding the acid chloride to the Lewis acid at a rate such that the reaction mixture remains below -40°C, preferably below -60°C, and then bubbling the mixture with ethylene gas for 0.1 to 0.5 hours at -78 to -40°C, typically at -60 to -78°C and preferably at approximately -78°C. Further details of the reaction steps set forth in this paragraph are provided in Example 3.

### Preparation of Compounds of Formula II:

A method for making compounds of Formula II in which R²¹ is -CH₂NR²⁵R²⁶ is depicted by the following Reaction Scheme XVI: 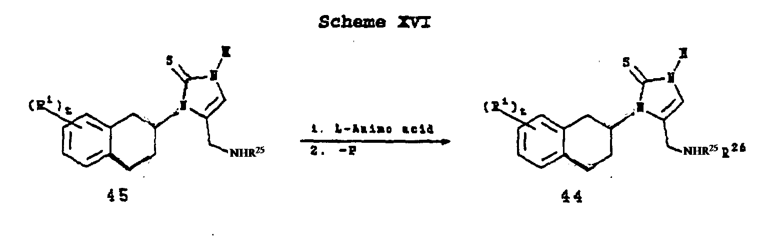 in which each t, R¹, R²⁵ and R²⁶ are as defined in the Summary of the Invention with respect to Formula II.

Compounds of Formula II in which R²¹ is -CH₂NR²⁵R²⁶ (Formula 44) can be prepared by reacting a compound of Formula I(a) in which R⁵ is -CH₂NHR²⁵ (Formula 45) with a protected derivative of an L-amino acid in a suitable solvent (e.g., DMF, methylene chloride, THF, any appropriate mixture of suitable solvents, etc.) and then removing all protective groups present. The reaction with the L-amino acid is carried out in the presence of a non-nucleophilic base (e.g., DIEA, *N,N*-dicyclohexylmethylamine, etc.) and a peptide coupling agent (e.g., PyBOP, benzotriazol-1-yloxytris(dimethylamino)-phosphonium hexafluorophosphate, *N,N*-dicyclohexylcarbodimide, bromotris(pyrrolidin-1-yl)phosphonium hexafluorophosphate) at 20 to 80°C, preferably approximately 20°C, and requires 8 to 24 hours (for further details see Example 42, infra.).

Protected L-amino acids are prepared by reacting an appropriate L-amino acid with a suitable protecting agent (e.g., di-*tert*-butyldicarbonate, 9-fluorenylmethylsuscinimidylcarbonate). For example, the protected derivative of L-lysine is prepared by reacting L-lysine with di-*tert*-butyldicarbonate in a suitable solvent (e.g., THF, DMF, any appropriate mixture of suitable solvents). The reaction is carried out at 0 to 20°C, preferably at approximately 20°C, and requires 8 to 48 hours. Removal of the protective groups can be effected by acid hydrolysis (e.g., 15% hydrogen chloride) in a suitable solvent (e.g., ethyl acetate, 1,2-dimethoxyethane, any appropriate mixture of suitable solvents). 9-Fluorenylmethoxycarbonyl protective groups can be removed with piperidine in DMF. Carbobenzyloxy protective groups can be removed by hydrogenating with palladium on carbon.

Proceeding similarly, compounds of Formula II in which R²⁰ is -CH₂NR²⁵R²⁶ are prepared from compounds of Formula I(a) in which R⁴ is -CH₂NHR²⁵. Compounds of Formula II in which R²¹ is -NR²⁵R²⁶ are prepared from compounds of Formula I (a) in which R⁵ is -NHR²⁵. Compounds of Formula II in which R¹⁹ is -NR²⁵R²⁶ can be prepared from a compound of Formula I(a), wherein R³ is -NHR²⁵.

### Preparation of Compounds of Formula III:

A method for making compounds of Formula III is depicted by the following Reaction scheme XVII: 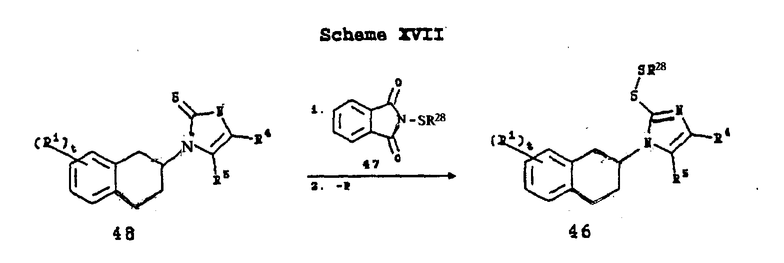 in which each t, R¹ and R²⁸ are as defined in the Summary of the Invention with respect to Formula III.

Compounds of Formula III can be prepared by reacting a compound of Formula I (a) wherein R³ is hydro (Formula 48) with a compound of Formula 47, or a protected derivative thereof, in a suitable solvent (e.g., ethyl acetate, ethylenedichloride, THF, any appropriate mixture of suitable solvents.) and then removing all protective groups present. The reaction with the compound of Formula 47 is carried out at 20 to 110°C, preferably approximately 80°C, for 1 to 4 hours.

Compounds of Formula 47 can be prepared by reacting a compound of the formula X-SR²⁸, in which X is halo, or the protected derivatives thereof, with potassium phthalimide in a suitable solvent (e.g., ethylenedichloride, carbon tetrachloride, 1,1,1,2-tetrachloroethane). The reaction is carried out at -30 to 20°C, preferably approximately -20°C, and requires 1 to 3 hours. Compounds of the formula X-SR²⁸ can be readily prepared by those of skill in the art. For example, a compound of the formula X-SR²⁸ in which X is bromo and R²⁸ is 2-tert-butoxycarbonyl-2-(*tert*-butyloxycarbonylamino)ethyl can be prepared by reacting *N*-(*tert*-butoxycarbonyl)cystine with bromine in a suitable solvent (e.g., dichloroethane). The reaction is carried out at -40 to 20°C, preferably at approximately -23°C, and requires 1 to 3 hours. Further details of the reaction steps set forth by Reaction Scheme XVII is provided in Reference Example 4, infra..

### Reference Example 1

### 5,6-Difluoro-1-hydroxyindane

3-(3,4-Difluorophenyl)propionic acid (80.0 g, 0.43 mol) was dissolved in 3 drops of DMF and 350 mL of methylene chloride. Oxalyl chloride (75 mL, 0.860 mol) was added and the mixture was stirred under nitrogen at room temperature for approximately 3 hours. Excess oxalyl chloride was removed by evaporation and the residue was co-evaporated twice with 200 mL of carbon tetrachloride giving 95.0 g of residue as an oil.

Aluminum chloride (200 g) was suspended in 800 mL of carbon disulfide and the suspension was cooled to 0°C. A solution of the residue in 300 mL of carbon disulfide was added to the suspension over 20 minutes and the mixture was stirred at reflux for 4 hours. The mixture was poured onto 2 kg of crushed ice and the aqueous layer was extracted ethyl acetate (3 x 300 mL). The carbon disulfide layer and ethyl acetate extract were both dried over magnesium sulfate, filtered and then combined. The solvents were removed by evaporation and the residue was crystallized from isopropyl ether giving 54.3 g of 5,6-difluoroindan-1-one as a solid. The mother liquor was purified by column chromatography on silica gel (elution: 10% ethyl acetate/hexane) giving an additional 5.2 g of 5,6-difluoroindan-1-one.

5,6-Difluoroindan-1-one (59.3 g, 0.353 mol) was dissolved in 1 L of ethanol and sodium borohydride (6.68 g, 0.176 mol) was added. The mixture was stirred for 48 hours and the ethanol was removed by evaporation under reduced pressure. The residue was partitioned between diethyl ether and water and the mixture was acidified with 1N hydrochloric acid. The mixture was dried over magnesium sulfate and filtered. The solvents were evaporated giving 5,6-difluoro-1-hydroxyindane (59.8 g) as an oil.

### Example 1

Proceeding as in Reference Example 1 but substituting a different starting material for 3-(3,4-difluorophenyl)propionic acid, the following compounds of Formula 6 were made:
substituting 4-(2,4-difluorophenyl)butyric acid gave 5,7-difluoro-1,2,3,4-tetrahydro-1-hydroxynaphthalene as an oil;
substituting 4-(3,4-difluorophenyl)butyric acid gave 6,7-difluoro-1,2,3,4-tetrahydro-1-hydroxynaphthalene as an oil;
substituting 4-(4-fluorophenyl)butyric acid gave 7-fluoro-1,2,3,4-tetrahydro-1-hydroxynaphthalene as an oil;
substituting 4-(3-fluorophenyl)butyric acid gave 6-fluoro-1,2,3,4-setrahydro-1-hydroxynaphthalene as an oil; and
substituting 4-(3,5-difluorophenyl)butyric acid gave 6,8-difluoro-1,2,3,4-tetrahydro-1-hydroxynaphthalene, m.p. 102-103°C.

### Reference Example 2

### 5,6-difluoro-2-hydroxyindane

5,6-Difluoro-1-hydroxyindane (59.8 g, 356 mmol), prepared as in Example 1, was dissolved in 600 mL of benzene and *p*-toluenesulfonic acid monohydrate (3.36 g, 17.66 mmol) was added. The mixture was heated to 120°C, distilled for approximately 2 hours and then allowed to cool. Saturated sodium bicarbonate solution was added and the benzene layer was dried over magnesium sulfate. The benzene layer was then filtered and the filter residue was washed with diethyl ether.

The benzene/diethyl ether mixture was cooled to 0°C and *m*-CPBA (75.5 g, 0.35 mol) was added over 15 minutes. The mixture was stirred at room temperature for 5 hours. The diethyl ether was removed by rotary evaporation, 400 mL of methylene chloride was added and the mixture was stirred for 2 hours. Additional *m*-CPBA (30.0 g, 0.14 mol) was added and the mixture was stirred for 2 hours. The mixture was cooled to 0°C and potassium iodide (45 g) in 150 mL of water was added. The mixture was stirred for approximately 15 minutes and then sodium thiosulfate (40 g) in 150 mL of water was added. Kugelrohr distillation (bp 60-90°C [0.1 mm])) gave 30 g of impure 1,2-epoxy-5,6-difluoroindane as an oil.

Impure 1,2-epoxy-5,6-difluoroindane (4.5 g) was dissolved in 100 mL of ethanol and hydrogenated over 10% palladium on carbon (450 mg) for 15 hours. The mixture was filtered and evaporation gave 5,6-difluoro-2-hydroxyindane (2.72 g, 15.9 mmol), m.p. 57-58°C.

### Example 2

Proceeding as in Reference Example 2 but substituting a different starting material for 5, 6-difluoro-1-hydroxyindane, the following compounds of Formula 6 were made:
substituting 5,7-difluoro-1,2,3,4-tetrahydro-1-hydroxynaphthalene gave 5,7-difluoro-1,2,3,4-tetrahydro-2-hydroxynaphthalene, m.p. 93-94°C;
substituting 6,7-difluoro-1,2,3,4-tetrahydro-1-hydroxynaphthalene gave 6,7-difluoro-1,2,3,4-tatrahydro-2-hydroxynaphthalene as an oil; and
substituting 6,8-difluoro-1,2,3,4-tetrahydro-1-hydroxynaphthalene gave 6,8-difluoro-1,2,3,4-tetrahydro-2-hydroxynaphthalene.

### Example 3

### (R)-5,7-Difluoro-1,2,3,4-tetrahydro-2-hydroxynaphthalene

The following is the preparation of a compound of Formula 6 in which t is 2 and R¹ is fluoro at the 5- and 7-position.

A mixture 3,5-difluorophenylacetic acid (100 g, 0.58 mmol) and thionyl chloride (13.7 M, 100 mL, 1.37 mol) was stirred for 15 hours at room temperature. Evaporation gave 3,5-difluorophenylacetyl chloride as an oily residue. A stirred suspension of aluminum chloride (154 g, 1.16 mmol) in 1 L of methylene chloride was cooled to -65°C and the acid chloride in 200 mL of methylene chloride was added dropwise such that the reaction temperature did not exceed -60°C. Ethylene gas was bubbled through the suspension at a rapid rate for 10 minutes at -65°C. The reaction mixture was allowed to warm to 0°C over 2 hours, then cooled to -10°C and treated with 500 mL of water. The organic layer was separated, washed with 100 mL of aqueous sodium chloride, and then dried over magnesium sulfate. The mixture was filtered and the filtrate was concentrated under reduced pressure. Distillation of the residue *in vacuo* (bp 90-110°C [1.0 to 0.7 mm]) gave a clear distillate. Redistillation (bp 100-105°C [0.3 mm]) gave 5,7-difluoro-1,2,3,4-tetrahydronaphthalen-2-one (73.56 g, 0.342 mol) as a white solid, m.p. 46°C.

A solution of (1*R*,2*S*)-*N*-methylephedrine (81.3 g, 0.454 mol) in 1.2 L of anhydrous diethyl ether was added to lithium aluminum hydride (1.0 M in diethyl ether, 416 mL, 0.416 mol) over 45 minutes. The mixture was heated under reflux for 1 hour and then allowed to cool to room temperature. A solution of 2-ethylaminopyridine (110.7 g, 0.907 mol) in 100 mL of anhydrous diethyl ether was added over 45 minutes. The mixture was heated under reflux for 1 hour and then cooled to -65°C. A solution of 5,7-difluoro-1,2,3,4-tetrahydro-naphthalen-2-one (23.0 g, 0.107 mol) in 100 mL of diethyl ether was added dropwise such that the reaction temperature did not exceed -60°C. The mixture was stirred at -65°C to -68°C for 3 hours and then 100 mL of methanol was added such that the reaction temperature did not exceed -60°C. The mixture was stirred at -65°C to -68°C for 10 minutes, then allowed to warm to -20°C, and 3.0 L of 3N hydrochloric acid was added such that the reaction temperature did not exceed 35°C. The diethyl ether layer was separated, washed with 200 mL of saturated sodium chloride, and dried over magnesium sulfate. The mixture was filtered and the filtrate concentrated under reduced pressure. Crystallization from 20 mL of diethyl ether and 200 mL of hexane and drying *in vacuo* gave (*R*)-5,7-difluoro-1,2,3,4-tetrahydro-2-hydroxynaphthalene (10.87 g, 0.05 mol), m.p. 85°C. [α]_{D}²⁵ = +36.03° (c = 1.59, CHCl₃).

Proceeding as in Example 3 but substituting a different starting material for 3,5-difluorophenylacetic acid, the following compounds of Formula 6 were made:
substituting 3-fluorophenylacetic acid gave (*R*)-7-fluoro-1,2,3,4-tetrahydro-2-hydroxynaphthalene, m.p. 59-61°C, [α]_{D}²⁵ = +48.8° (c = 2.0, CHCl₃) ;
substituting 4-fluorophenylacetic acid gave (*R*)-6-fluoro-1,2,3,4-tetrahydro-2-hydroxynaphthalene, [α]_{D}²⁵ = +49.2° (c = 1.84, CHCl₃); substituting phenylacetic acid gave (*R*)-1,2,3,4-tetrahydro-2-hydroxynaphthalene, as an oil, [α]_{D}²⁵ = +62.2° (c = 1.6, CHCl₃); and
substituting 3,4-difluorophenylacetic acid gave (*R*)-6,7-difluoro-1,2,3,4-tetrahydro-2-hydroxynaphthalene, m.p. 88-92°C, [α]_{D}²⁵ = +39.0° (c = 2.2, CHCl₃).

Proceeding as in Example 3 but substituting (1*S*,2*R*)-*N*-methylephedrine for (1*R*,2*S*)-*N*-methylephedrine, gave (*S*)-5,7-difluoro-1,2,3,4-tetrahydro-2-hydroxynaphthalene, m.p. 84-85°C, [α]_{D}²⁵ = -37.4° (c = 2.6, CHCl₃).

Proceeding as in Example 3 but substituting a different starting material for 3,5-difluorophenylacetic acid and reducing without (1*R*,2*S*)-*N*-methylephedrine or 2-ethylaminopyridine present, the following compounds of Formula 6 were made:
substituting 4-bromophenylacetic acid gave 6-bromo-1,2,3,4-tetrahydro-2-hydroxynaphthalene as an oil;
substituting 4-chlorophenylacetic acid gave 6-chloro-1,2,3,4-tetrahydro-2-hydroxy-naphthalene as an oil;
substituting 3-chlorophenylacetic acid gave 7-chloro-1,2,3,4-tetrahydro-2-hydroxynaphthalene, m.p. 79-81°C;
substituting 3,5-dichlorophenylacetic acid gave 5,7-dichloro-1,2,3,4-tetrahydro-2-hydroxynaphthalene, m.p. 84-86°C;
substituting 3,5-difluorophenylacetic acid gave 5,7-difluoro-1,2,3,4-tetrahydro-2-hydroxynaphthalene;
substituting 3,4-difluorophenylacetic acid gave 6,7-difluoro-1,2,3,4-tetrahydro-2-hydroxynaphthalene,;
substituting 3,4-dichlorophenylacetic acid gave 6,7-dichloro-1,2,3,4-tetrahydro-2-hydroxynaphthalene, m.p. 103-105°C; and
substituting 4-fluorophenylacetic acid gave 6-fluoro-1,2,3,4-tetrahydro-2-hydroxynaphthalene.

### Example 4

### (S)-5,7-Difluoro-1,2,3,4-tetrahydronaphthalen-2-ylamine hydrochloride

The following is the preparation of a compound of Formula 3 in which t is 2 and R¹ is fluoro at the 5- and 7-position.
(a) Trifluoroacetic anhydride (7.7 kg, 5.3 L, 37.5 mol) was heated to reflux and a solution of L-aspartic acid (2.0 kg, 15.0 mol) in 9 L of trifluoroacetic acid (prepared by gradually heating to 65°C and stirring for 3 hours) was added to the refluxing TFAA over 30 minutes. The mixture then was distilled and 9 L of TFA was removed. The remaining mixture was added to 8 L of cold hexane under nitrogen. The hexane mixture was stirred for 3 hours in an ice bath giving a crystalline material. The material was isolated by filtration and the filtered residue was washed with approximately 25 L of hexane. Drying to constant weight in a vacuum oven at 50°C under a nitrogen gas bleed gave *N*-(trifluoroacetyl)-L-aspartic anhydride (2.9 kg, 13.7 mol), m.p. 140-141°C. [α]_{D} -27.4° (c = 3.28, THF).
(b) A solution of 1,3-difluorobenzene (2.3 kg, 20.0 mol) in 5 L of methylene chloride was added to a mixture of *N*-(trifluoroacetyl)-L-aspartic anhydride (4.2 kg, 20.0 mol) and aluminum chloride (7.4 kg, 55.5 mol) in 25 L of methylene chloride. The temperature of the reaction mixture was increased gradually over 1.5 hours and held at reflux for an additional 3 hours. The mixture then was cooled and 10 L of water and 20 L of 6N hydrochloric acid were added with good agitation. The methylene chloride layer was separated, washed with water and then brine, and the volatiles were removed by distilling at atmospheric pressure.
   The residue was dissolved in 40 L of toluene and 8 L of volatiles was removed by distilling the mixture *in vacuo*. The solution was heated to 50°C and 8 L of hexane was added. The mixture was cooled to 30°C and 90 L of hexane was added. The mixture then was stirred at 25°C for 3 hours giving a crystalline material. The material was isolated by filtration and the filtered residue was washed with hexane (3 x 10 L). Drying to a constant weight in a vacuum oven at room temperature under a nitrogen bleed gave (*S*)-2-[(trifluoroacetyl)amino]-4-(2,4-difluorophenyl)-4-oxobutanoic acid (5.2 kg, 16.0 mol), m.p. 82.4-84.0°C. [α]_{D} +15.2° (c = 0.956, CH₃OH).
(c) A mixture of (*S*)-2-[(trifluoroacetyl)amino]-4-(2,4-difluorophenyl)-4-oxobutanoic acid (4.8 kg, 14.7 mol) and activated carbon, Darco®, (0.4 kg) in 5 L of acetic acid was stirred at room temperature for 1 hour. The mixture was filtered on to Pearlman's catalyst (0.5 kg, 50% wet) and washed in with 15 L of glacial acetic acid. Sulfuric acid (1.2 L, 21.8 mol) in 1 L of glacial acetic acid was filtered into the mixture and washed in with 2.8 L of glacial acetic acid. The reaction vessel was vacuum/pressure purged 3 times with nitrogen and then 6 times with hydrogen to 10 psig. The mixture was stirred vigorously under hydrogen at atmospheric pressure at room temperature, for 24 hours. The reaction vessel then was purged with nitrogen and the mixture was filtered onto 4.6 kg of sodium acetate trihydrate. The filter was washed with 10 L of glacial acetic acid. The glacial acetic acid was removed by distilling the mixture in vacuo.
   The residue was partitioned between 20 L of methylene chloride and 40 L of water. The aqueous layer was extracted with 10 L of methylene chloride and the combined methylene chloride was washed with 10 L of water. The methylene chloride mixture then was dried over sodium sulfate (10 kg) and filtered. The solvent was removed *in vacuo* and the residue was dissolved in 5 L of methylene chloride. The solution was added under nitrogen to 15 L of hexane at a rate such that the temperature of the hexane mixture remained between 0 and 5°C. The mixture was allowed to stand for 1 hour giving a crystalline material. The material was isolated by filtration and the filter residue was washed with 10 L of hexane. Drying to constant weight in vacuo at 25°C with a nitrogen bleed gave (*S*)-2-[(trifluoroacetyl)amino]-4-(2,4-difluorophenyl)butanoic acid (3.3 kg, 10.4 mol), m.p. 62-83.5°C. An analytically pure sample had a melting point of 86-89°C. [α]_{D} +6.8° (c = 0.995, CH₃OH).
(d) A suspension of phosphorus pentachloride (2.2 kg, 10.6 mol) in 12 L of methylene chloride was cooled to 5°C and (*S*)-4-(2,4-difluorophenyl)-2-[(trifluoroacetyl)amino]butanoic acid (3.1 kg, 9.9 mol) in 12 L of methylene chloride was added over 20 minutes. Thin layer chromatography of a methanol-quenched aliquot confirmed that the butanoic acid had converted to the corresponding acid chloride.
   The mixture was stirred for 30 minutes and then was added to a slurry of aluminum chloride (4.3 kg) in 38.8 L of methylene chloride at a rate such that the temperature of the slurry remained between 1 and 5°C. The reaction mixture was stirred for 1 hour and then added to 28 kg of ice and 5.3 kg of concentrated hydrochloric acid. The mixture was stirred for 1 hour and the temperature allowed to rise to 20°C.
   The aqueous layer was separated and extracted with methylene chloride (2 x 15 L). The methylene chloride layer was washed once with water and combined with the methylene chloride extracts. The combined methylene chloride then was washed with water. The pH of the aqueous phase was adjusted to 6 by addition of aqueous sodium bicarbonate solution. The methylene chloride layer was washed with water and then brine. The methylene chloride was dried over sodium sulfate and filtered. The mixture was concentrated by evaporation at atmospheric pressure and the residue was dissolved in 15 L of methanol. The methanol solution was distilled to remove residual methylene chloride and then 9.9 L of water was added. The mixture was warmed to 56°C, allowed to cool to room temperature and then stirred for approximately 12 hours. A crystalline material was obtained and isolated by filtration. The filter residue was washed with 15 L of water. The isolated material was dried to constant weight *in vacuo* at room temperature with a nitrogen bleed.
   The material was dissolved in 5 L of toluene at a temperature of 90°C and combined with 10 L of heptane at a temperature of 80°C. The temperature of the mixture gradually was decreased over 1.5 hours. The mixture then was stirred at 5°C for approximately 12 hours giving a crystalline material. The material was isolated by filtration and the filter residue was washed with 15 L of heptane. Drying to constant weight *in vacuo* at room temperature with a nitrogen bleed gave (*S*)-5,7-difluoro-2-[(trifluoroacetyl)amino]-3,4-dihydro-(2*H*)-naphthalen-1-one (2.0 kg, 6.8 mol), m.p. 142.4-144.6°C, [α]_{D} =59.4° (c = 0.994, CH₃OH).
(e) A reaction vessel containing a mixture of (*S*)-5,7-difluoro-2-[(trifluoroacetyl)amino]-3,4-dihydro-(2*H*)-naphthalen-1-one (1.1 kg, 3.8 mol) and Pearlman's catalyst (0.55 kg, 50% wet) in 11 L of TFA was vacuum/pressure purged 8 times with nitrogen and then 8 times with hydrogen to 11 psig. The mixture was stirred vigorously under hydrogen (125 psig) at room temperature for 24 hours. Thin layer chromatography confirmed that the naphthalen-1-one had converted to (*S*)-1-hydroxy-5,7-difluoro-2-[(trifluoroacetyl)amino]-3,4-dihydro-(2*H*)-naphthalene.
(f) Sulfuric acid (1.1 L, 19.4 mol) in 1 L of TFA then was added and the mixture stirred under hydrogen (125 psig) at room temperature for an additional 24 hours. The reaction vessel then was purged with nitrogen and the mixture was filtered over Celite and washed through with 11 L of TFA. The filtrate was combined with 2.8 kg sodium acetate trihydrate and 80 L of water. The mixture was cooled to 10°C giving a crystalline material. The material was isolated by filtration and the filter residue was washed with 10 L of ice water. Drying gave (*S*)-5,7-difluoro-2-[(trifluoroacetyl)amino]-1,2,3,4-tetrahydronaphthalene (0.8 kg, 2.9 mol), m.p. 159.9-160.9°C. [α]_{D} -56.0° (c= 1.01, CH₃OH).
(g) Lithium hydroxide monohydrate (7.8 g, 0.2 mol) was added to a solution of (*S*)-5,7-difluoro-2-[(trifluoroacetyl)amino]-1,2,3,4-tetrahydronaphthalene (20.8 g, 74.5 mmol) in 187 mL of methanol and 21 mL of water. The mixture was stirred at reflux for 30 minutes and diluted with 200 mL of methanol. The diluted mixture then was combined with 60 mL of water, 24.8 mL of concentrated hydrochloric acid and 4.2 g of activated carbon, Darco®. The mixture was stirred for 30 minutes and then filtered through Celite. The filtrate was distilled until the head temperature reached 75°C. The remaining mixture was allowed to cool and let stand for approximately 60 hours. The mixture then was cooled in an ice bath giving a crystalline material. The material was isolated by filtration and the filter residue was washed with water. Drying to constant weight in vacua at room temperature under a nitrogen stream gave (*S*)-5,7-difluoro-1,2,3,4-tetrahydronaphthalen-2-ylamine hydrochloride (14.8 g, 67.6 mol), m.p. > 280°C. [α]_{D} -66.2° (c = 0.162, CH₃OH).

### Example 5

### (R)-5,7-Difluoro-1,2,3,4-tetrahydronaphthalen-2-yl methanesulfonate

The following is the preparation of a compound of Formula 5 in which t is 2, R¹ is fluoro at the 5- and 7-position and L is mesyloxy.

A mixture of (*R*)-5,7-difluoro-1,2,3,4-tetrahydro-2-hydroxynaphthalene (59.0 g, 0.32 mol), prepared as in Example 3, and triethylamine (13.8 M, 74.2 mL, 0.53 mol) in 1.78 L of diethyl ether was cooled using a methanol/ice bath. Methanesulfonyl chloride (12.9 M, 37.2 mL, 0.48 mol) was added under argon over 5-10 minutes and the mixture was stirred at room temperature for 18 hours. The mixture was partitioned between water and ether and the ether layer was separated and the aqueous layer was extracted with ether. The combined ether layers were washed once with each of 1N hydrochloric acid, saturated sodium bicarbonate solution and brine and then dried over magnesium sulfate. Evaporation gave crude (*R*)-5,7-difluoro-1,2,3,4-tetrahydro-naphthalen-2-yl methanesulfonate as an off-white solid (87.12 g), m.p. 79-80°C. [α]_{D}²⁵ = + 16.77° (c = 2.0, CHCl₃).

Proceeding as in Example 5 but substituting different starting materials for (*R*)-5,7-difluoro-1,2,3,4-tetrahydro-2-hydroxynaphthalene, the following compounds of Formula 5 were made:
substituting 1,2,3,4-tetrahydro-2-hydroxynaphthalene gave 1,2,3,4-tetrahydro-naphthalen-2-yl methanesulfonate;
substituting (*R*)-1,2,3,4-tetrahydro-2-hydroxynaphthalene gave (*R*)-1,2,3,4-tetrahydronaphthalen-2-yl methanesulfonate;
substituting (*R*)-5-fluoro-1,2,3,4-tetrahydro-2-hydroxynaphthalene gave (*S*)-5-fluoro-1,2,3,4-tetrahydronaphthalen-2-yl methanesulfonate;
substituting (*R*)-6-fluoro-1,2,3,4-tetrahydro-2-hydroxynaphthalene gave (*S*)-6-fluoro-1,2,3,4-tetrahydronaphthalen-2-yl methanesulfonate;
substituting (*R*)-7-fluoro-1,2,3,4-tetrahydro-2-hydroxynaphthalene gave (*S*)-7-fluoro-1,2,3,4-tetrahydronaphthalen-2-yl methanesulfonate;
substituting 6,7-dichloro-1,2,3,4-tetrahydro-2-hydroxynaphthalene gave 2-6,7-dichloro-1,2,3,4-tetrahydronaphthalen-2-yl methanesulfonate;
substituting (-)-6,7-difluoro-1,2,3,4-tetrahydro-2-hydroxynaphthalene gave (-)-6,7-difluoro-1,2,3,4-tetrahydronaphthalen-2-yl methanesulfonate;
substituting 6,7-difluoro-1,2,3,4-tetrahydro-2-hydroxynaphthalene gave 6,7-difluoro-1,2,3,4-tetrahydronaphthalen-2-yl methanesulfonate;
substituting (*S*)-5,7-difluoro-1,2,3,4-tetrahydro-2-hydroxynaphthalene gave (*R*)-5,7-difluoro-1,2,3,4-tetrahydronaphthalen-2-yl methanesulfonate;
substituting 5,7-difluoro-1,2,3,4-tetrahydro-2-hydroxynaphthalene gave 5,7-difluoro-1,2,3,4-tetrahydronaphthalen-2-yl methanesulfonate;
substituting 6,8-difluoro-1,2,3,4-tetrahydro-2-hydroxynaphthalene gave 6,8-difluoro-1,2,3,4-tetrahydronaphthalen-2-yl methanesulfonate;
substituting (*R*)-6,8-difluoro-1,2,3,4-tetrahydro-2-hydroxynaphthalene gave (*S*)-6,8-difluoro-1,2,3,4-tetrahydronaphthalen-2-yl methanesulfonate;
substituting 6,8-difluoro-1,2,3,4-tetrahydro-2-hydroxy-7-methoxynaphthalene gave 6,8-difluoro-1,2,3,4-tetrahydro-7-methoxynaphthalen-2-yl methane sulfonate;

### Example 6

### (S)-5,7-Difluoro-1,2,3,4-tetrahydronaphthalen-2-ylamine hydrochloride

The following is the preparation of a compound of Formula 3 in which t is 2 and R¹ is fluoro at the 5- and 7-position.

A mixture of (*R*)-5,7-difluoro-1,2,3,4-tetrahydronaphthalen-2-yl methanesulfonate (54.0 g), prepared as in Example 5, and lithium azide (15.8 g, 0.322 mol) in 400 mL of DMF was stirred under argon at 50°C for 16 hours. The reaction was quenched with 200 mL of water and the mixture extracted with 1 L of pentane. The extract was washed with 50 mL of water and dried over magnesium sulfate. Evaporation under reduced pressure at 35°C gave crude (*S*)-2-azido-5,7-difluoro-1,2,3,4-tetrahydronaphthalene as a yellow oil residue (59.8 g).

The azide residue was dissolved in 1.2 L of ethyl acetate and hydrogenated over 10% palladium on carbon (6 g) for 6 hours, recharging with hydrogen every hour to remove evolved nitrogen gas. The mixture waa then filtered through Celite and stirred with ethereal hydrogen chloride (1N, 250 mL) giving a crystalline material. The material was isolated by filtering over 4 hours and the filter residue was washed with ethyl acetate. Removing the remaining solvents *in vacuo* gave (*S*)-5,7-difluoro-1,2,3,4-tetrahydronaphthalen-2-ylamine hydrochloride (48.2 g, 0.22 mol) as a white solid, m.p. > 280°C. [α]_{D}²⁵ = -60.15° (c = 2.7, CH₃OH).

Proceeding as in Example 6 but substituting a different starting material for (*R*)-5,7-difluoro-1,2,3,4-tetrahydronaphthalen-2-yl methanesulfonate, the following compounds of Formula 3 were made:
substituting 1,2,3,4-tetrahydronaphthalen-2-yl methanesulfonate gave 1,2,3,4-tetrahydronaphthalen-2-ylamine hydrochloride, m.p. 239-242°C;
substituting (*R*)-1,2,3,4-tetrahydronaphthalen-2-yl methanesulfonate gave (*S*)-1,2,3,4-tetrahydronaphthalen-2-ylamine hydrochloride, m.p. 241-244°C;
substituting (*R*)-5-fluoro-1,2,3,4-tetrahydronaphthalen-2-yl methanesulfonate gave (*S*)-5-fluoro-1,2,3,4-tetrahydronaphthalen-2-ylamine hydrochloride, m.p. > 280°C;
substituting (*R*)-6-fluoro-1,2,3,4-tetrahydronaphthalen-2-yl methanesulfonate gave (*S*)-6-fluoro-1,2,3,4-tetrahydronaphthalen-2-ylamine hydrochloride, m.p. 264-265°C;
substituting (*R*)-7-fluoro-1,2,3,4-tetrahydronaphthalen-2-yl methanesulfonate gave (*S*)-7-fluoro-1,2,3,4-tetrahydronaphthalen-2-ylamine hydrochloride, m.p. > 280°C;
substituting 6,7-dichloro-1,2,3,4-tetrahydronaphthalen-2-yl methanesulfonate gave 6,7-difluoro-1,2,3,4-tetrahydronaphthalen-2-ylamine hydrochloride, m.p. > 280°C;
substituting (-)-6,7-difluoro-1,2,3,4-tetrahydronaphthalen-2-yl methanesulfonate gave (-)-6,7-difluoro-1,2,3,4-tetrahydronaphthalen-2-ylamine hydrochloride;
substituting 6,7-difluoro-1,2,3,4-tetrahydronaphthalen-2-yl methanesulfonate gave 6,7-difluoro-1,2,3,4-tetrahydronaphthalen-2-ylamine hydrochloride;
substituting (*S*)-5,7-difluoro-1,2,3,4-tetrahydronaphthalen-2-yl methanesulfonate gave (*R*)-5,7-difluoro-1,2,3,4-tetrahydronaphthalen-2-ylamine hydrochloride, m.p. > 280°C;
substituting 5,7-difluoro-1,2,3,4-tetrahydronaphthalen-2 -yl methanesulfonate gave 5,7-difluoro-1,2,3,4-tetrahydronaphthalen-2-ylamine hydrochloride;
substituting 6,8-difluoro-1,2,3,4-tetrahydronaphthalen-2-yl methanesulfonate gave 6,8-difluoro-1,2,3,4-tetrahydronaphthalen-2-ylamine hydrochloride;
substituting (*R*)-6,8-difluoro-1,2,3,4-tetrahydronaphthalen-2-yl methanesulfonate gave (*S*)-6,8-difluoro-1,2,3,4-tetrahydronaphthalen-2-ylamine hydrochloride;
substituting 6,8-difluoro-1,2,3,4-tetrahydro-7-methoxynaphthalen-2-yl methanesulfonate gave 6,8-difluoro-7-methoxy-1,2,3,4-tetrahydronaphthalen-2-ylamine hydrochloride;

### Reference Example 3

### 5,7-difluoro-1-(1,2,3,4-tetrahydronaphthalen-2-yl)-1,3-dihydroimidazole-2-thione

The following is the preparation of a compound of Formula I(a) in which t is 2 and R³, R⁴ and R⁵ are each hydro and R¹ flucro at the 5-and 7-position.

A mixture of (*S*)-5,7-difluoro-1,2,3,4-tetrahydronaphthalen-2-ylamine (2.05 g, 11.2 mmol), prepared as in Example 8, and dimethoxyacetaldehyde (1.73 g, 13.1 mmol) in 50 mL of ethanol was hydrogenated over 10% palladium on carbon (500 mg) for 18 hours. The mixture was filtered and concentrated by evaporation. The residue was combined with potassium thiocyanate (1.57 g, 16.2 mmol) in 30 mL of 1 N hydrochloric acid and 20 mL of ethanol and the mixture was heated at 70-80°C for 18 hours. The mixture was cooled in an ice bath giving a crystalline material which was isolated by filtration. Recrystallization from ethyl acetate/hexane gave 1-(5,7-difluoro-1,2,3,4-tetrahydronaphthalen-2-yl)-1,3-dihydroimidazole-2-thione (1.27 g, 4.76 mmol), m.p. 250-251°C (Reference).

Proceeding as in Reference Example 3 but substituting a different starting material for (*S*)-5,7-difluoro-1,2,3,4-tetrahydronaphthalen-2-ylamine, the following compounds of Formula I were made:
substituting 7-fluoro-1,2,3,4-tetrahydronaphthalen-2-ylamine gave 1-(7-fluoro-1,2,3,4-tetrahydronaphthalen-2-yl)-1,3-dihydroimidazole-2-thione, m.p. 215-217°C (Reference).
substituting 6,7-difluoro-1,2,3,4-tetrahydronaphthalen-2-ylamine gave 1-(6,7-difluoro-1,2,3,4-tetrahydronaphthalen-2-yl)-1,3-dihydroimidazole-2-thione, m.p. 242-243°C (Reference);
substituting 6,8-difluoro-1,2,3,4-tetrahydronaphthalen-2-ylamine gave 1-(6,8-difluoro-1,2,3,4-tetrahydronaphthalen-2-yl)-1,3-dihydroimidazole-2-thione, m.p. 260-261°C (Reference);
substituting 5-methoxy-1,2,3,4-tetrahydronaphthalen-2-ylamine gave 1-(1,2,3,4-tetrahydro-5-methoxynaphthalen-2-yl)-1,3-dihydroimidazole-2-thione, m.p. 233-235°C (Reference);
substituting 6-methoxy-1,2,3,4-tetrahydronaphthalen-2-ylamine gave 1-(1,2,3,4-tetrahydro-6-methoxynaphthalen-2-yl)-1,3-dihydroimidazole-2-thione, m.p. 226-227°C (Reference);
substituting 7-methoxy-1,2,3,4-tetrahydronaphthalen-2-ylamine gave 1-(1,2,3,4-tetrahydro-7-methoxynaphthalen-2-yl)-1,3-dihydroimidazole-2-thione, m.p. 271-273°C (Reference);
substituting 8-methoxy-1,2,3,4-tetrahydronaphthalen-2-ylamine gave 1-(1,2,3,4-tetrahydro-8-methoxynaphthalen-2-yl)-1,3-dihydroimidazole-2-thione, m.p. 249-251°C (Reference);
substituting 6, 8-difluoro-7-methoxy-1,2,3,4-tetrahydronaphthalen-2-ylamine gave 1-(6,8-difluoro-1,2,3,4-tetrahydro-7-methoxynaphthalen-2-yl)-1,3-dihydroimidazole-2-thione, m.p. 228-230°C (Reference);
substituting 5-methoxy-1,2,3,4-tetrahydronaphthalen-1-ylamine gave 1-(1,2,3,4-tetrahydro-5-methoxynaphthalen-1-yl)-1,3-dihydroimidazole-2-thione, m.p. 176-177°C (Reference);
substituting 6-methoxy-1,2,3,4-tetrahydronaphthalen-1-ylamine gave 1-(1,2,3,4-tetrahydro-6-methoxynaphthalen-1-yl)-1,3-dihydroimidazole-2-thione, m.p. 190-192°C (Reference);
substituting 7-methoxy-1,2,3,4-tetrahydronaphthalen-1-ylamine gave 1-(1,2,3,4-tetrahydro-7-methoxynaphthalen-1-yl)-1,3-dihydroimidazole-2-thione, m.p. 142-143°C (Reference);

### Example 7

### (-)-6,7-Difluoro-2-isothiocyano-1,2,3,4-tetrahydronaphthalene

The following is the preparation of a compound of Formula 9 in which t is 2 and R¹ is fluoro at the 6- and 7-position.

A mixture of (-)-6,7-difluoro-1,2,3,4-tetrahydronaphthalen-2-ylamine (0.56 g, 3.06 mmol), prepared as in Example 6, and 1,1'-thiocarboxyldiimidazole (0.82 g, 4.59 mmol) in 15 mL of ethyl acetate was stirred until the reaction was complete. The solvent was removed by evaporation. Purification of the residue by column chromatography on silica gel (elution: 5% acetone/methylene chloride) gave (-)-6,7-difluoro-2-isothiocyano-1,2,3,4-tetrahydronaphthalene (0.55 g, 2.42 mmol). [α]_{D}²⁵ - 15.5°, (c = 1.0, CH₃OH).

Proceeding as in Example 7 but substituting a different starting material for (-)-6,7-difluoro-1,2,3,4-tetrahydronaphthalen-2-ylamine, the following compounds of Formula 9 were made:
substituting 1,2,3,4-tetrahydronaphthalen-2-ylamine gave 2-isothiocyano-1,2,3,4-tetrahydronaphthalene as an oil; and
substituting (+)-6,7-difluoro-1,2,3,4-tetrahydronaphthalen-2-ylamine gave (+)-6,7-difluoro-2-isothiocyano-1,2,3,4-tetrahydronaphthalene, m.p. 206-207°C.

### Example 8

### 1-(1,2,3,4-Tetrahydronaphthalen-2-yl)imidazole

The following is the preparation of a compound of Formula 11 in which t is 0.

A mixture of 1,2,3,4-tetrahydronaphthalen-2-yl methanesulfonate (15.74 g, 69.6 mmol), prepared as in Example 5, and imidazole (23.68 g, 349 mmol) in 100 mL of DMF was heated at 80 to 90°C under argon for 24 hours. The solvent was removed under vacuum by rotary evaporation. The residue was dissolved in 500 mL of ethyl acetate and the solution was washed with water (5 x 250 mL). The combined aqueous layer was extracted with 500 mL of ethyl acetate and the extract was then washed with water (5 x 250 mL). The combined ethyl acetate extract was washed with saturated sodium chloride solution, dried over magnesium sulfate and concentrated by rotary evaporation. Purification of the residue by column chromatography on silica gel (elution: 5% methanol/methylene chloride) gave 1-(1,2,3,4-tetrahydronaphthalen-2-yl)imidazole (5.18 g, 26.1 mmol), m.p. 93-95°C.

Proceeding as in Example 8, but substituting 4-methylimidazole for imidazole and 2-bromo-1,2,3,4-tetranaphthalen-1-one for 1,2,3,4-tetrahydronaphthalen-2-yl methanesulfonate and then reducing gave 1-(1,2,3,4-tetrahydro-naphthalen-2-yl)-4-methylimidazole.

### Example 9

### (S)-N-[3-(5,7-Difluoro-1,2,3,4-tetrahydronaphthalen-2-yl)-2,3-dihydro-1H-imidazol-4-ylmethyl]formamide

The following is the preparation of a compound of Formula 11 in which t is 2, R¹ is fluoro at the 5- and 7-positions and R⁴ is hydro.

A mixture of formamide, prepared as in Example 20, (*S*)-*N*-[3-(5,7-difluoro-1,2,3,4-tetrahydronaphthalen-2-yl)-2-thioxo-2,3-dihydro-1*H*-imidazol-4-ylmethyl]-formamide (1.38 g, 4.27 mmol) and activated Raney® nickel (11 g) in 50 mL of ethanol was stirred rapidly at 80°C for 1 hour. The mixture was filtered through Celite and the filtrate evaporated to give a white solid (0.98 g). The solid was recrystallized from ethyl acetate/methanol to give (*S*)-*N*-[3-(5,7-difluoro-1,2,3,4-tetrahydronaphthalen-2-yl)-2,3-dihydro-1*H*-imidazol-4-ylmethyl]formamide, m.p. 194-195°C.

Proceeding as in Example 9, but substituting a different starting material for (*S*)-*N*-[3-(5,7-difluoro-1,2,3,4-tetrahydronaphthalen-2-yl)-2-thioxo-2,3-dihydro-1*H*-imidazol-4-ylmethyl]formamide gave the following compounds of Formula 11:
substituting (*S*)-5-aminomethyl-1-(5,7-difluoro-1,2,3,4-tetrahydronaphthalen-2-yl)-1,3-dihydroimidazole-2-thione hydrochloride gave (*S*)-[3-(5,7-difluoro-1,2,3,4-tetrahydronaphthalen-2-yl)-2,3-dihydro-1*H*-imidazol-4-ylmethyl]amine, m.p. 273-274°C; and
substituting 1-(5,7-difluoro-1,2,3,4-tetrahydronaphthalen-2-yl)-1,3-dihydro-imidazole-2-thione gave 1-(5,7-difluoro-1,2,3,4-tetrahydronaphthalen-2-yl)-1,3-dihydroimidazole.

### Example 10

### 3-Amino-1-(1,2,3,4-tetrahydronaphthalen-2-yl)-1,3-dihydroimidazole-2-thione

The following is the preparation of a compound of Formula I (a) in which t is 0, R³ is amino and R⁴ and R⁵ are each hydro.

A mixture of 1-(1,2,3,4-tetrahydronaphthalen-2-yl)imidazole (198 mg, 1 mmol), prepared as in Example 8, and *O*-mesitylenesulfonylhydroxylamine (215 mg, 1 mmol) in acetonitrile was stirred under argon for 18 hours. The reaction mixture was diluted with diethyl other giving a precipitate as an oil. The solvents were decanted away and the residue was washed with diethyl ether (2 x 10 mL). Residual solvent was evaporated under vacuum. A mixture of the residue and lac sulfur (32 mg, 1.0 mmol) in 1 mL of pyridine and 0.5 mL of triethylamine were heated at approximately 90°C under argon for 4 hours. The solvent was removed by evaporation and the residue was co-evaporated with toluene. The residue was purified by column chromatography on silica gel eluting with methylene chloride to give 3-amino-1-(1,2,3,4-tetrahydro-naphthalen-2-yl)-1,3-dihydroimidazole-2-thione (5.18 g, 26.1 mmol), m.p. 187-189°C.

Proceeding as in Example 10, but substituting different starting materials for *O*-mesitylenesulfonatehydroxylamine and/or 1-(1,2,3,4-tetrahydro-naphthalen-2-yl)imidazole gave the following compounds of Formula I:
substituting *tert*-butyl bromoacetate gave *tert*-butyl 3-(1,2,3,4-tetrahydronaphthalan-3-yl)-2-thioxo-2,3-dihydro-1*H*-imidazol-1-ylacetate, m.p. 167-169°C;
substituting methyl 4-bromobutyrate and 1-(1,2,3,4-tetrahydronaphthalen-2-yl)imidazole gave methyl 4-[3-(1,2,3,4-tetrahydronaphthalen-2-yl)-2-thioxo-2,3-dihydro-1*H*-imidazol-1-yl]butyrate;
substituting methyl 5-bromomethylpicolinate and 1-(1,2,3,4-tetrahydronaphthalen-2-yl)imidazole gave methyl 5-[3-(1,2,3,4-tetrahydronaphthalen-2-yl)-2-thioxo-2,3-dihydro-1*H*-imidazol-1-ylmethyl]picolinate as an oil;
substituting methyl 4-bromomethylbenzoate and 1-(1,2,3,4-tetrahydronaphthalen-2-yl)imidazole gave methyl 4-[3-(1,2,3,4-tatrahydronaphthalen-2-yl)-2-thioxo-2,3-dihydro-1*H*-imidazol-1-ylmethyl]benzoate, m.p. 133-135°C;
substituting methyl 3-bromomethylbenzoate and 1-(1,2,3,4-tetrahydronaphthalen-2-yl)imidazole gave methyl 3-[3-(1,2,3,4-tetrahydronaphthalen-2-yl)-2-thioxo-2,3-dihydro-1*H*-imidazol-1-ylmethyl]benzoate, m.p. 130-132°C;
substituting 3,4-dimethoxybenzyl chloride and 1-(1,2,3,4-tetrahydronaphthalen-2-yl)imidazole gave 3-(3,4-dimethoxybenzyl)-1-(1,2,3,4-tetrahydronaphthalen-2-yl)-1,3-dihydroimidazole-2-thione, m.p. 129-131°C;
substituting methyl 4-bromomethylbenzoate and 1-(1,2,3,4-tetrahydronaphthalen-2-yl)-4-methylimidazole gave methyl 4-[3-(1,2,3,4-tetrahydronaphthalen-2-yl)-5-methyl-2-thioxo-2,3-dihydro-1*H*-imidazol-1-ylmethyl]benzoate, m.p. 140-141°C;
substituting 6-dimethylamino-3-bromopyridazine and 1-(1,2,3,4-tetrahydronaphthalen-2-yl) imidazole gave 3-(6-dimethylaminopyridazin-3-yl)-1-(1,2,3,4-tetrahydronaphthalen-2-yl)-1,3-dihydroimidazole-2-thione, m.p. 198-199°C;
substituting 2-bromoethylbenzene and 1-(1,2,3,4-tetrahydronaphthalen-2-yl)-imidazole gave 3-(2-phenylethyl)-1-(1,2,3,4-tetrahydronaphthalen-2-yl)-1,3-dihydroimidazole-2-thione, m.p. 140-142°C;
substituting methyl 4-(2-bromoethyl)benzoate and 1-(1,2,3,4-tetrahydronaphthalen-2-yl)imidazole gave methyl 4-{2-[3-(1,2,3,4-tetrahydronaphthalen-2-yl)-2-thioxo-2,3-dihydro-1*H*-imidazol-1-yl]ethyl}benzoate, m.p. 159-161°C;
substituting 4-(2-bromoethyl)benzoic acid and 1-(1,2,3,4-tetrahydronaphthalen-2-yl)imidazole gave 4-[3-(1,2,3,4-tetrahydronaphthalen-2-yl)-2-thioxo-2,3-dihydro-1*H*-imidazol-1-ylethyl]benzoic acid, m.p. 227-230;
substituting 3,4-dimethoxy-1-(2-bromoethyl)benzene and 1-(1,2,3,4-tetrahydronaphthalen-2-yl)imidazole gave 3-[2-(3,4-dimethoxyphenyl)ethyl]-1-(1,2,3,4-tetrahydronaphthalen-2-yl)-1,3-dihydroimidazole-2-thione, m.p. 97-101°C;
substituting 4-(2-bromoethyl)benzoic acid and (*S*)-1-(5,7-difluoro-1,2,3,4-tetrahydronaphthalen-2-yl)imidazole gave (*S*)-4-{2-[3-(5,7-difluoro-1,2,3,4-tetrahydronaphthalen-2-yl)-2-thioxo-2,3-dihydro-1*H*-imidazol-1-yl]ethyl}-benzoic acid, m.p. 222-224°C, and treating with potassium hydroxide in methanol evaporating to dryness and recrystallizing from methanol/isopropanol gave potassium (*S*)-4-{2-[3-(5,7-difluoro-1,2,3,4-tetrahydronaphthalen-2-yl)-2-thioxo-2,3-dihydro-1*H*-imidazol-1-yl]ethyl}benzoate, m.p. >280°C;
substituting 4-(2-bromoethyl)-1-cyanobenzene and (*S*)-1-(5,7-difluoro-1,2,3,4-tetrahydronaphthalen-2-yl)imidazole gave (*S*)-3-[2-(4-cyanophenyl)ethyl]-1-(5,7-difluoro-1,2,3,4-tetrahydronaphthalen-2-yl)-1,3-dihydro-imidazole-2-thione, m.p. 130-133°C;
substituting 4-(2-bromoethyl)benzene and (*S*)-1-(5,7-difluoro-1,2,3,4-tetrahydronaphthalen-2-yl)imidazole gave (*S*)-3-(2-phenylethyl)-1-(5,7-difluoro-1,2,3,4-tetrahydronaphthalen-2-yl)-1,3-dihydroimidazole-2-thione, m.p. 131-133°C;
substituting 4-(2-bromoethyl)-1-cyanobenzene and 1-(1,2,3,4-tetrahydronaphthalen-2-yl)imidazole gave 3-[2-(4-cyanophenyl)ethyl]-1-(1,2,3,4-tetrahydronaphthalen-2-yl)-1,3-dihydroimidazole-2-thione, m.p. 169-170°C;
substituting 4-(2-bromoethyl)benzoic acid and (*S*)-*N*-[3-(5,7-difluoro-1,2,3,4-tetrahydronaphthalen-2-yl)-2,3-dihydro-1*H*-imidazol-4-ylmethyl]formamide gave (*S*)-4-{2-[4-formylaminomethyl-3-(5,7-difluoro-1,2,3,4-tetrahydro-naphthalen-2-yl)-2-thioxo-2,3-dihydro-1*H*-imidazol-1-yl]ethyl}benzoic acid; and
substituting ethyl 3-bromopropionate and (*S*)-*N*-[3-(5,7*-*difluoro-1,2,3,4-tetrahydronaphthalen-2-yl)-2,3-dihydro-1*H*-imidazol-4-ylmethyl]formamide gave ethyl (*S*)-3-[4-formylaminomethyl-3-(5,7-difluoro-1,2,3,4-tetrahydro-naphthalen-2-yl)-2-thioxo-2,3-dihydro1*H*-imidazol-1-yl]propionate.

### Reference Example 3a

### 1-(1,2,3,4-tetrahydronaphthalen-2-yl)-1,3-dihydroimidazole-2-thione

The following is the preparation of a compound of Formula I (a) in which t is 0 and R³, R⁴ and R⁵ are each hydro.

A solution of 1-(1,2,3,4-tetrahydronaphthalen-2-yl)imidazole (1.6 g, 8.1 mmol), prepared as in Example 13, in 30 mL of THF was cooled to -78°C and n-butyllithium (6 mL, 9.7 mmol) was added over 15 minutes. The mixture was stirred at -78°C for 1 hour and lac sulfur (0.34 g, 10.5 mmol) was added. The mixture was stirred at -78°C for an additional 2 hours and then allowed to warm to room temperature. The mixture was poured into 100 mL of water giving a crystalline material. The material was isolated by filtration, washed with ethyl ether and dried. The filtrate was extracted with methylene chloride (2 x 100 mL) and the extracts were washed with brine, dried over sodium sulfate and concentrated by evaporation. The residue was purified by column chromatography on silica gel [elution: 1.5% methanol (containing 2% concentrated ammonium hydroxide)]. Combining the crystalline material gave 1-(1,2,3,4-tetrahydronaphthalen-2-yl)-1,3-dihydroimidazole-2-thione (0.81 g, 3.5 mmol), m.p. 233-234°C.

### Example 11

### 5-Amino-1-(1,2,3,4-tetrahydronaphthalen-2-yl)-1,3-dihydroimidazole-2-thione

The following is the preparation of a compound of Formula I (a) in which t is 0, R³ and R⁴ are hydro and R⁵ is amino.

A mixture of 2-isothiocyano-1,2,3,4-tetrahydronaphthalene (1.92 g, 10.1 mmol), prepared as in Example 7, and aminoacetonitrile hydrochloride (0.94 g, 10.1 mmol) in 1.41 mL of triethylamine was heated at 60°C for 1 hour. The solvent was evaporated and the residue was purified by flash chromatography (elution: methylene chloride followed by 3% methanol in methyLene chloride). The purified residue was recrystallized from ethyl acetate/hexane. The residue (1.06 g) and 44 mL of 0.1N potassium hydroxide was stirred under nitrogen for 15 minutes. The residue was isolated by filtration, washed with water, air dried and then stirred with methylene chloride. Filtration and drying gave 5-amino-1-(1,2,3,4-tetrahydronaphthalen-2-yl)-1,3-dihydroimidazole-2-thione, m.p. 169-171°C.

### Example 12

### (S)-5-Hydroxymethyl-1-(5,7-difluoro-1,2,3,4-tetrahydronaphthalen-2-yl)-1,3-dihydroimidazole-2-thione

The following is the preparation of a compound of Formula I(a) in which t is 2, R¹ is fluoro at the 5- and 7-position and R⁵ is hydroxymethyl.

Potassium thiocyanate (15.9 g, 162.6 mmol) was dried by heating to 175°C under nitrogen and then cooled to 35°C under vacuum with several nitrogen purges. A mixture of dihydroxyacetone (15.9 g, 176.7 mmol) and (*S*)-5,7-difluoro-1,2,3,4-tetrahydronaphthalen-2-ylamine hydrochloride (30.0 g, 137.0 mmol), prepared as in Example 6, in 540 mL of ethyl acetate was added to the dry potassium thiocyanate. The reaction vessel was purged with nitrogen and 40.83 g of glacial acetic acid was added. The reaction mixture was stirred at 35°C for 2 hours and 100 mL of 1.0 M sulfuric acid was added. The mixture was stirred for 15 minutes, then cooled in an ice bath and 2.5 M sodium hydroxide was added until the mixture was pH 7. The organic layer was washed with 50 mL of saturated aqueous sodium bicarbonate and then 50 mL of brine. The organic layer was concentrated to 480 mL by distillation and the mixture was cooled to 6°C and allowed to stand for 12 hours giving a crystalline material. The material was isolated by filtration, the filter residue was washed with cold ethyl acetate and the isolated material dried.

The material was dissolved in 650 mL of ethyl acetate and 25 mL of water. The mixture was distilled until 500 mL of volatiles were removed. The mixture was cooled to room temperature and stirred for 45 minutes giving a crystalline material. The material was isolated by filtration and the filter residue was washed with cold ethyl acetate. Drying *in vacuo* with a nitrogen bleed gave (*S*)-5-hydroxymethyl-1-(5,7-difluoro-1,2,3,4-tetrahydronaphthalen-2-yl)-1,3-dihydroimidazole-2-thione (30.4 g, 107.4 mol), m.p. 206-207°C. [α]_{D} -40° (c = 0.682, CH₃OH).

Proceeding similarly as in Example 18, but substituting (*S*)-6,7-dichloro-1,2,3,4-tetrahydronaphthalen-2-ylamine hydrochloride for (*S*)-5,7-difluoro-1,2,3,4-tetrahydronaphthalen-2-ylamine hydrochloride gave (*S*)-5-hydroxymethyl-1-(6,7-dichloro-1,2,3,4-tetrahydronaphthalen-2-yl)-1,3-dihydro-imidazole-2-thione, m.p. 247-248°C.

### Example 13

### (S)-5-Cyano-1-(5,7-difluoro-1,2,3,4-tetrahydronaphthalen-2-yl)-1,3-dihydroimidazole-2-thione

The following is the preparation of a compound of Formula I(a) in which t is 2, R¹ is fluoro at the 5- and 7-position, R³ and R⁴ are each hydro and R⁵ is cyano.

A solution of (*S*)-5,7-difluoro-1,2,3,4-tetrahydronaphthalen-2-ylamine hydrochloride (50.3 g, 0.23 mol), prepared as in Example 8, and sodium hydroxide (10.0 g, 0.25 mol) in 450 mL of water was heated to 50°C and then formaldehyde sodium bisulfite complex (30.8 g, 0.23 mol) was added. The mixture was stirred for 30 minutes and potassium cyanide (15.0 g, 0.23 mol) was added. The mixture was heated to 80°C, stirred for 1 hour, cooled to room temperature, and then extracted with ethyl acetate. Evaporation gave an oily residue (51.3 g). Purification by column chromatography on silica gel (elution: 5% methanol/ methylene chloride) gave (*S*)-(5,7-difluoro-1,2,3,4-tetrahydronaphthalen-2-yl-amino)acetonitrile (39.4 g) and (*S*)-5,7-difluoro-1,2,3,4-tetrahydro-naphthalen-2-ylamine (7.12 g). Proceeding similarly as above, recycling the recovered starting material and combining yields gave (*S*)-(5,7-difluoro-1,2,3,4-tetrahydronaphthalen-2-yl-amino)acetonitrile (44.8 g, 0.20 mol), m.p. 73-76°C. [α]_{D}²⁵ = -58.04° (c = 1.6, CHCl₃).

A solution of the (*S*)-(5,7-difluoro-1,2,3,4-tetrahydronaphthalen-2-yl-amino)acetonitrile in butyl formate (8.7 M, 240 mL, 2.10 mol) was heated to reflux and stirred under nitrogen for 19 hours. The solvents were removed under reduced pressure, toluene was added and then evaporated. Drying gave (*S*)-*N*-(cyanomethyl)-*N*-(5,7-difluoro-1,2,3,4-tetrahydronaphthalen-2-yl)formamide as an oily residue (53.2 g). A stirring mixture of the formamide (53.2 g) and ethyl formate (12.4 M, 48.7 mL, .604 mol) in 0.925 L of anhydrous THE was cooled to -15°C. A solution of potassium *tert*-butoxide in THF (1.0 M, 302 mL, 0.302 mol) was added over 20 minutes and the mixture was stirred for 18 hours. Evaporation of the solvent gave potassium (*S*)-*N*-(1-cyano-2-oxyvinyl)-*N*-(5,7-difluoro-1,2,3,4-tetrahydronaphthalen-2-yl)formamide as a residue.

A mixture of the potassium (*S*)-*N*-(1-cyano-2-oxyvinyl)-*N*-(5,7-difluoro-1,2,3,4-tetrahydronaphthalen-2-yl)formamide and potassium thiocyanate (78.1 g, 0.80 mol) in 0.99 L of 1N hydrochloric acid and 0.497 L of ethanol was heated to 85°C and stirred for 135 minutes. The mixture was then cooled in an ice bath to form a precipitate which was collected as a slurry. Purification by column chromatography on silica gel packed in hexane (elution: 10% acetone/methylene chloride) gave (*S*)-5-cyano-1-(5,7-difluoro-1,2,3,4-tetrahydronaphthalen-2-yl)-1,3-dihydroimidazole-2-thione (18.1 g, 0.06 mol), m.p. 241-249°C. [α]_{D}²⁵ = -69.1° (c = 1.20, DMSO).

Proceeding as in Example 13, but substituting a different starting material for (*S*) -5,7-difluoro-1,2,3,4-tetrahydronaphthalen-2-ylamine hydrochloride gave the following compounds of Formula I:
substituting 5,7-difluoro-1,2,3,4-tetrahydronaphthalen-2-ylamine gave 5-cyano-1-(5,7-difluoro-1,2,3,4-tetrahydronaphthalen-2-yl)-1,3-dihydroimidazole-2-thione, m.p. 255°C (dec) ;
substituting (*R*)-5,7-difluoro-1,2,3,4-tetrahydronaphthalen-2-ylamine gave (*R*)-5-cyano-1-(5,7-difluoro-1,2,3,4-tetrahydronaphthalen-2-yl)-1,3-dihydroimidazole-2-thione;
substituting (*S*)-7-fluoro-1,2,3,4-tetrahydronaphthalen-2-ylamine gave (*S*)-5-cyano-1-(7-fluoro-1,2,3,4-tetrahydronaphthalen-2-yl)-1,3-dihydroimidazole-2-thione;
substituting (*S*)-6-fluoro-1,2,3,4-tetrahydronaphthalen-2-ylamine gave (*S*)-5-cyano-1-(6-fluoro-1,2,3,4-tetrahydronaphthalen-2-yl)-1,3-dihydroimidazole-2-thione;
substituting (*S*)-5-fluoro-1,2,3,4-tetrahydronaphthalen-2-ylamine gave (*S*)-5-cyano-1-(5-fluoro-1,2,3,4-tetrahydronaphthalen-2-yl)-1,3-dihydroimidazole-2-thione;
substituting (*S*)-1,2,3,4-tetrahydronaphthalen-2-ylamine gave (*S*)-5-cyano-1-(1,2,3,4-tetrahydronaphthalen-2-yl)-1,3-dihydroimidazole-2-thione;
substituting 6,7-difluoro-1,2,3,4-tetrahydronaphthalen-2-ylamine gave 5-cyano-1-(6,7-difluoro-1,2,3,4-tetrahydronaphthalen-2-yl)-1,3-dihydroimidazole-2-thione;
substituting (*S*)-6,7-difluoro-1,2,3,4-tetrahydronaphthalen-2-ylamine gave (*S*)-5-cyano-1-(6,7-difluoro-1,2,3,4-tetrahydronaphthalen-2-yl)-1,3-dihydroimidazole-2-thione; and

### Example 14

### (S)-5-Aminomethyl-1-(5,7-difluoro-1,2,3,4-tetrahydronaphthalen-2-yl)-1,3-dihydroimidazole-2-thioxo Hydrochloride

The following is the preparation of a compound of Formula I(a) in which t is 2, R¹ is fluoro at the 5- and 7-position, R³ and R⁴ are hydro and R⁵ is aminomethyl.

A solution of (*S*)-5-cyano-1-(5,7-difluoro-1,2,3,4-tetrahydronaphthalen-2-yl)-1,3-dihydroimidazole-2-thione (5.0 g, 0.017 mol), prepared as in Example 13, in 75 mL of THF was stirred under argon in an ice bath and a solution of LAH in THF (1.0 M, 34.3 mL, 34.3 mmol) was added dropwise over 10 minutes. The mixture was cooled to 0°C, stirred for 30 minutes, allowed to warm to room temperature, and let stand for 1.5 hours. The mixture was cooled to 0°C and then a sufficient amount of saturated sodium potassium tartrate solution was added such that the mixture could by freely stirred. An additional 30 mL of saturated sodium potassium tartrate solution and 200 mL of 10% methanol in methylene chloride were added, the mixture was stirred for 15 minutes, and then 100-150 mL of water was added. The organic layer was separated and the aqueous phase was extracted twice with 10% methanol in methylene chloride (2x 125 mL). The combined extracts were washed with 75 mL of water, dried over magnesium sulfate, and concentrated by evaporation to a residue (5.2 g). Purification by column chromatography on silica gel (elution: 5% methanol/methylene chloride) gave (*S*)-5-aminomethyl-1-(5,7-difluoro-1,2,3,4-tetrahydronaphthalen-2-yl)-1,3-dihydroimidazole-2-thione (2.92 g, 10.0 mmol).

The (*S*)-5-aminomethyl-1-(5,7-difluoro-1,2,3,4-tetrahydronaphthalen-2-yl)-1,3-dihydroimidazole-2-thione was dissolved in methanol and treated with 1.5 equivalents of anhydrous hydrogen chloride in ethyl ether. Removal of the solvents by co-evaporation with ethyl acetate gave (*S*)-5-aminomethyl-1-(5,7-difluoro-1,2,3,4-tetrahydronaphthalen-2-yl)-1,3-dihydroimidazole-2-thione hydrochloride, m.p. 245°C, [α]_{D}²⁵ = 11.30° (c = 0.5, DMSO).

Proceeding as in Example 14, but substituting a different starting material for (*S*)-5-cyano-1-(5,7-difluoro-1,2,3,4-tetrahydronaphthalen-2-yl)-1,3-dihydroimidazole-2-thione gave the following compounds of Formula I:
substituting 5-cyano-1-(5,7-difluoro-1,2,3,4-tetrahydronaphthalen-2-yl)-1,3-dihydroimidazole-2-thione gave 5-aminomethyl-1-(5,7-difluoro-1,2,3,4-tetrahydronaphthalen-2-yl)-1,3-dihydroimidazole-2-thione, m.p. 172-178°C and 5-aminomethyl-1-(5,7-difluoro-1,2,3,4-tetrahydronaphthalen-2-yl)-1,3-dihydroimidazole-2-thione hydrochloride, m.p. 265-270°C;
substituting (*R*)-5-cyano-1-(5,7-difluoro-1,2,3,4-tetrahydronaphthalen-2-yl)-1,3-dihydroimidazole-2-thione gave (*R*)-5-aminomethyl-1-(5,7-difluoro-1,2,3,4-tetrahydronaphthalen-2-yl)-1,3-dihydroimidazole-2-thione hydrochloride;
substituting (*S*)-5-cyano-1-(7-fluoro-1,2,3,4-tetrahydronaphthalen-2-yl)-1,3-dihydroimidazole-2-thione gave (*S*)-5-aminomethyl-1-(7-fluoro-1,2,3,4-tetrahydronaphthalen-2-yl)-1,3-dihydroimidazole-2-thione hydrochloride, m.p. 237-247°C;
substituting (*S*)-5-cyano-1-(6-fluoro-1,2,3,4-tetrahydronaphthalen-2-yl)-1,3-dihydroimidazole-2-thione gave (*S*)-5-aminomethyl-1-(6-fluoro-1,2,3,4-tetrahydronaphthalen-2-yl)-1,3-dihydroimidazole-2-thione hydrochloride, m.p. 240-249°C;
substituting (*S*)-5-cyano-1-(5-fluoro-1,2,3,4-tetrahydronaphthalen-2-yl)-1,3-dihydroimidazole-2-thione gave (*S*)-5-aminomethyl-1-(5-fluoro-1,2,3,4-tetrahydronaphthalen-2-yl)-1,3-dihydroimidazole-2-thione hydrochloride, m.p. 273-276°C;
substituting (*S*)-5-cyano-1-(1,2,3,4-tetrahydronaphthalen-2-yl)-1,3-dihydroimidazole-2-thione gave (*S*)-5-aminomethyl-1-(1,2,3,4-tetrahydronaphthalen-2-yl)-1,3-dihydroimidazole-2-thione hydrochloride, m.p. 255-258°C; substituting 5-cyano-1-(6,7-difluoro-1,2,3,4-tetrahydronaphthalen-2-yl)-1,3-dihydroimidazole-2-thione gave 5-aminomethyl-1-(6,7-difluoro-1,2,3,4-tetrahydronaphthalen-2-yl)-1,3-dihydroimidazole-2-thione hydrochloride, m.p. 260-263°C (dec);
substituting (*S*)-5-cyano-1-(6,7-difluoro-1,2,3,4-tetrahydronaphthalen-2-yl)-1,3-dihydroimidazole-2-thione gave (*S*)-5-aminomethyl-1-(6,7-difluoro-1,2,3,4-tetrahydronaphthalen-2-yl)-1,3-dihydroimidazole-2-thione hydrochloride, m.p. 253-270°C.

### Example 15

### 1-(5,7-Difluoro-1,2,3,4-tetrahydronaphthalen-2-yl)-5(1H)-tetrazol-5-yl-1,3-dihydroimidazole-2-thione

The following is the preparation of a compound of Formula I(a) in which t is 2, R¹ is fluoro at the 5- and 7-position and R⁵ is 1*H*-tetrazol-5-yl.

A mixture of 5-cyano-1-(5,7-difluoro-1,2,3,4-tetrahydronaphthalen-2-yl)-1,3-dihydroimidazole-2-thione (0.554 g, 1.9 mmol), prepared as in Example 19, in 1.6 mL of tributyltin azide was heated at 130°C under a nitrogen atmosphere for 2.5 hours and then 10 mL of toluene was added. The mixture was allowed to cool to room temperature and approximately 5 mL of diethyl ether was added. The mixture was cooled to 0°C and then treated with 10 mL of 1N hydrogen chloride in diethyl ether for approximately 15 minutes. The mixture was poured into a solution of potassium fluoride monohydrate (15.0 g) in 15 to 20 mL of water and 75 mL of ethyl acetate. The ethyl acetate layer was extracted with 2*N* sodium hydroxide and the aqueous layer was washed 6 times with methylene chloride. The aqueous layer was acidified with concentrated hydrochloric acid and then extracted with ethyl acetate. Evaporation of the solvents gave 1-(5,7-difluoro-1,2,3,4-tetrahydronaphthalen-2-yl)-5(1*H*)-tetrazol-5-yl-1,3-dihydro-imidazole-2-thione (0.57 g, 1.7 mmol), m.p. 214-215°C.

Proceeding as in Example 15, but substituting a different starting material for 5-cyano-1-(5,7-difluoro-1,2,3,4-tetrahydronaphthalen-2-yl)-1,3-dihydroimidazole-2-thione gave the following compound of Formula I:
substituting 5-cyano-1-(6,7-difluoro-1,2,3,4-tetrahydronaphthalen-2-yl)-1,3-dihydroimidazole-2-thione gave 1-(6,7-difluoro-1,2,3,4-tetrahydronaphthalen-2-yl)-5(1*H*)-tetrazol-5-yl-1,3-dihydroimidazole-2-thione, m.p. 195-212°C.

### Example 16

### 3-(1,2,3,4-Tetrahydronaphthalen-2-yl)-2-thioxo-2,3-dihydro-1H-imidazole-5-carbaldehyde

The following is the preparation of a compound of Formula I(a) in which t is 0, R³ and R⁵ are each hydro and R⁴ is formyl.

A mixture of 2-isothiocyano-1,2,3,4-tetrahydronaphthalene (1.89 g, 10 mmol), prepared as in Example 7, and D-(+)-glucosamine (1.78 g, 10 mmol) was stirred at 90°C until homogenous and then 0.8 mL of acetic acid was added. The mixture was stirred at 90°C for 30 minutes and then cooled. The solvents were removed by rotary evaporation and the residue was co-evaporated with toluene (2 x 25 mL). The residue was dissolved in acetic acid and heated at 90 to 100°C for 30 minutes. The mixture was cooled and triturated with acetone giving a crystalline material. The material was isolated by filtration and the filter residue washed with acetone. Drying gave 4-(1*R*,2*R*,3*S*,4)-tetrahydroxybut-1-yl)-1-(1,2,3,4-tetrahydronaphthalen-2-yl)-1,3-dihydroimidazole-2-thione (1.48 g, 4.23 mmol).

A suspension of 4-(1*R*,2*R*,3*S*,4)-tetrahydroxybut-1-yl)-1-(1,2,3,4-tetrahydronaphthalen-2-yl)-1,3-dihydroimidazole-2-thione (0.252 g, 0.72 mmol) and lead tetraacetate (0.851 g, 1.92 mmol) in 15 mL of 33% acetic acid/benzene was stirred until the mixture was homogenous and 30 minutes. The reaction mixture was poured into 125 mL of saturated sodium carbonate solution and the mixture was filtered. The organic layer was separated, dried over magnesium sulfate and concentrated. The residue was dissolved in 40 mL of THF and 2 mL of sulfurous acid (6% SO₂). Evaporation of the solvent gave 3-(1,2,3,4-tetrahydronaphthalen-2-yl)-2-thioxo-2,3-dihydro-1*H*-imidazole-5-carbaldehyde (0.15 g, 0.59 mmol), m.p. 206-210°C.

Proceeding as in Example 16, but substituting (*S*)-2-isothiocyano-5,7-difluoro-1,2,3,4-tetrahydronaphthalene for 2-isothiocyano-1,2,3,4-tetrahydronaphthalene gave (*S*)-3-(5,7-difluoro-1,2.3,4-tetrahydronaphthalen-2-yl)-2-thioxo-2,3-dihydro-1*H*-imidazole-5-carbaldehyde, m.p. >285°C.

### Example 17

### Ethyl [(5,7-Difluoro-1,2,3,4-tetrahydronaphthalen-2-yl)(formyl)amino]acetate

The following is the preparation of a compound of Formula 17 in which t is 2, R¹ is fluoro at the 5- and 7-position and R³² is ethozycarbonyl.

A mixture of 5,7-difluoro-1,2,3,4-tetrahydronaphthalen-2-ylamine (6.0 g, 32.8 mmol), prepared as in Example 6, and ethyl glyoxylate (4.6 g, 36.0 mmol) in 300 mL of ethanol was hydrogenated over 10% palladium on carbon (0.75 g) for 10 hours. The mixture was filtered and concentrated by evaporation. Purification of the residue by column chromatography on silica gel (elution: 30% ethyl acetate/hexane) gave ethyl [(5,7-difluoro-1,2,3,4-tetrahydro-naphthalen-2-yl)amino]acetate (7.5 g, 27.9 mmol) as an oil.

A solution of ethyl [(5,7-difluoro-1,2,3,4-tetrahydronaphthalen-2-yl)-amino]acetate (7.15 g, 25.6 mmol) in 20 mL of methylene chloride under argon was cooled to approximately 0°C. Acetic formic anhydride (9.7 mL, 67.3 mmol) was cooled to 0°C and added over 5 to 10 minutes. The mixture was stirred at 0°C for 2 hours and then allowed to warm to room temperature. The solvent was removed by co-evaporation with toluene (3 x 50 mL). Crystallization of the residue under high vacuum gave ethyl [(5,7-difluoro-1,2,3,4-tetrahydro-naphthalen-2-yl)(formyl)amino]acetate (7.68 g, 25.0 mmol).

Proceeding as in Example 17, but substituting a different starting material for 5,7-difluoro-1,2,3,4-tetrahydronaphthalen-2-ylamine gave the following compounds of Formula 17:
substituting 6,8-difluoro-1,2,3,4-tetrahydronaphthalen-2-ylamine gave ethyl [(6,8-difluoro-1,2,3,4-tetrahydronaphthalen-2-yl) (formyl)amino]acetate.

### Example 18

### Ethyl 3-(5,7-difluoro-1,2,3,4-tetrahydronaphthalen-2-yl)-2-thioxo-2,3-dihydro-1H-imidazole-4-carboxylate

The following is the preparation of a compound of Formula I(a) in which t is 2, R¹ is fluoro at the 5- and 7-position, R³ and R⁴ are hydro and R⁵ is ethoxycarbonyl.

A mixture of ethyl [(5,7-difluoro-1,2,3,4-tetrahydronaphthalen-2-yl)-(formyl)amino]acetate (7.58 g, 24.7 mmol), prepared as in Example 17, and ethyl formate (5.91 mL, 72.7 mmol) in 60 mL of THF was cooled to -10°C under argon. Potassium tert-butoxide (4.08 g, 36.4 mmol) in 45 mL of THF was added and the mixture was stirred at -10°C for 2 hours. The mixture then was allowed to warm to room temperature and stirred for an additional 4 hours. The solvent was removed by evaporation and the residue was dissolved in 75 mL of 1N hydrochloric acid and 50 mL of ethanol. Potassium thiocyanate (2.65 g, 75 mmol) was added and the mixture was stirred at 85°C for 15 hours. The mixture was cooled, diluted with 125 mL of water and extracted with ethyl acetate. The extract was dried over magnesium sulfate and the ethyl acetate was removed by rotary evaporation. Purification of the residue by column chromatography on silica gel (elution: 2.5% methanol/methylene chloride) gave ethyl 3-(5,7-difluoro-1,2,3,4-tetrahydronaphthalen-2-yl)-2-thioxo-2,3-dihydro-1*H*-imidazole-4-carboxylate (8.07 g, 24.6 mmol), m.p. 159-161°C.

Proceeding as in Example 18, but substituting a different starting material for ethyl [(5,7-difluoro-1,2,3,4-tetrahydronaphthalen-2-yl)-(formyl)amino]acetate gave the following compounds of Formula I:
substituting ethyl [(6,8-difluoro-1,2,3,4-tetrahydronaphthalen-2-yl)-(formyl)amino]acetate gave ethyl 3-(6,8-difluoro-1,2,3,4-tetrahydronaphthalen-2-yl)-2-thioxo-2,3-dihydro-1*H*-imidazole-4-carboxylate, m.p. 187-189°C;
substituting ethyl (1,2,3,4-tetrahydronaphthalen-2-yl)(formyl)aminoacetate gave ethyl 3-(1,2,3,4-tetrahydronaphthalen-2-yl)-2-thioxo-2,3-dihydro-1*H*-imidazole-4-carboxylate, m.p. 70-72;
substituting ethyl (7-fluoro-1,2,3,4-tetrahydronaphthalen-2-yl)(formyl)-aminoacetate gave ethyl 3-(7-fluoro-1,2,3,4-tetrahydronaphthalen-2-yl)-2-thioxo-2,3-dihydro-1*H*-imidazole-4-carboxylate.

### Example 19

### Ethyl 3-(1,2,3,4-tetrahydronaphthalen-2-yl]-5 -methyl-2-thioxo-2,3-dihydro-1H-imidazole-4-carboxylate

The following is the preparation of a compound of Formula I(a) in which t is 0, R³ is hydro, R⁴ is methyl and R⁵ is ethoxycarbonyl.

A solution of diisopropylamine (1.54 mL, 11 mmol) in 30 mL of dry THF under argon was cooled to 0°C and n-butyllithium (6.25 mL, 10 mmol) was added. The mixture was cooled to -78°C and ethyl [(1,2,3,4-tetrahydronaphthalen-2-yl)-(formyl)amino]acetate (1.33 g, 5.0 mmol), prepared as in Example 17, in 20 mL of THF was added over 15 minutes. The mixture was stirred at -78°C for 1 hour and acetyl chloride (0.427 mL, 6.0 mmol) was added over 5 minutes. The mixture was stirred for 3 hours at -78°C and then warmed to room temperature over 1 hour. The solvent was removed by evaporation and the residue was dissolved in 25 mL of 1N hydrochloric acid and 25 mL of ethanol. Potassium thiocyanate (1.94 g, 20 mmol) was added and the mixture was stirred at 75-80°C for 18 hours. The mixture was cooled, diluted with water and extracted twice with ethyl acetate. The ethyl acetate was concentrated to a dark oil. Purification of the residue by column chromatography on silica gel (elution: 2% methanol/methylene chloride) and trituration with ethyl acetate/isopropyl ether gave ethyl 3-(1,2,3,4-tetrahydronaphthalen-2-yl)-5-methyl-2-thioxo-1,3-dihydro-1*H*-imidazole-4-carboxylate, m.p. 225-227°C, as a light yellow solid.

Proceeding as in Example 19, but substituting a different material for ethyl [(1,2,3,4-tetrahydronaphthalen-2-yl)(formyl)amino]acetate and/or acetyl chloride gave the following compounds of Formula I:
substituting isobutyryl chloride gave ethyl 3-(1,2,3,4-tetrahydronaphthalen-2-yl)-5-prop-2-yl-2-thioxo-1,3-dihydro-1*H*-imidazole-4-carboxylate, m.p. 195-197°C;
substituting trimethylacetyl chloride gave ethyl 3-(1,2,3,4-tetrahydronaphchalen-2-yl)-5-(1,1-dimethylethyl)-2-thioxo-1,3-dihydro-1*H*-imidazole-4-carboxylate as a foam;
substituting ethyl (5,7-difluoro-1,2,3,4-tetrahydronaphthalen-2-yl) (formyl)-aminoacetate gave ethyl 3-(5,7-difluoro-1,2,3,4-tetrahydronaphthalen-2-yl)-5-methyl-2-thioxo-1,3-dihydro-1*H*-imidazole-4-carboxylate, m.p. 207-209°C; and
substituting ethyl oxalyl chloride gave ethyl 3-(5,7-difluoro-1,2,3,4-tetrahydronaphthalen-2-yl)-5-ethoxycarbonyl-2-thioxo-1,3-dihydro-1*H*-imidazole-4-carboxylate, m.p. 160-161°C.

### Example 20

### (S)-N-[3-(5,7-Difluoro-1,2,3,4-tetrahydronaphthalen-2-yl)-2-thioxo-2,3-dihydro-1H-imidazol-4-ylmethyl]formamide

The following is the preparation of a compound of Formula I(a) in which t is 2, R¹ is fluoro at the 5- and 7-position and R⁵ is formylaminomethyl.

Formamide (250 mL, 6.3 mol) was heated to 175°C and (*S*)-5-hydroxymethyl-1-(5,7-difluoro-1,2,3,4-tetrahydronaphthalen-2-yl)-1,3-dihydroimidazole-2-thione (25.0 g, 88.3 mmol), prepared as in Example 18, was added in portions over 30 minutes and the reaction mixture was stirred for 1 hour under a nitrogen sweep. The mixture was cooled to 50°C and 2.5 g of activated carbon, Darco®, was added. The mixture was cooled to 30°C, filtered through Celite and washed in with 25 mL of formamide. The filtrate was heated to 95°C and then 1 L of water was added dropwise. The mixture was allowed to cool and then stirred at room temperature for 12 hours. The mixture was cooled to 0°C giving a crystalline material. The material was isolated by filtration and dried.

The material was stirred with approximately 5 times by weight of 70% THF/30% hexanes for five minutes. The material was isolated by filtration and the filter residue was washed with 50% THF/50% hexanes. Drying to constant weight gave (*S*)-*N*-[3-(5,7-difluoro-1,2,3,4-tetrahydronaphthalen-2-yl)-2-thioxo-2,3-dihydro-1*H*-imidazol-4-ylmethyl]formamide (19.5 g, 62.7 mmol), m.p. 245-246°C. [α]_{D} +48.9° (c = 0.613, DMSO).

Proceeding as in Example 20, but substituting different starting materials for (*S*)-5-hydroxymethyl-1-(5,7-difluoro-1,2,3,4-tetrahydronaphthalen-2-yl)-1,3-dihydroimidazole-2-thione or formamide gave the following compounds of Formula I(a):
substituting (*S*)-5-hydroxymethyl-1-(6,7-dichloro-1,2,3,4-tetrahydronaphthalen-2-yl)-1,3-dihydroimidazole-2-thione gave (*S*)-*N*-[3-(6,7-difluoro-1,2,3,4-tetrahydronaphthalen-2-yl)-2-thioxo-2,3-dihydro-1*H*-imidazol-4-yl-methyl]formamide; and
substituting urea gave (*S*)-5-ureidomethyl-1-(5,7-difluoro-1,2,3,4-tetrahydro-napbthalen-2-yl)-1,3-dihydroimidazole-2-thione, m.p. 258-260°C, [α]_{D} +34.3° (c = 0.574, DMSO).

### Example 21

### (S)-N-[3-(5,7-Difluoro-1,2,3,4-tetrahydronaphthalen-2-yl)-2-thioxo-2,3-dihydro-1H-imidazol-4-ylmethyl]formamide

The following is the preparation of a compound of Formula I(a) in which t is 2, R¹ is fluoro at the 5- and 7-position and R⁵ is formylaminomethyl.

A mixture of (*S*)-5-hydroxymethyl-1-(5,7-difluoro-1,2,3,4-tetrahydronaphthalen-2-yl)-1,3-dihydroimidazole-2-thione (1.0 g, 3.5 mmol), prepared as in Example 18, and ammonium formate (10 g, 158.6 mmol) was stirred at approximately 125°C for 1 hour. The mixture was then heated to approximately 138°C and stirred for an additional 35 minutes. The mixture was diluted with 25 mL of water and allowed to cool to room temperature. The mixture was aged for approximately 18 hours giving a crystalline material. The material was isolated by filtration and the filter residue was washed with water. Drying to constant weight gave (*S*)-*N*-[3-(5,7-difluoro-1,2,3,4-tetrahydronaphthalen-2-yl)-2-thioxo-2,3-dihydro-1*H*-imidazol-4-ylmethyl]formamide (0.92 g, 2.96 mmol).

Proceeding as in Example 21, but substituting ammonium acetate for ammonium formate gave (*S*)-*N*-[3-(5,7-difluoro-1,2,3,4-tetrahydronaphthalen-2-yl)-2-thioxo-2,3-dihydro-1*H*-imidazol-4-ylmethyl]acetamide, m.p. 275.5-276°C dec. [α]_{D} +41.3° (c = 1.00, DMSO).

### Example 22

### (S)-5-Aminomethyl-1-(5,7-difluoro-1,2,3,4-tetrahydronaphthalen-2-yl)-1,3-dihydroimidazole-2-thion hydrochloride

The following is the preparation of a compound of Formula I (a) in which t is 2, R¹ is fluoro at the 5- and 7-position and R⁵ is aminomethyl.

A mixture of (*S*)-*N*-[3-(5,7-difluoro-1,2,3,4-tetrahydronaphthalen-2-yl)-2-thioxo-2,3-dihydro-1*H*-imidazol-4-ylmethyl]formamide (19.1 g, 59.0 mmol), prepared as in Example 30, and 25 mL of concentrated hydrochloric acid (12.0 M, 25 mL, 300 mmol) in 400 mL of isopropanol was heated to reflux over 12 minutes and stirred for 1 hour 40 minutes. The mixture was distilled removing 150 mL of isopropanol. The mixture was gradually cooled to room temperature and stirred for 3 hours 45 minutes. The material was isolated by filtration and the filter residue was washed with 75 mL of isopropanol. Drying *in vacuo* at 110 to 125°C with a nitrogen bleed gave (*S*)-5-aminomethyl-1-(5,7-difluoro-1,2,3,4-tetrahydro-naphthalen-2-yl)-1,3-dihydroimidazole-2-thione hydrochloride (15.6 g, 47.1 mmol), m.p. 251.9°C. [α]_{D} +10.2° (c = 0.500, DMSO).

Proceeding as in Example 22, but substituting different starting materials for (*S*)-*N*-[3-(5,7-difluoro-1,2,3,4-tetrahydronaphthalen-2-yl)-2-thioxo-2,3-dihydro-1*H*-imidazol-4-ylmethyl]formamide gave the following compounds of Formula I:
substituting (*S*)-*N*-[3-(6,7-dichloro-1,2,3,4-tetrahydronaphthalen-2-yl)-2-thioxo-2,3-dihydro-1*H*-imidazol-4-yl-methyl]formamide gave (*S*)-5-aminomethyl-1-(6,7-dichloro-1,2,3,4-tetrahydronaphthalen-2-yl)-1,3-dihydroimidazole-2-thione hydrochloride, m.p. 190°C dec;
substituting (*S*)-4-{2-[4-formylaminomethyl-3-(5,7-difluoro-1,2,3,4-tetrahydro-naphthalen-2-yl)-2-thioxo-2,3-dihydro-1*H*-imidazol-1-yl]ethyl}benzoic acid gave (*S*)-4-{2-[4-aminomethyl-3-(5,7-difluoro-1,2,3,4-tetrahydronaphthalen-2-yl)-2-thioxo-2,3-dihydro-1*H*-imidazol-1-yl]ethyl}benzoic acid hydrochloride, m.p. 246-248°C; and
substituting (*S*)-3-[4-formylaminomethyl-3-(5,7-difluoro-1,2,3,4-tetrahydronaphthalen-2-yl)-2-thioxo-2,3-dihydro-1*H*-imidazol-1-yl]propionic acid gave (*S*)-3-[4-aminomethyl-3-(5,7-difluoro-1,2,3,4-tetrahydronaphthalen-2-yl)-2-thioxo-2,3-dihydro-1*H*-imidazol-1-yl]propionic acid, m.p. 191°C (eff.).

### Example 23

### (S)-1-(5,7-Difluoro-1,2,3,4-tetrahydronaphthalen-2-yl)-5-pyrrolidin-1-ylmethyl-1,3-dihydroimidazole-2-thione

The following is the preparation of a compound of Formula I (a) in which t is 2, R¹ is fluoro at the 5- and 7-position, R³ and R⁴ are hydro and R⁵ is pyrrolidin-1-ylmethyl.

A solution of (*S*)-1-(5,7-difluoro-1,2,3,4-tetrahydronaphthalen-2-yl)-5-hydroxymethyl-1,3-dihydroimidazole-2-thione (140 mg, 0.47 mmol), prepared as in Example 18, in 20 mL of THF and 1 drop of DMF was cooled to between 0 and 5°C and thionyl chloride (13.7 M, 109 µL, 1.49 mmol) was added drop-wise under a nitrogen atmosphere. The mixture was stirred at room temperature for 0.5 hours, under reflux for 0.5 hours and again at room temperature for 0.5 hours. The mixture then was cooled to between 0 and 5°C and pyrrolidine (12.0 M, 818 µL, 9.8 mmol) was added drop-wise. The mixture was stirred under reflux for 1.5 hours. The solvents were removed by evaporation and the residue was diluted with water. Ethyl acetate was added to the dilution and the mixture was adjusted to pH 7. The ethyl acetate layer was dried and concentrated by evaporation. Purification of the residue by column chromatography gave (*S*)-1-(5,7-difluoro-1,2,3,4-tetrahydronaphthalen-2-yl)-1,3-dihydro-5-(pyrrolidin-1-ylmethyl)-imidazole-2-thione (100 mg, 0.29 mmol). [α]_{D}²⁵ = -10.96° (c = 1.3, DMSO).

Treatment with 2 molar equivalents of 1M anhydrous hydrogen chloride in diethyl ether gave (*S*)-1-(5,7-difluoro-1,2,3,4-tetrahydronaphthalen-2-yl)-1,3-dihydro-5-(pyrrolidin-1-ylmethyl)imidazole-2-thione hydrochloride (100 mg, 0.26 mmol), m.p. 187-189°C.

Proceeding as in Example 23, but substituting a different starting material for (*S*)-5-hydroxymethyl-1-(5,7-difluoro-1,2,3,4-tetrahydronaphthalen-2-yl)-1,3-dihydroimidazole-2-thione gave the following compounds of Formula I:
substituting methylamine gave (*S*)-1-(5,7-difluoro-1,2,3,4-tetrahydronaphthalen-2-yl)-5-(methylaminomethyl)-1,3-dihydroimidazole-2-thione, m.p. 250-260°C, and (*S*)-1-(5,7-difluoro-1,2,3,4-tetrahydronaphthalen-2-yl)-5-(methylaminomethyl)-1,3-dihydroimidazole-2-thione hydrochloride, m.p. 250°C, [α]_{D}²⁵ = +7.7° (c = 2.4, DMSO) ;
substituting dimethylamine gave (*S*)-1-(5,7-difluoro-1,2,3,4-tetrahydronaphthalen-2-yl)-5-(dimethylaminomethyl)-1,3-dihydroimidazole-2-thione and (*S*)-1-(5,7-difluoro-1,2,3,4-tetrahydronaphthalen-2-yl)-5-(dimethylaminomethyl)-1,3-dihydroimidazole-2-thione hydrochloride, m.p. 207-208°C;
substituting piperidine gave (*S*)-1-(5,7-difluoro-1,2,3,4-tetrahydronaphthalen-2-yl)-5-(piperidin-1-ylmethyl)-1,3-dihydroimidazole-2-thione and (*S*)-1-(5,7-difluoro-1,2,3,4-tetrahydronaphthalen-2-yl)-5-(piperidin-1-ylmethyl)-1,3-dihydroimidazole-2-thione hydrochloride, m.p. 169-170°C;
substituting morpholine gave (*S*)-1-(5,7-difluoro-1,2,3,4-tetrahydronaphthalen-2-yl)-5-(morpholin-4-ylmethyl)-1,3-dihydroimidazole-2-thione, m.p. 198-201, [α]_{D}²⁵ = -7.56° (c = 2.38, DMSO) and (*S*)-1-(5,7-difluoro-1,2,3,4-tetrahydronaphthalen-2-yl)-5-(morpholin-4-ylmethyl)-1,3-dihydroimidazole-2-thione hydrochloride, m.p. 182-184°C; and
substituting 1-methylpiperazine gave (*S*)-1-(5,7-difluoro-1,2,3,4-tetrahydro-naphthalen-2-yl)-5-(4-methylpiperazin-1-ylmethyl)-1,3-dihydroimidazole-2-thione and (*S*)-1-(5,7-difluoro-1,2,3,4-tetrahydronaphthalen-2-yl)-5-(4-methyl-piperazin-1-ylmethyl)-1,3-dihydroimidazole-2-thione hydrochloride, m.p. 237-245°C.

### Example 24

### 1-(1,2,3,4-Tetrahydronaphthalen-2-yl)-4,5-di(hydroxmethyl)-1,3-dihydroimidazole-2-thione

The following is the preparation of a compound of Formula I(a) in which t is 0, R³ is hydro and R⁴ and R⁵ are each hydroxymethyl.

A mixture of sodium borohydride (0.22 g, 5.8 mmol) and anhydrous calcium chloride 0.34 g, 3.1 mmol) in 10 mL of dry THF was stirred at approximately 25°C for 1 hour and then ethyl 3-(1,2,3,4-tetrahydronaphthalen-2-yl)-5-ethoxycarbonyl-2-thioxo-1,3-dihydro-1*H*-imidazole-4-carboxylate (0.37 g, 1 mmol), prepared as in Example 19, in 10 mL of dry THF was added. The mixture was stirred at 50°C for approximately 72 hours and then concentrated. The residue was treated with 20 mL of 10% sodium hydroxide and 50 mL of ethyl acetate and filtered and the aqueous phase was extracted again with ethyl acetate (3x 50 mL). The combined extracts were dried (MgSO₄) and concentrated. The residue was stirred with methylene chloride/methanol (93:7) and the mixture was filtered. The above aqueous phase was evaporated to dryness and the residue was stirred with methanol. The methanol mixture was filtered and then combined with the methylene chloride/methanol filtrate. The combined mixture was concentrated and the residue was purified by flash chromatography on silica gel eluting with methylene chloride/methanol (93:7 to 96:4) to give 1-(1,2,3,4-tetrahydronaphthalen-2-yl)-4,5-di(hydroxmethyl)-1,3-dihydro-imidazole-2-thione (35 mg, 0.12 mmol), m.p. 199-200°C.

### Example 25

### Ethyl 3-[3-(1,2,3,4-tetrahydronaphthalen-2-yl)-2-thioxo-2,3-dihydro-1H-imidazol-1-yl]propionate

The following is the preparation of a compound of Formula I(a) in which t is 0, R³ is 2-ethoxycarbonylethyl and R⁴ and R⁵ are each hydro.

A mixture of 1-(1,2,3,4-tetrahydronaphthalen-2-yl)-1,3-dihydroimidazole-2-thione (1.3 g, 5.6 mmol), prepared as in Example 9, and ethyl acrylate (3.1 mL, 28.2 mmol) in 14 mL of ethanol and 1.28 mL of *N*-benzyltrimethylammonium hydroxide (2.8 mmol) in methanol was heated at 80°C under nitrogen for 2 hours. The mixture was allowed to cool and concentrated by rotoevaporation. The residue was purified by chromatography on silica gel eluting with hexanes/ethyl acetate (3:1) to give ethyl 3-[3-(1,2,3,4-tetrahydronaphthalen-2-yl)-2-thioxo-2,3-dihydro-1*H*-imidazol-1-yl]propionate (1.2 g, 3.7 mmol), m.p. 71-73°C.

Proceeding as in Example 25, but substituting different starting materials for 1-(1,2,3,4-tetrahydronaphthalen-2-yl)-1,3-dihydroimidazole-2-thione gave the following compounds of Formula I:
substituting tert-butyl 4-(1,2,3,4-tetrahydronaphthalen-2-yl)-5-thioxo-1,5-dihydro[1,2,4]triazol-3-ylmethylaminoformate gave ethyl 3-[4-(1,2,3,4-tetrahydronaphthalen-2-yl)-3-(*tert*-butoxycarbonylaminomethyl)-5-thioxo-1,5-dihydro[1,2,4]triazol-1-yl]propionate; and
substituting (*S*)-(1-(5,7-difluoro-1,2,3,4-tetrahydronaphthalen-2-yl)-1,3-dihydroimidazole-2-thione gave ethyl (*S*)-3-[3-(5,7-difluoro-1,2,3,4-tetrahydronaphthalen-2-yl)-2-thioxo-2,3-dihydro-1*H*-imidazol-1-yl]-propionate, m.p. 105-107°C.

### Example 26

### Ethyl 3-[3-(1,2,3,4-tetrahydronaphthalen-2-yl)-4-dimethylaminomethyl 2-thioxo-2,3-dihydro-1H-imidazol-1-yl]propionate

The following is the preparation of a compound of Formula I(a) in which t is 0, R³ is 2-(ethoxycarbonyl)ethyl, R⁴ is hydro and R⁵ is dimethylaminomethyl.

A mixture of ethyl 3-[3-(1,2,3,4-tetrahydronaphthalen-2-yl)-2-thioxo-2,3-dihydro-1*H*-imidazol-1-yl]propionate (0.5 g, 1.5 mmol), prepared as in Example 34, and *N,N*-dimethylmethyleneammonium chloride (0.17 g, 1.8 mmol) in 7 mL of DMF was heated at 80°C under nitrogen for 16 hours. The mixture then was partitioned between saturated sodium bicarbonate solution and ethyl acetate. The organic layer was separated, washed with brine, dried (NaSO₄), filtered and concentrated. The residue was purified by column chromatography on silica gel eluting with ethyl acetate/hexane to give ethyl 3-[3-(1,2,3,4-tetrahydro-naphthalen-2-yl)-4-dimethylaminomethyl-2-thioxo-2,3-dihydro-1*H*-imidazol-1-yl]-propionate (277 mg, 0.7 mmol), m.p. 128-130°C.

Proceeding as in Example 26 but substituting methyl 4-[3-(1,2,3,4-tetrahydronaphthalen-2-yl)-5-methyl-2-thioxo-2,3-dihydro-1*H*-imidazol-1-ylmethyl]benzoate for ethyl 3-[3-(1,2,3,4-tetrahydronaphthalen-2-yl)-2-thioxo-2,3-dihydro-1*H*-imidazol-1-yl]propionate gave methyl 4-[3-(1,2,3,4-tetrahydronaphthalen-2-yl)-4-dimethylamino-5-methyl-2-thioxo-2,3-dihydro-1*H*-imidazol-1-ylmethyl]benzoate, as a foam.

### Example 27

### 1-(1,2,3,4-tetrahydronaphthalen-2-yl)-4,5-di(dimethylamino)-1,3-dihydroimidazole-2-thione

The following is the preparation of a compound of Formula I(a) in which t is 0, R³ is hydro and R⁴ and R⁵ are each dimethylaminomethyl.

A mixture of 1-(1,2,3,4-tetrahydronaphthalen-2-yl)-1,3-dihydroimidazole-2-thione (1 g, 4.3 mmol), prepared as in Reference Example 3, ethyl acrylate (4.7 mL, 43 mmol) and hydrochloric acid (1*N* in ether, 8.7 mL, 8.7 mmol) in 20 mL of ethanol was heated at 80°C under nitrogen for approximately 5 hours. The mixture was allowed to cool, concentrated and partitioned between saturated sodium bicarbonate solution and methylene chloride. The organic layer was separated and dried (K₂CO₃), filtered and concentrated. The residue was purified by column chromacography on silica gel eluting with methylene chloride/methanol (99:1) to give ethyl 3-[1-(1,2,3,4-tetrahydronaphthalen-2-yl)imidazol-2-yl-thio]propionate (1.36 g, 4.1 mmol).

A mixture of ethyl 3-[1-(1,2,3,4-tetrahydronaphthalen-2-yl)-imidazol-2-ylthio]propionate (1.36 g, 4.1 mmol) and *N,N*-dimethylmethylene ammonium chloride (1.66 g, 17.7 mmol) in 25 mL of DMF was heated at 100°C under nitrogen, for approximately 22 hours. The reaction mixture was allowed to cool to approximately 90°C and then additional *N,N*-dimethylmethyleneammonium chloride (0.83 g, 8.8 mmol) was added. The mixture was heated for 31.5 hours and then partitioned between sodium bicarbonate and ethyl acetate. The organic layer was separated, washed with brine, dried (K₂CO₃), filtered and concentrated. The residue was purified by column chromatography on silica gel eluting with 5-10% methanol in methylene chloride to give ethyl 3-[1-(1,2,3,4-tetrahydro-naphthalen-2-yl)-4,5-di(dimethylaminomethyl)imidazol-2-ylthio]propionate (0.55 g, 1.2 mmol).

A mixture of ethyl 3-[1-(1,2,3,4-tetrahydronaphthalen-2-yl)-4,5-di(dimethylaminomethyl)imidazol-2-ylthio]propionate (0.55 g, 1.2 mmol) and sodium ethoxide (3.5 mL of a solution prepared from 450 mg sodium in 45 mL of ethanol, 1.4 mmol) in 5 mL of ethanol was stirred at approximately 25°C for 1.75 hours. The mixture was concentrated and partitioned between water and ethyl acetate. The organic layer was separated, washed with brine, dried (K₂CO₃), filtered and concentrated. The residue was purified by column chromatography on silica gel eluting with methylene chloride/methanol (97:3) to give 1-(1,2,3,4-tetrahydronaphthalen-2-yl)-4,5-di(dimethylamino)-1,3-dihydro-imidazole-2-thione (0.24 g, 0.7 mmol), m.p. 182-184°C.

### Example 28

### 3-(5-7-Difluoro-1,2,3,4-tetrahydronaphthalen-2-yl)-2-thioxo-2,3-dihydro-1H-imidazole-4-carboxylic acid

The following is the preparation of a compound of Formula I(a) in which t is 2, R¹ is fluoro at the 5- and 7-position, R³ and R⁴ are hydro and R⁵ is carboxy.

A mixture of ethyl 3-(5,7-difluoro-1,2,3,4-tetrahydronaphthalen-2-yl)-2-thioxo-2,3-dihydro-1*H*-imidazole-4-carboxylate (4.6 g, 13.6 mmol), prepared as in Example 24, and potassium hydroxide (3.14 g, 47.6 mmol) in 130 mL of ethanol/water (10:3) was stirred at 85-90°C for 5 hours. The solvent was removed by evaporation and the residue was dissolved in water. The solution was acidified with 1N hydrochloric acid to a pH of 1 giving a crystalline material. Isolation of the material by filtration gave 3-(5,7-difluoro-1,2,3,4-tetrahydro-naphthalen-2-yl)-2-thioxo-2,3-dihydro-1*H*-imidazole-4-carboxylic acid (3.86 g, 12.5 mmol), m.p. 250-252°C.

Proceeding as in Example 28, but substituting a different starting material for ethyl 3-(5,7-difluoro-1,2,3,4-tetrahydronaphthalen-2-yl)-2-thioxo-2,3-dihydro-1*H*-imidazole-4-carboxylate gave the following compounds of Formula I(a):
substituting ethyl 3-(1,2,3,4-tetrahydronaphthalen-2-yl)-2-thioxo-2,3-dihydro-1*H*-imidazole-4-carboxylate gave 3-(1,2,3,4-tetrahydronaphthalen-2-yl)-2-thioxo-2,3-dihydro-1*H*-imidazole-4-carboxylic acid, m.p. 231-332°C (dec);
substituting ethyl 3-(7-fluoro-1,2,3,4-tetrahydronaphthalen-2-yl)-2-thioxo-2,3-dihydro-1*H*-imidazole-4-carboxylate gave 3-(7-fluoro-1,2,3,4-tetrahydronaphthalen-2-yl)-2-thioxo-2,3-dihydro-1*H*-imidazole-4-carboxylic acid, m.p. 207-209°C;
substituting ethyl 3-(6,8-difluoro-1,2,3,4-tetrahydronaphthalen-2-yl)-2-thioxo-2,3-dihydro-1*H*-imidazole-4-carboxylate gave 3-(6,8-difluoro-1,2,3,4-tetrahydro-naphthalen-2-yl)-2-thioxo-2,3-dihydro-1*H*-imidazole-4-carboxylic acid, m.p. 207-208°C, and treating with potassium hydroxide in methanol, evaporating to dryness and recrystallizing from methanol/isopropanol gave potassium 3-(6,8-difluoro-1,2,3,4-tetrahydronaphthalen-2-yl)-2-thioxo-2,3-dihydro-1*H*-imidazole-4-carboxylate, m.p. 160-163°C;
substituting methyl 3-[3-(1,2,3,4-tetrahydronaphthalen-2-yl)-2-thioxo-2,3-dihydro-1*H*-imidazol-1-ylmethyl]benzoate and sodium hydroxide gave 3-[3-(1,2,3,4-tetrahydronaphthalen-2-yl)-2-thioxo-2,3-dihydro-1*H*-imidazol-1-ylmethyl]benzoic acid, m.p. 252-254°C;
substituting methyl 4-[3-(1,2,3,4-tetrahydronaphthalen-2-yl)-2-thioxo-2,3-dihydro-1*H*-imidazol-1-ylmethyl]benzoate and sodium hydroxide gave 4-[3-(1,2,3,4-tetrahydronaphthalen-2-yl)-2-thioxo-2,3-dihydro-1*H*-imidazol-1-ylmethyl]benzoic acid, m.p. 211-212°C;
substituting methyl 4-[3-(1,2,3,4-tetrahydronaphthalen-2-yl)-2-thioxo-2,3-dihydro-1*H*-imidazol-1-yl]butyrate gave 4-[3-(1,2,3,4-tetrahydronaphthalen-2-yl)-2-thioxo-2,3-dihydro-1*H*-imidazol-1-yl]butyric acid, m.p. 156-158°C;
substituting methyl 5-[3-(1,2,3,4-tetrahydronaphthalen-2-yl)-2-thioxo-2,3-dihydro-1*H*-imidazol-1-ylmethyl]picolinate and recrystallizing from a solution of hydrochloric acid in ethanol gave 5-[3-(1,2,3,4-tetrahydronaphthalen-2-yl)-2-thioxo-2,3-dihydro-1*H*-imidazol-1-ylmethyl]picolinic acid hydrochloride, m.p. 204-205°C; substituting methyl 3-[3-(1,2,3,4-tetrahydronaphthalen-2-yl)-2-thioxo-2,3-dihydro-1*H*-imidazol-1-yl]propionate gave 3-[3-(1,2,3,4-tetrahydronaphthalen-2-yl)-2-thioxo-2,3-dihydro-1*H*-imidazol-1-yl]propionic acid, m.p. 167-168°C;
substituting methyl 4-[3-(1,2,3,4-tetrahydronaphthalen-2-yl)-5-methyl-2-thioxo-2,3-dihydro-1*H*-imidazol-1-ylmethyl]benzoate gave 4-[3-(1,2,3,4-tetrahydronaphthalen-2-yl)-5-methyl-2-thioxo-2,3-dihydro-1*H*-imidazol-1-ylmethyl]benzoic acid, m.p. 181-182°C;
substituting ethyl 3-[4-(1,2,3,4-tetrahydronaphthalen-2-yl)-3-(*tert*-butoxycarbonylaminomethyl)-5-thioxo-1,5-dihydro[1,2,4]triazol-1-yl]propionate gave 3-[4-(1,2,3,4-tetrahydronaphthalen-2-yl)-3-(*tert*-butoxycarbonylaminomethyl)-5-thioxo-1,5-dihydro[1,2,4]triazol-1-yl]propionic acid, m.p. 76-78;
substituting ethyl (*S*)-3-[3-(5,7-difluoro-1,2,3,4-tetrahydronaphthalen-2-yl)-2-thioxo-2,3-dihydro-1*H*-imidazol-1-yl]propionate gave (*S*)-3-[3-(5,7-difluoro-1,2,3,4-tetrahydronaphthalen-2-yl)-2-thioxo-2,3-dihydro-1*H*-imidazol-1-yl]propionic acid, m.p. 182-184°C;
substituting ethyl 3-[3-(1,2,3,4-tetrahydronaphthalen-2-yl)-4-dimethylamino-methyl-2-thioxo-2,3-dihydro-1*H*-imidazol-1-yl]propionate gave 3-[3-(1,2,3,4-tetrahydronaphthalen-2-yl)-4-dimethylaminomethyl-2-thioxo-2,3-dihydro-1*H*-imidazol-1-yl]propionic acid, m.p. 171-174°C;
substituting methyl (*S*)-4-{2-[3-(1,2,3,4-tetrahydronaphthalen-2-yl)-2-thioxo-2,3-dihydro-1*H-*imidazol-4-yl-methylamino]ethyl}benzoate gave (*S*)-4-{2-[3-(1,2,3,4-tetrahydronaphthalen-2-yl)-2-thioxo-2,3-dihydro-1*H*-imidazol-4-ylmethylamino]ethyl}benzoic acid hydrochloride, m.p. 240-241°C; and
substituting ethyl (*S*)-3-[4-formylaminomethyl-3-(5,7-difluoro-1,2,3,4-tetrahydronaphthalen-2-yl)-2-thioxo-2,3-dihydro-1*H*-imidazol-1-yl]-propionate gave (*S*)-3-[4-formylaminomethyl-3-(5,7-difluoro-1,2,3,4-tetrahydronaphthalen-2-yl)-2-thioxo-2,3-dihydro-1*H*-imidazol-1-yl]-propionic acid.

Proceeding as in Example 28, but substituting a different starting material for ethyl 3-(5,7-difluoro-1,2,3,4-tetrahydronaphthalen-2-yl)-2-thioxo-2,3-dihydro-1*H-*imidazole-4-carboxylate and performing an acid catalyzed hydrolysis gave the following compounds of Formula I(a):
substituting *tert*-butyl 3-(1,2,3,4-tetrahydronaphthalen-2-yl)-2-thioxo-2,3-dihydro-1*H*-imidazol-1-ylacetate and trifluoroacetic acid gave 3-(1,2,3,4-tetrahydronaphthalen-2-yl)-2-thioxo-2,3-dihydro-1*H*-imidazol-1-yl-acetic acid, m.p. 228-230°C;
substituting *tert*-butyl 1-(5,7-difluoro-1,2,3,4-tetrahydronaphthalen-2-yl)-2-thioxo-2,3-dihydro-1*H*-imidazol-4-ylmethylaminoacetate and hydrochloric acid gave 1-(5,7-difluoro-1,2,3,4-tetrahydronaphthalen-2-yl)-2-thioxo-2,3-dihydro-1*H*-imidazol-4-ylmethylaminoacetic acid hydrochloride, m.p. 214-216°C;
substituting *tert*-butyl (*S*)-1-(5,7-difluoro-1,2,3,4-tetrahydronaphthalen-2-yl)-2-thioxo-2,3-dihydro-1*H*-imidazol-4-ylmethylaminoacetate and hydrochloric acid gave (*S*)-1-(5,7-difluoro-1,2,3,4-tetrahydronaphthalen-2-yl)-2-thioxo-2,3-dihydro-1*H*-imidazol-4-ylmethylaminoacetic acid hydrochloride, m.p. 214°C (eff.) ; and
substituting tert-butyl 1-(1,2,3,4-tetrahydronaphthalen-2-yl)-2-thioxo-2,3-dihydro-1*H*-imidazol-4-ylmethylaminoacetate and hydrochloric acid gave 1-(1,2,3,4-tetrahydronaphthalen-2-yl)-2-thioxo-2,3-dihydro-1*H*-imidazol-4-yl-methylaminoacetic acid hydrochloride, m.p. 208-211°C.

### Example 29

### 4-[3-(1,2,3,4-Tetrahydronaphthalen-2-yl)-2-thioxo-2,3-dihydro-1H-imidazol-1-yl]butyramide

The following is the preparation of a compound of Formula I(a) in which t is 0, R³ is 4-(carbamoyl)propyl and R⁴ and R⁵ are each hydro.

A mixture 4-[3-(1,2,3,4-tetrahydronaphthalen-2-yl)-2-thioxo-2,3-dihydro-1*H*-imidazol-1-yl]butyric acid (100 mg, 0.32 mol), prepared as in Example 37, oxalyl chloride (2 M, 0.32 mL, 0.64 mol) and 5 drops of DMF in 10 mL of methylene chloride was stirred for 2 hours. Solvents and excess oxalyl chloride were removed by evaporation and the residue was treated with 5 mL of 30% aqueous ammonium hydroxide and stirred for 16 hours. The mixture was poured into aqueous sodium bicarbonate and extracted with methylene chloride. The extract was dried (Na₂SO₄) and concentrated. The residue was purified by flash chromatography on silica gel eluting with methylene chloride/methanol (99:1 to 96:4) to give 4-[3-(1,2,3,4-tetrahydronaphthalen-2-yl)-2-thioxo-2,3-dihydro-1*H*-imidazol-1-yl]butyramide (70 mg, 0.22 mmol), as a foam.

Proceeding as in Example 29 but substituting different starting materials for 4-[3-(1,2,3,4-tetrahydronaphthalen-2-yl)-2-thioxo-2,3-dihydro-1*H*-imidazol-1-yl]butyric acid gave the following compounds of Formula I:
substituting 4-[3-(1,2,3,4-tetrahydronaphthalen-2-yl)-2-thioxo-2,3-dihydro-1*H*-imidazol-1-ylethyl]benzoic acid gave 4-[3-(1,2,3,4-tetrahydronaphthalen-2-yl)-2-thioxo-2,3-dihydro-1*H*-imidazol-1-ylethyl]benzamide, m.p. 158-160°C; and
substituting 3-[3-(1,2,3,4-tetrahydronaphthalen-2-yl)-2-thioxo-2,3-dihydro-1*H*-imidazol-1-yl]propionic acid gave 3-[3-(1,2,3,4-tetrahydronaphthalen-2-yl)-2-thioxo-2,3-dihydro-1*H*-imidazol-1-yl]propionamide, m.p. 180-181°C.

### Example 30

### 3- [2-(4(1H)-Tetrazol-5-ylphenyl)ethyl]-1-(1,2,3,4-tetrehydronaphthalen-2-yl)-1,3-dihydroimidazole-2-thione

The following is the preparation of a compound of Formula I (a) in which t is 0, R³ is 2-(4(1*H*)-tetrazol-5-ylphenyl)ethyl and R⁴ and R⁵ are each hydro.

A mixture 3-[2-(4-cyanophenyl)ethyl]-1-(1,2,3,4-tetrahydronaphthalen-2-yl)-1,3-dihydroimidazole-2-thione (0.5 g, 1.4 mmol), prepared as in Example 10, and tributyltin azide (1.39 g, 4.2 mmol) in 3 mL of xylene was heated at 120°C under nitrogen for approximately 16 hours. The mixture was purified by chromatography on silica gel eluting with methylene chloride/methanol and the purified product was recrystallized from ethyl acetate/methanol to give 3-[2-(4(1*H*)-tetrazol-5-ylphenyl)ethyl]-1-(1,2,3,4-tetrahydronaphthalen-2-yl)-1,3-dihydroimidazole-2-thione (0.5 g, 1.4 mmol), m.p. 218-220°C.

### Example 31

### (S)-4-(1-hydroxy)ethyl-1-(5,7-difluoro-1,2,3,4-tetrahydronaphthalen-2-yl)-1,3-dihydroimidazole-2-thione

The following is the preparation of a compound of Foroula I(a) in which t is 2, R³ and R⁵ are each hydro and R⁴ is 1-hydroxyethyl.

A mixture of (*S*)-3-(5,7-difluoro-1,2,3,4-tetrahydronaphthalen-2-yl)-2-thioxo-2,3-dihydro-1*H*-imidazole-5-carbaldehyde (178 mg, 0.6 mmol), prepared as in Example 22, and methylmagnesium chloride (3 M, 0.4 mL, 1.2 mmol) was stirred at approximately 0°C for 1 hour and at approximately 25° for an additional 1 hour. The mixture was treated with 5 mL of dilute sulfuric acid and extracted with ethyl acetate (2x). The combined extracts were dried (MgSO₄) and concentrated and the residue was purified by flash chromatography on silica gel eluting with methylene chloride/methanol (98:2). The purified residue was further purified by preparative thin layer chromatography on silica gel eluting with methylene chloride/methanol (95:5) to give (*S*)-4-(1-hydroxy)ethyl-1-(5,7-difluoro-1,2,3,4-tetrahydronaphthalen-2-yl)-1,3-dihydroimidazole-2-thione (22 mg, 0.1 mmol), m.p. 210-211°C.

Proceeding as in Example 31, but substituting *n*-propylmagnesium chloride for methylmagnesium chloride gave (*S*)-4-(1-hydroxy)but-1-yl-1-(5,7-difluoro-1,2,3,4-tetrahydronaphthalen-2-yl)-1,3-dihydroimidazole-2-thione, m.p. 154-156°C.

### Example 32

### N-1H-Tetrazol-5-yl-3-(5,7-difluoro-1,2,3,4-tetrahydronaphthalen-2-yl)-2-thioro-2,3-dibydro-1H-imidazole-4-carboxamide

The following is the preparation of a compound of Formula I (a) in which t is 2, R¹ is fluoro at the 5- and 7-position and R³ is 1*H*-tetrazol-5-yl-carbamoyl.

3-(5,7-Difluoro-1,2,3,4-tetrahydronaphthalen-2-yl)-2-thioxo-2,3-dihydro-1*H*-imidazole-4-carboxylic acid (1 g, 3.33 mmol), prepared as in Example 28, was dissolved in 15 mL of oxalyl chloride and 1 drop of DMF and the solution was stirred under nitrogen for 3 hours. The excess oxalyl chloride was removed by rotary evaporation and the residue was co-evaporated with carbon tetrachloride (2 x 25 mL). The residue was cooled at 0°C and dry 5-amino-1*H*-tetrazole (0.85 g, 10 mmol) and 25 mL of pyridine were added. The mixture was allowed to warm to room temperature and then stirred for 16 hours. The solvent was removed by evaporation and the residue was co-evaporated with toluene. Purification of the residue by column chromatography on silica gel packed in 5% methanol/methylene chloride containing 1% acetic acid gave 0.9 g of impure product. The impure product was dissolved in aqueous potassium carbonate and the solution was extracted with ethyl acetate. The aqueous layer was acidified giving a solid material. Isolation of the material by filtration gave *N*-1*H*-tetrazol-5-yl-3-(5,7-difluoro-1,2,3,4-tetrahydronaphthalen-2-yl)-2-thioxo-2,3-dihydro-1*H*-imidazole-4-carboxamide (0.54 g, 1.56 mmol) as a light orange solid, m.p. 228-230°C.

### Example 33

### (4-Methylpiperazin-1-yl)[3-(5,7-difluoro-1,2,3,4-tetrahydronaphthalen-2-yl)-2-thioxo-2,3-dihydro-1H-imidazol-4-yl]methanone

The following is the preparation of a compound of Formula I(a) in which t is 2, R¹ is fluoro at the 5- and 7-position and R⁵ is 4-methyl-piperazin-1-ylcarbonyl.

A mixture of 3-(5,7-difluoro-1,2,3,4-tetrahydronaphthalen-2-yl)-2-thioxo-1,3-dihydro-1*H*-imidazole-4-carboxylic acid, prepared as in Example 28, (0.75 g, 2.42 mmol) and 1,1'-carbonyldiimidazole (0.43 g, 2.65 mmol) in 6 mL of THF was stirred under argon at room temperature for approximately 18 hours. *N*-Methylpiperazine (0.29 mL, 2.65 mmol) was added and the mixture was stirred under argon at room temperature for approximately 18 hours. The mixture was partitioned between methylene chloride and water. The methylene chloride layer was washed 4 times with water, dried over magnesium sulfate and concentrated by evaporation. Recrystallization of the residue from ethyl acetate/methanol gave (4-methylpiperazin-1-yl)[3-(5,7-difluoro-1,2,3,4-tetrahydronaphthalen-2-yl)-2-thioxo-2,3-dihydro-1*H*-imidazol-4-yl]methanone (0.69 g, 1.76 mmol), 248-250°C.

Proceeding as in Example 33, but substituting a different starting material for 3-(5,7-difluoro-1,2,3,4-tetrahydronaphthalen-2-yl)-2-thioxo-1,3-dihydro-1*H*-imidazole-4-carboxylic acid and/or *N*-methylpiperazine gave the following compounds of Formula I:
substituting 3-(6,8-difluoro-1,2,3,4-tetrahydronaphthalen-2-yl)-2-thioxo-1,3-dihydro-1*H*-imidazole-4-carboxylic acid gave (4-methylpiperazin-1-yl)[3-(6,8-difluoro-1,2,3,4-tetrahydronaphthalen-2-yl)-2-thioxo-2,3-dihydro-1*H*-imidazol-4-yl]methanone as an oil;
substituting 3-(5,7-difluoro-1,2,3,4-tetrahydronaphthalen-2-yl)-2-thioxo-1,3-dihydro-1*H*-imidazole-4-carboxylic acid and *N,N*-dimethylethylenediamine gave *N*-(2-dimethylaminoethyl)-3-(5,7-difluoro-1,2,3,4-tetrahydronaphthalen-2-yl)-2-thioxo-2,3-dihydro-1*H*-imidazole-4-carboxamide, m.p. 125°C;
substituting 3-(5,7-difluoro-1,2,3,4-tetrahydronaphthalen-2-yl)-2-thioxo-1,3-dihydro-1*H*-imidazole-4-carboxylic acid and p-methylsulfonylaminoaniline gave *N*-[4-(methylsulfonylamino)phenyl]-3-(5,7-difluoro-1,2,3,4-tetrahydronaphthalen-2-yl)-2-thioxo-2,3-dihydro-1*H*-imidazole-4-carboxamide, m.p. 225-230°C;
substituting 3-(5,7-difluoro-1,2,3,4-tetrahydronaphthalen-2-yl)-2-thioxo-1,3-dihydro-1*H*-imidazole-4-carboxylic acid and dimethylaminoethanethiol hydrochloride in the presence of dichlorohexylcarbodiimide gave 3-(5,7-difluoro-1,2,3,4-tetrahydronaphthalen-2-yl)-2-thioxo-2,3-dihydro-1*H*-imidazole-4-carbothioic acid *S*-(2-dimethylaminoethyl) ester, m.p. 204-206°C; and
substituting 3- (6,8-difluoro-1,2,3,4-tetrahydronaphthalen-2-yl)-2-thioxo-1,3-dihydro-1*H*-imidazole-4-carboxylic acid and dimethylaminoethanethiol hydrochloride in the presence of dichlorohexylcarbodiimide gave 3-(6,8-difluoro-1,2,3,4-tetrahydronaphthalen-2-yl)-2-thioxo-2,3-dihydro-1*H*-imidazole-4-carbothioic acid *S*-(2-dimethylaminoethyl) ester, m.p. 275-277°C.

### Example 34

### 3-(3,4-Dihydroxybenzyl)-1-(1,2,3,4-tetrahydronaphthalen-2-yl)-1,3-dihydroimidazole-2-thione

The following is the preparation of a compound of Formula I(a) in which t is 0, R³ is 3,4-dihydroxybenzyl and R⁴ and R⁵ are each hydro.

A solution of 3-(3,4-dimethoxybenzyl)-1-(1,2,3,4-tetrahydronaphthalen-2-yl)-1,3-dihydroimidazole-2-thione (900 mg, 2.37 mol), prepared as in Example 10, in methylene chloride was cooled to 0°C under nitrogen and then boron tribromide (1M, 7.1 mL, 7.1 mmol) in an additional 10 mL of methylene chloride was added dropwise. The mixture was allowed to cool to room temperature, stirred for 16 hours and then slowly added to water. The organic layer was separated, washed with brine, dried (Na₂SO₄) and concentrated. The residue was purified by flash chromatography on silica gel eluting with methylene chloride/methanol (96:4) and then crystallized from methanol/ethanol/hexane to give 3-(3,4-dihydroxybenzyl)-1-(1,2,3,4-tetrahydro-naphthalen-2-yl)-1,3-dihydroimidazole-2-thione (520 mg), m.p. 173-174°C.

Proceeding as in Example 34, but substituting different starting materials for 3-(3,4-dimethoxybenzyl)-1-(1,2,3,4-tetrahydronaphthalen-2-yl)-1,3-dihydroimidazole-2-thione gave the following compounds of Formula I:
substituting 3-[2-(3,4-dimethoxyphenyl)ethyl]-1-(1,2,3,4-tetrahydronaphthalen-2-yl)-1,3-dihydroimidazole-2-thione gave 3-[2-(3,4-dihydroxyphenyl)ethyl]-1-(1,2,3,4-tetrahydronaphthalen-2-yl)-1,3-dihydroimidazole-2-thione, m.p. 165-167°C;
substituting 1-(1,2,3,4-tetrahydro-5-methoxynaphthalen-2-yl)-1,3-dihydroimidazole-2-thione gave 1-(1,2,3,4-tetrahydro-5-hydroxynaphthalen-2-yl)-1,3-dihydro-imidazole-2-thione, m.p. 263-265°C (Reference) ;
substituting 1-(1,2,3,4-tetrahydro-6-methoxynaphthalen-2-yl)-1,3-dihydroimidazole-2-thione gave 1-(1,2,3,4-tetrahydro-6-hydroxynaphthalen-2-yl)-1,3-dihydro-imidazole-2-thione, m.p. 240-241°C (Reference) ;
substituting 1-(1,2,3,4-tetrahydro-7-methoxynaphthalen-2-yl)-1,3-dihydroimidazole-2-thione gave 1-(1,2,3,4-tetrahydro-7-hydroxynaphthalen-2-yl)-1,3-dihydro-imidazole-2-thione, m.p. 248-250°C (Reference) ;
substituting 1-(1,2,3,4-tetrahydro-8-methoxynaphthalen-2-yl)-1,3-dihydroimidazole-2-thione gave 1-(1,2,3,4-tetrahydro-8-hydroxynaphthalen-2-yl)-1,3-dihydroimidazole-2-thione, m.p. 274-276°C (Reference) ; and
substituting 1-(6,8-difluoro-1,2,3,4-tetrahydro-7-methoxynaphthalen-2-yl)-1,3-dihydroimidazole-2-thione gave 1-(6,8-difluoro-1,2,3,4-tetrahydro-7-hydroxynaphthalen-2-yl)-1,3-dihydroimidazole-2-thione, m.p. 258-260°C (Reference).

### Example 35

### (S)-N-[3-(5,7-Difluoro-1,2,3,4-tetrahydronaphthalen-2-yl)-2-thioxo-2,3-dihydro-1H-imidazol-4-ylmethyl]-4-butylbenzamide

The following is the preparation of a compound of Formula I(a) in which t is 2, R¹ is fluoro at the 5- and 7-position and R⁵ is 4 -butylbenzoylaminomethyl.

A mixture of (*S*)-5-aminomethyl-1-(5,7-difluoro-1,2,3,4-tetrahydronaphthalen-2-yl)-1,3-dihydroimidazole-2-thione (0.30 g, 1 mmol), prepared as in Example 22, and 4-butylbenzoyl chloride (0.21 mL, 1.1 mmol) in 10 mL of dry pyridine was stirred under argon at approximately 0°C for 1 hour and then at approximately 25°C for an additional 2 hours. The mixture was concentrated and the residue was treated with water. The mixture was extracted with ethyl acetate (3x) and the combined extracts were dried (MgSO₄) and concentrated. The residue was recrystallized from ethyl acetate to give (*S*)-*N*-[3-(5,7-difluoro-1,2,3,4-tetrahydronaphthalen-2-yl)-2-thioxo-2,3-dihydro-1*H*-imidazol-4-ylmethyl]-4-butylbenzamide (0.25 g, 0.55 mmol), m.p. 242-243°C.

Proceeding as in Example 35 but substituting different starting materials for (*S*)-5-aminomethyl-1-(5,7-difluoro-1,2,3,4-tetrahydronaphthalen-2-yl)-1,3-dihydroimidazole-2-thione and/or 4-butylbenzoyl chloride gave the following compounds of Formula I:
substituting nicotinoyl chloride hydrochloride gave (*S*)-*N*-[3-(5,7-difluoro-1,2,3,4-tetrahydronaphthalen-2-yl)-2-thioxo-2,3-dihydro-1*H*-imidazol-4-ylmethyl]-nicotinamide, m.p. 218-221°C;
substituting benzoyl chloride gave (*S*)-*N*-[3-(5,7-difluoro-1,2,3,4-tetrahydro-naphthalen-2-yl)-2-thioxo-2,3-dihydro-1*H*-imidazol-4-ylmethyl]benzamide, m.p. 260-261°C;
substituting dimethylcarbamyl chloride gave (*S*)-*N*-[3-(5,7-difluoro-1,2,3,4-tetrahydronaphthalen-2-yl)-2-thioxo-2,3-dihydro-1*H*-imidazol-4-yl-methyl]dimethylcarbamide, m.p. 218-220°C;
substituting methyl chloroformate gave methyl (*S*)-3-(5,7-difluoro-1,2,3,4-tetrahydronaphthalen-2-yl)-2-thioxo-2,3-dihydro-1*H*-imidazol-4-ylmethyl-carbamate, m.p. 220-222°C;
substituting (*S*)-3-amino-1-(1,2,3,4-tetrahydronaphthalen-2-yl)-1,3-dihydroimidazole-2-thione and acetic anhydride gave *N*-[3-(1,2,3,4-tetrahydronaphthalen-2-yl)-2-thioxo-2,3-dihydro-1*H*-imidazol-4-yl]acetamide, m.p. 196-200°C;
substituting 2-furancarboxylic acid chloride gave (*S*)-*N*-[3-(5,7-difluoro-1,2,3,4-tetrahydronaphthalen-2-yl)-2-thioxo-2,3-dihydro-1*H*-imidazol-4-ylmethyl]-2-furancarboxamide, m.p. 227-231°C;

### Example 36

### (S)-N-[3-(5,7-Difluoro-1,2,3,4-tetrahydronaphthalen-2-yl)-2-thioxo-2,3-dihydro-1H-imidazol-4-ylmethyl]picolinamide

The following is the preparation of a compound of Formula I (a) in which t is 2, R¹ is fluoro at the 5- and 7-position and R⁵ is picolinoylaminomethyl.

A mixture of (*S*)-5-aminomethyl-1-(5,7-difluoro-1,2,3,4-tetrahydronaphthalen-2-yl)-1,3-dihydroimidazole-2-thione (295 mg, 1 mmol), prepared as in Example 22, picolinic acid (123 mg, 1 mmol) and PyBOP (620 mg, 1.2 mmol) in 10 mL of dry DMF was stirred under argon at approximately 25°C for 5 minutes and then *N,N*-diisopropylethylamine (0.58 mL, 3.3 mmol) was added. The mixture was stirred for approximately 12 hours and then 10 mL of water was added. The aqueous layer was extracted with ethyl acetate (3x 10 mL) and the combined extracts were washed with water, dried (MgSO₄) and concentrated. The residue was purified by flash chromatography on silica gel eluting with hexane/THF (1:1 to 8:2) to give (*S*)-*N*-[3-(5,7-difluoro-1,2,3,4-tetrahydronaphthalen-2-yl)-2-thioxo-2,3-dihydro-1*H*-imidazol-4-ylmethyl]picolinamide (80 mg, 0.2 mmol), m.p. 216-217°C.

Proceeding as in Example 36, but substituting a different starting material for picolinic acid gave the following compounds of Formula I:
substituting *N*-(*tert*-butoxycarbonyl)glycine and deprotecting gave (*S*)-*N*-[3-(5,7-difluoro-1,2,3,4-tetrahydronaphthalen-2-yl)-2-thioxo-2,3-dihydro-1*H*-imidazol-4-ylmethyl]aminoacetamide, m.p. 144-153°C;
substituting *N*-(*tert*-butoxycarbonyl) -2-methylalanine and deprotecting gave (*S*)-*N*-[3-(5,7-difluoro-1,2,3,4-tetrahydronaphthalen-2-yl)-2-thioxo-2,3-dihydro-1*H*-imidazol-4-ylmethyl]-2-amino-2-methylpropionamide trifluoroacetate, m.p. 158°C; and
substituting 5-butylpicolinic acid gave (S)-*N*-[3-(5,7-difluoro-1,2,3,4-tetrahydronaphthalen-2-yl)-2-thioxo-2,3-dihydro-1*H*-imidazol-4-ylmethyl]-5-butylpicolinamide, m.p. 99-104°C.

### Example 37

### (S)-N-[3-(5,7-Difluoro-1,2,3,4-tetrahydronaphthalen-2-yl)-2-thioxo-2,3-dihydro-1H-imidazol-4-ylmethyl]ethylcarbamide

The following is the preparation of a compound of Formula I(a) in which t is 2, R¹ is fluoro at the 5- and 7-position and R⁵ is ethyluriedomethyl.

A mixture of (S)-5-aminomethyl-1-(5,7-difluoro-1,2,3,4-tetrahydronaphthalen-2-yl)-1,3-dihydroimidazole-2-thione (294 mg, 1 mmol), prepared as in Example 22, and ethyl isocyanate (0.16 mL, 2 mmol) in 10 mL of THF was stirred at approximately 50°C under argon for approximately 60 hours. The mixture was filtered and the filtered solid was recrystallized from ethyl acetate/methanol to give (*S*)-*N*-[3-(5,7-difluoro-1,2,3,4-tetrahydronaphthalen-2-yl)-2-thioxo-2,3-dihydro-1*H*-imidazol-4-ylmethyl]ethylcarbamide (110 mg, 0.3 mmol), m.p. 219-220°C.

### Example 38

### 4-Aminomethyl-1-(1,2,3,4-tetrahydronaphthalen-2-yl)-1,3-dihydroimidazole-2-thione

The following is the preparation of a compound of Formula I(a) in which t is 0, R³ and R⁵ are each hydro and R⁴ is aminomethyl.

A mixture of 3-(1,2,3,4-tetrahydronaphthalen-2-yl)-2-thioxo-2,3-dihydro-1*H*-imidazole-4-carbaldehyde (0.25 g, 1.0 mmol), hydroxylamine hydrochloride (0.09 g, 1.3 mmol) and sodium hydroxide (0.064 g, 1.6 mmol) in 2 mL of ethanol and 2 mL of water was stirred at 60°C for 1 hour. The mixture was cooled giving a crystalline material. The material was isolated by filtration and dried. The filtrate was stirred with ethyl acetate giving more crystalline material. The material was isolated by filtration and dried. Combining the crystalline material gave 1-(1,2,3,4-tetrahydronaphthalen-2-yl)-2-thioxo-2,3-dihydro-1*H*-imidazole-5-carbaldehyde oxime (0.186 g, 0.68 mmol).

A suspension of 3-(1,2,3,4-tetrahydronaphthalen-2-yl)-2-thioxo-2,3-dihydro-1*H*-imidazole-5-carbaldehyde oxime (0.158 g, 0.58 mmol), prepared as in Example 16, in 20 mL of THF was cooled to 0°C and LAH (1.0 M, 1.16 mL, 1.16 mmol) in THF was added slowly. The mixture was stirred at 0°C for 1 hour and then saturated ammonium chloride, water and ethyl acetate were added. The aqueous layer was separated and extracted with ethyl acetate. The combined ethyl acetate was dried over magnesium sulfate and concentrated by evaporation. Purification of the residue by flash chromatography (elution: 3-10% methanol/methylene chloride) gave 4-aminomethyl-1-(1,2,3,4-tetrahydro-naphthalen-2-yl)-1,3-dihydroimidazole-2-thione, m.p. 197-200°C.

### Example 39

### (S)-1-(5,7-difluoro-1,2,3,4-tetrahydronaphthalen-2-yl)-4-(2-phenylethyl)aminomethyl-1,3-dihydroimidazole-2-thione

The following is the preparation of a compound of Formula I(a) in which t is 2, R¹ is fluoro at the 5- and 7-position and R⁴ is 2- (phenyl)ethylaminomethyl.

A mixture of (*S*)-3-(5,7-difluoro-1,2,3,4-tetrahydronaphthalen-2-yl)-2-thioxo-2,3-dihydro-1*H*-imidazole-5-carbaldehyde (147 mg, 0.5 mmol), prepared as in Example 16, phenethyl amine (75µL, 0.6 mmol) and sodium cyanoborohydride (47 mg, 0.75 mmol) in 10 mL of methanol was stirred at approximately 60°C for 2 hours. The mixture was concentrated and the residue was purified by flash chromatography on silica gel eluting with methylene chloride/methanol (97:3). The purified product was concentrated, converted to the hydrochloride salt and recrystallized from ethyl acetate/methanol to give (*S*)-1-(5,7-difluoro-1,2,3,4-tetrahydronaphthalen-2-yl)-4-(2-phenylethyl)aminomethyl-1,3-dihydroimidazole-2-thione hydrochloride (68 mg, 0.2 mmol), m.p. 227-229°C.

Proceeding as in Example 39 but substituting a different starting material for (*S*)-3-(5,7-difluoro-1,2,3,4-tetrahydronaphthalen-2-yl)-2-thioxo-2,3-dihydro-1*H*-imidazole-5-carbaldehyde and/or phenethylamine gave the following compounds of Formula I:
substituting 3-(5,7-difluoro-1,2,3,4-tetrahydronaphthalen-2-yl)-2-thioxo-2,3-dihydro-1*H*-imidazole-5-carbaldehyde and glycine *tert*-butyl ester hydrochloride gave *tert*-butyl 3-(5,7-difluoro-1,2,3,4-tetrahydronaphthalen-2-yl)-2-thioxo-2,3-dihydro-1*H*-imidazol-5-ylmethylaminoacetate;
substituting (*S*)-3-(5,7-difluoro-1,2,3,4-tetrahydronaphthalen-2-yl)-2-thioxo-2,3-dihydro-1*H*-imidazole-5-carbaldehyde and glycine *tert*-butyl ester hydrochloride gave *tert*-butyl (*S*)-3-(5,7-difluoro-1,2,3,4-tetrahydronaphthalen-2-yl)-2-thioxo-2,3-dihydro-1*H*-imidazol-5-ylmethylaminoacetate;
substituting 3-(1,2,3,4-tetrahydronaphthalen-2-yl)-2-thioxo-2,3-dihydro-1*H*-imidazole-5-carbaldehyde and glycine *tert*-butyl ester hydrochloride gave *tert*-butyl 3-(1,2,3,4-tetrahydronaphthalen-2-yl)-2-thioxo-2,3-dihydro-1*H*-imidazol-5-ylmethylaminoacetate;
substituting glycinamide hydrochloride gave (*S*)-3-(5,7-difluoro-1,2,3,4-tetrahydronaphthalen-2-yl)-2-thioxo-2,3-dihydro-1*H*-imidazol-5-yl-methylaminoacetamide, m.p. 212-213°C; and
substituting methyl 4-(2-aminoethyl)benzoate hydrochloride gave methyl (*S*)-4-{2-[3-(5,7-difluoro-1,2,3,4-tetrahydronaphthalen-2-yl)-2-thioxo-2,3-dihydro-1*H*-imidazol-5-yl-methylamino]ethyl}benzoate, m.p. 159-160°C.

### Example 40

### (S)-N³-[3-(5,7-Difluoro-1,2,3,4-tetrahydronaphthalen-2-yl)-2-thioxo-2,3-dihydro-1H-imidazol-4-ylmethyl]-N¹,N ²-di(tert-butoxycarbonyl)formamidine

The following is the preparation of a compound of Formula I(a) in which t is 2, R¹ is fluoro at the 5- and 7-position and R⁵ is *N*¹,*N*²-di(*tert*-butoxycarbonyl)amidinoaminomethyl.

A mixture of (*S*)-1-(5,7-difluoro-1,2,3,4-tetrahydronaphthalen-2-yl)-5-aminomethyl-1,3-dihydroimidazole-2-thione (0.6 g, 2 mmol), prepared as in Example 22, and *N*¹,*N*²-di(*tert*-butoxycarbonyl)methylthioamidine (0.65 g, 2.2 mmol) in 15 mL of THF and 0.3 mL of water was stirred at approximately 50°C under argon for 4 hours. The mixture was concentrated and the residue was combined with 5% aqueous sodium bicarbonate. The mixture was extracted with ethyl acetate and the extract was dried (MgSO₄) and concentrated. The residue was purified by flash chromatography on silica gel eluting with methylene chloride/methanol (99:1) to give (*S*)-*N*³-[3-(5,7-difluoro-1,2,3,4-tetrahydro-naphthalen-2-yl)-2-thioxo-2,3-dihydro-1*H*-imidazol-4-ylmethyl]-*N*¹*,N*²-di(*tert*-butoxycarbonyl)formamidine (.54 g, 1 mmol), m.p. 155°C (eff.).

Proceeding as in Example 40, but subsituting a different starting material for *N*¹,*N*²-di(*tert*-butoxycarbonyl)methylthioamidine gave the following compounds of Formula I:
substituting *N*¹,*N*²-di(acetyl)methylthioamidine gave (*S*)-*N*³-[3-(5,7-difluoro-1,2,3,4-tetrahydronaphthalen-2-yl)-2-thioxo-2,3-dihydro-1*H*-imidazol-4-yl-methyl]-*N*¹*,N*²-di(acetyl)formamidine, m.p. 213-214°C; and
substituting *N*¹-(*tert*-butoxycarbonyl)methylthioamidine gave (*S*)-*N*³-[3-(5,7-difluoro-1,2,3,4-tetrahydronaphthalen-2-yl)-2-thioxo-2,3-dihydro-1*H*-imidazol-4-yl-methyl]-*N*¹-(*tert*-butoxycarbonyl)formamidine, m.p. >280°C.

### Example 41

### (S)-N³-[3-(5,7-Difluoro-1,2,3,4-tetrahydronaphthalen-2-yl)-2-thioxo-2,3-dihydro-1H-imidazol-4-ylmethyl]formamidine

The following is the preparation of a compound of Formula I(a) in which t is 2, R¹ is fluoro at the 5- and 7-position and R⁵ is amidinoaminomethyl.

A solution of (*S*)-*N*³-[3-(5,7-difluoro-1,2,3,4-tetrahydronaphthalen-2-yl)-2-thioxo-2,3-dihydro-1*H*-imidazol-4-ylmethyl]-*N*¹,*N*²-di(*tert*-butoxycarbonyl)-formamidine (.34 g, 0.6 mmol), prepared as in Example 41, in 20 mL of trifluoroacetic acid was stirred at approximately 25°C for 1.5 hours. The solution was concentrated and the residue was combined with 100 mL of diethyl ether. The diethyl ether was decanted away and the residue was combined with 100 mL of diethyl ether. The mixture was filtered and the filtered residue was dissolved in ethyl acetate. The solution was concentrated, evacuated and the resulting foam was treated with diethyl ether. The mixture was filtered to give (*S*)-*N*³-[3-(5,7-difluoro-1,2,3,4-tetrahydronaphthalen-2-yl)-2-thioxo-2,3-dihydro-1*H*-imidazol-4-ylmethyl]formamidine trifluoroacetate (0.28 g, 0.6 mmol), m.p. 103° (eff).

### Example 42

### 2S-Amino-3-(3H-imidazol-4-yl)-N-[3-(5,7-difluoro-1,2,3,4-tetrahydronaphthalen-2R-yl) -2-thioxo-2,3-dihydro-1H-imidazol-4-ylmethyl]propionamide hydrochloride

The following is the preparation of a compound of Formula II in which t is 2, R¹ is fluoro at the 5- and 7-position and R¹⁸ is a group of Formula (d) wherein R²¹ is L-histidylaminomethyl.

A mixture of (*R*)-5-aminomethyl-1-(5,7-difluoro-1,2,3,4-tetrahydronaphthalen-2-yl)-1,3-dihydroimidazole-2-thione (0.55 g, 1.86 mmol), prepared as in Example 22, (*S*)-2-(*tert*-butoxycarbonyl)amino-3-(3-*tert*-butoxycarbonyl-3*H*-imidazol-4-yl)propionic acid (0.76 g, 1.86 mmol) and PyBOP (1.07 g, 2.05 mmol) in 6.2 mL of DMF was stirred under argon until homogeneous. Diethylisopropylethylamine (1.07 mL, 6.15 mmol) was added and the mixture was stirred for approximately 18 hours. The mixture partitioned between water and ethyl acetate and the organic layer was washed twice with water, dried over magnesium sulfate and concentrated by evaporation. Purification of the residue by column chromatography (elution: 5% methanol/methylene chloride) gave 2*S*-(*tert*-butoxycarbonyl)amino-3-(3-*tert*-butoxycarbonyl-3*H*-imidazol-4-yl)-*N*-[3-(5,7-difluoro-1,2,3,4-tetrahydronaphthalen-2*R*-yl)-2-thioxo-2,3-dihydro-1*H*-imidazol-4-yl-methyl]propionamide (1.003 g) as a foam.

2*S*-(*tert*-Butoxycarbonyl)amino-3-(3-tert-butoxycarbonyl-3*H*-imidazol-4-yl)-*N*-[3-(5,7-difluoro-1,2,3,4-tetrahydronaphthalen-2*R*-yl)-2-thioxo-2,3-dihydro-1*H*-imidazol-4-ylmethyl]propionamide (0.935 g) was dissolved in 45 mL of 30% anhydrous hydrogen chloride/ethyl acetate and the mixture was stirred for 18 hours giving a crystalline material. Isolation of the material by filtration and drying at 60°C under vacuum gave 2*S*-amino-3-(3*H*-imidazol-4-yl)-*N*-[3-(5,7-difluoro-1,2,3,4-tetrahydronaphthalen-2*R*-yl)-2-thioxo-2,3-dihydro-1*H*-imidazol-4-ylmethyl]propionamide hydrochloride (0.655 g, 1.24 mmol), m.p. 228°C.

Proceeding as in Example 42 but substituting a starting material for (*R*)-5-aminomethyl-1-(5,7-difluoro-1,2,3,4-tetrahydronaphthalen-2-yl)-1,3-dihydroimidazole-2-thione and/or (*S*)-2-(*tert*-butoxycarbonyl)amino-3-(3-*tert*-butoxycarbonyl-3*H*-imidazol-4-yl)propionic acid gave the following compounds of Formula II:
substituting 5-aminomethyl-1-(5,7-difluoro-1,2,3,4-tetrahydronaphthalen-2-yl)-1,3-dihydroimidazole-2-thione and (*S*)-3-*tert*-butoxycarbonyl-2-*tert*-butoxycarbonylaminopropionic acid gave 3*S*-amino-*N*-[3-(5,7-difluoro-1,2,3,4-tetrahydronaphthalen-2-yl)-2-thioxo-2,3-dihydro-1*H*-imidazol-4-yl-methyl)succinamic acid hydrochloride, m.p. 220°C;
substituting 5-aminomethyl-1-(5,7-difluoro-1,2,3,4-tetrahydronaphthalen-2-yl)-1,3-dihydroimidazole-2-thione and (*S*)-3-(*tert*-butoxycarbonyl)-3-(*tert*-butoxycarbonylamino)propionic acid gave 2*S*-amino-*N*-[3-(5,7-difluoro-1,2,3,4-tetrahydronaphthalen-2-yl)-2-thioxo-2,3-dihydro-1*H*-imidazol-4-yl-methyl]succinamic acid hydrochloride, m.p. 220°C;
substituting 5-aminomethyl-1-(5,7-difluoro-1,2,3,4-tetrahydronaphthalen-2-yl)-1,3-dihydroimidazole-2-thione and (*S*)-2,5-di(*tert*-butoxycarbonylamino)valeric acid gave 2*S*,5-diamino-*N*-[3-(5,7-difluoro-1,2,3,4-tetrahydronaphthalen-2-yl)-2-thioxo-2,3-dihydro-1*H*-imidazol-4-ylmethyl]valeramide hydrochloride, m.p. 212-216°C;
substituting 5-aminomethyl-1-(1,2,3,4-tetrahydronaphthalen-2-yl)-1,3-dihydroimidazole-2-thione and (*S*)-2,5-di(*tert*-butoxycarbonylamino)valeric acid gave 2*S*,5-diamino-*N*-[3-(1,2,3,4-tetrahydronaphthalen-2-yl)-2-thioxo-2,3-dihydro-1*H*-imidazol-4-ylmethyl]valeramide hydrochloride, m.p. 191-205°C;
substituting 5-aminomethyl-1-(1,2,3,4-tetrahydronaphthalen-2-yl)-1,3-dihydroimidazole-2-thione and (*S*)-2-(*tert*-butoxycarbonylamino)-5-(*tert*-butoxycarbonyl)guanidinovaleric acid gave 2*S*-amino-*N*-[3-(1,2,3,4-tetrahydronaphthalen-2-yl)-2-thioxo-2,3-dihydro-1*H*-imidazol-4-ylmethyl]-5-guanidinovaleramide hydrochloride, m.p. 160°C;
substituting 3-amino-1-(1,2,3,4-tetrahydronaphthalen-2-yl)-1,3-dihydroimidazole-2-thione and (*S*)-2-(*tert*-butoxycarbonyl)amino-3-(3-*tert*-butoxycarbonyl-3*H*-imidazol-4-yl)propionic acid gave 2*S*-amino-3-(3*H*-imidazol-4-yl)-*N*-[3-(1,2,3,4-tetrahydronaphthalen-2-yl)-2-thioxo-2,3-dihydro-1*H*-imidazol-1-yl]propionamide hydrochloride, m.p. 197-205°C;
substituting 5-aminomethyl-1-(5,7-difluoro-1,2,3,4-tetrahydronaphthalen-2-yl)-1,3-dihydroimidazole-2-thione and (*S*)-2-(*tert*-butoxycarbonyl)amino-3-(3-*tert*-butoxycarbonyl-3*H*-imidazol-4-yl)propionic acid gave 25-amino-3-(3*H*-imidazol-4-yl)-*N*-[3-(5,7-difluoro-1,2,3,4-tetrahydronaphthalen-2-yl)-2-thioxo-2,3-dihydro-1*H*-imidazol-4-ylmethyl)propionamide hydrochloride, m.p. 195-238°C;
substituting (*S*)-5-aminomethyl-1-(5,7-difluoro-1,2,3,4-tetrahydronaphthalen-2-yl)-1,3-dihydroimidazole-2-thione and (*S*)-2-*tert*-butoxycarbonyl-amino-3-(3-*tert*-butoxycarbonyl-3*H*-imidazol-4-yl)propionic acid gave 2*S*-amino-3-(3*H*-imidazol-4-yl)*-N-*[3-(5,7-difluoro-1,2,3,4-tetrahydronaphthalen-2*S*-yl) -2-thioxo-2,3-dihydro-1*H*-imidazol-4-ylmethyl]propionamide hydrochloride, m.p. 225°C;
substituting (*S*)-5-aminomethyl-1-(5,7-difluoro-1,2,3,4-tetrahydronaphthalen-2-yl)-1,3-dihydroimidazole-2-thione and (*S*)-4-acetylamino-4-*tert*-butoxycarbonylbutyric acid gave 2*S*-acetylamino-4-[3-(5,7-difluoro-1,2,3,4-tetrahydronaphthalen-2*S*-yl)-2-thioxo-2,3-dihydro-1*H*-imidazol-4-ylmethylaminocarbonyl]butyric acid, m.p. 159°C;
substituting (*S*)-5-aminomethyl-1-(5,7-difluoro-1,2,3,4-tetrahydronaphthalen-2-yl)-1,3-dihydroimidazole-2-thione and (*S*)-2,5-di(*tert*-butoxycarbonylamino)pentanoic acid gave 2*S*,5-diamino-*N*-[3-(5,7-difluoro-1,2,3,4-tetrahydronaphthalen-2*S*-yl)-2-thioxo-2,3-dihydro-1*H*-imidazol-4-yl-methyl]valeramide hydrochloride, m.p. 233-237°C;
substituting (*R*)-5-aminomethyl-1-(5,7-difluoro-1,2,3,4-tetrahydronaphthalen-2-yl)-1,3-dihydroimidazole-2-thione and (*S*)-2,5-di(*tert*-butoxycarbonylamino)pentanoic acid gave 2*S*,5-diamino-*N*-[3-(5,7-difluoro-1,2,3,4-tetrahydronaphthalen-2*R*-yl)-2-thioxo-2,3-dihydro-1*H*-imidazol-4-yl-methyl]valeramide hydrochloride, m.p. 128-150°C;
substituting (*S*)-5-aminomethyl-1-(5,7-difluoro-1,2,3,4-tetrahydronaphthalen-2-yl)-1,3-dihydroimidazole-2-thione and (*S*)-3-(*tert*-butoxycarbonyl)-2-(*tert*-butoxycarbonylamino)propionic acid gave 3*S*-amino-*N*-[3-(5,7-difluoro-1,2,3,4-tetrahydronaphthalen-2*S*-yl)-2-thioxo-2,3-dihydro-1*H*-imidazol-4-yl-methyl]succinamic acid hydrochloride, m.p. 194°C;
substituting (*R*)-5-aminomethyl-1-(5,7-difluoro-1,2,3,4-tetrahydronaphthalen-2-yl)-1,3-dihydroimidazole-2-thione and (*S*)-3-(*tert*-butoxycarbonyl)-2-(*tert*-butoxycarbonylamino)propionic acid gave 3*S*-amino-*N*-[3-(5,7-difluoro-1,2,3,4-tetrahydronaphthalen-2*R*-yl)-2-thioxo-2,3-dihydro-1*H*-imidazol-4-yl-methyl]succinamic acid hydrochloride, m.p. 193°C;
substituting (*S*)-5-methylaminomethyl-1-(5,7-difluoro-1,2,3,4-tetrahydronaphthalen-2-yl)-1,3-dihydroimidazole-2-thione and (*S*)-3-(*tert*-butoxycarbonyl)-2-(*tert*-butoxycarbonylamino)propionic acid gave 3*S*-amino-*N*-[3-(5,7-difluoro-1,2,3,4-tetrahydronaphthalen-2*S*-yl)-2-thioxo-2,3-dihydro-1*H*-imidazol-4-yl-methyl]-*N*-methylsuccinamic acid hydrochloride.

### Reference Example 4

### 2R-Amino-3-[1-(4,6-difluoroindan-1-yl)-1-H-imidazol-2-yldisulfanyl]propionic Acid Hydrochloride (reference)

A solution of (*R*,*R*')-3,3'-disulfanylbis[*tert*-butyl 2-(*tert*-butoxycarbonyl)aminopropionate (1.06 g, 2.0 mmol) in 5 mL of ethylenedichloride was cooled to -23°C under argon. Bromine (51.3 µL, 1.0 mmol) was added dropwise and the mixture was stirred for approximately 20 minutes and then diluted with an additional 2 mL of ethylenedichloride. A suspension of potassium phthalimide (370 mg, 2.0 mmol) in 5 mL of ethylenedichloride was cooled to approximately -23°C and the disulfide mixture was added. The mixture was stirred for 1 hour at -23°C and then warmed to room temperature over 45 minutes. The mixture was filtered and concentrated in vacuo. The residue was dissolved in benzene. Purification by chromatography gave (*R*)-*N*-[2-(*tert*-butoxycarbonylamino)-2-(*tert*-butoxycarbonyl)ethylsulfanyl]phthalimide (684 mg) as an oil.

A mixture of (R)-*N*-[2-(*tert*-butoxycarbonylamino)-2-(*tert*-butoxycarbonyl)-ethylsulfanyl]phthalimide (660 mg, 1.61 mmol) and 1-(4,6-difluoroindan-1-yl)-1,3-dihydroimidazole-2-thione (400 mg, 1.59 mmol), prepared as in Example 9, in 8 mL of ethyl acetate was heated at reflux under argon for 1 hour. The mixture was allowed to cool to room temperature giving a crystalline precipitate and then the solvents were removed by evaporation under reduced pressure. The remaining semisolid was triturated with benzene and the benzene mixture was filtered. The benzene solution was concentrated by evaporation. Purification of the residue by chromatography gave tert-butyl 2*R*-(*tert*-butoxycarbonyl)amino-3-[1-(4,6-difluoroindan-1-yl)-1*H*-imidazol-2-yldisulfanyl]propionate (672 mg).

A mixture of *tert*-butyl 2*R*-(*tert*-butoxycarbonyl)amino-3-[1-(4,6-difluoroindan-1-yl)-1*H*-imidazol-2-yldisulfanyl]propionate (672 mg) in 15 mL of trifluoroacetic acid and 15 mL of methylenechloride was stirred at room temperature under a nitrogen atmosphere for 2 hours. The solvent was removed by evaporation and the residue was co-evaporated with ethyl acetate (2 x 50 mL).

Treatment with ethereal hydrogen chloride gave 2*R*-amino-3-[5-aminomethyl-1-(4,6-difluoroindan-1-yl)-1*H*-imidazol-2-yldisulfanyl]propionic acid hydrochloride (768 mg, 1.74 mmol) as a solid, m.p. 147-154°C. (Reference).

Proceeding as in Reference Example 4, but substituting a different starting material for (*R*,*R*')-3,3'-disulfanylbis[*tert*-butyl 2- (*tert*-butoxycarbonyl)aminopropionate and/or 1-(4,6-difluoroindan-1-yl)-1,3-dihydroimidazole-2-thione gave the following compounds of Formula III:
substituting 1-(5,7-difluoro-1,2,3,4-tetrahydronaphthalen-2-yl)-1,3-dihydroimidazole-2-thione gave 2*R*-amino-3-[1-(5,7-difluoro-1,2,3,4-tetrahydronaphthalen-2-yl)-1*H*-imidazol-2-yldisulfanyl]propionic acid hydrochloride, m.p. 130°C;
substituting 1-(6,8-difluoro-1,2,3,4-tetrahydronaphthalen-2-yl)-1,3-dihydroimidazole-2-thione gave 2*R*-amino-3-[1-(6,8-difluoro-1,2,3,4-tetrahydronaphthalen-2-yl)-1*H*-imidazol-2-yldisulfanyl]propionic acid hydrochloride, m.p. 141°C;
substituting 1-(6,7-difluoro-1,2,3,4-tetrahydronaphthalen-2-yl)-1,3-dihydroimidazole-2-thione gave 2*R*-amino-3-[1-(6,7-difluoro-1,2,3,4-tetrahydronaphthalen-2-yl)-1*H*-imidazol-2-yldisulfanyl]propionic acid hydrochloride, m.p. 130°C;
substituting 1-(5,7-difluoro-1,2,3,4-tetrahydronaphthalen-2-yl)-2-thioxo-1,3-dihydro-imidazole-5-carboxylic acid gave 2*R*-amino-3-[5-carboxy-1-(5,7-difluoro-1,2,3,4-tetrahydronaphthalen-2-yl)imidazol-2-yl-disulfanyl]propionic acid hydrochloride, m.p. 129-138°C;
substituting 1-(5,7-difluoro-1,2,3,4-tetrahydronaphthalen-2-yl)-5(1*H*)-tetrazol-5-yl-1,3-dihydroimidazole-2-thione and 3,3'-disulfanylbis[2-(*tert*-butoxycarbonyl)aminoethyl] gave 2-(2-aminoethyldisulfanyl)-1-(5,7-difluoro-1,2,3,4-tetrahydronaphthalen-2-yl)-5(1*H*)-tetrazol-5-yl-1,3-dihydroimidazole hydrochloride, m.p. 165°C dec; and
substituting (*S*)-5-(*tert*-butoxycarbonyl)aminomethyl-1-(5,7-difluoro-1,2,3,4-tetrahydronaphthalen-2-yl)-1,3-dihydroimidazole-2-thione and (R,R')-3,3'-disulfanylbis[*tert*-butyl 2-(*tert*-butoxycarbonyl)aminopropionate gave 2*R*-amino-3-[5-aminomethyl-1-(5,7-difluoro-1,2,3,4-tetrahydronaphthalen-2-yl)-1*H*-imidazol-2-yldisulfanyl]propionic acid hydrochloride, m.p. 179-180°C.

### Reference EXAMPLE 5

### N-(Trifluoroacetyl)-L-aspartic Anhydride

Trifluoroacetic anhydride (7.7 kg, 5.3 L, 37.5 mol) was heated to reflux temperature and a solution of L-aspartic acid (2.0 kg, 15.0 mol) in 9 L of trifluoroacetic acid (prepared by gradually heating to 65°C and stirring for 3 hours) was added to the refluxing trifluoroacetic anhydride over 30 minutes. The mixture then was distilled and 9 L of trifluoroacetic acid was removed. The remaining mixture was added to 8 L of cold, hexane under nitrogen. The hexane mixture was stirred for 3 hours in an ice bath giving a crystalline material. The material was isolated by filtration and the filter residue was washed with approximately 25 L of hexane. Drying to constant weight in a vacuum oven at 50°C under a nitrogen gas bleed gave *N*-(trifluoroacetyl)-L-aspartic anhydride (2.9 kg, 13.7 mol), m.p. 140-141°C. [α]_{D} -27.4° (c = 3.28, THF).

### Reference EXAMPLE 6

### (S)-2-[(Trifluoroacetyl)amino]-4-(2,4-difluorophenyl)-4-oxobutanoic acid

The following is the preparation of a compound of Formula 31 in which t is 2 and R¹ is fluoro at the 2- and 4-position.

A solution of 1,3-difluorobenzene (2.3 kg, 20.0 mol) in 5 L of methylene chloride was added to a mixture of *N*-(trifluoroacetyl)-L-aspartic anhydride (4.2 kg, 20.0 mol), prepared as in Reference Example 5, and aluminum chloride (7.4 kg, 55.5 mol) in 25 L of methylene chloride. The temperature of the reaction mixture was increased gradually over 1.5 hours and held at reflux for an additional 3 hours. The mixture then was cooled and 10 L of water and 20 L of 6N hydrochloric acid were added with good agitation. The methylene chloride layer was separated, washed with water and then brine, and the volatiles were removed by distilling at atmospheric pressure.

The residue was dissolved in 40 L of toluene and 8 L of volatiles was removed by distilling the mixture *in vacuo*. The solution was heated to 50°C and 8 L of hexane was added. The mixture was cooled to 30°C and 90 L of hexane was added. The mixture then was stirred at 25°C for 3 hours giving a crystalline material. The material was isolated by filtration and the filter residue was washed with hexane (3 x 10 L). Drying to a constant weight in a vacuum oven at room temperature under a nitrogen bleed gave (*S*)-2-[(trifluoroacetyl)amino]-4-(2,4-difluorophenyl)-4-oxobutanoic acid (5.2 kg, 16.0 mol), m.p. 82.4-84.0°C. [α]_{D} +15.2° (c = 0.956, CH₃OH).

### Reference EXAMPLE 7

### (S)-2-[(Trifluoroacetyl)amino]-4-(2,4-difluorophenyl)butanoic acid

The following is the preparation of a compound of Formula 30 in which t is 2 and R¹ is fluoro at the 2- and 4-position.

A mixture of (*S*)-2-[(trifluoroacetyl)amino]-4-(2,4-difluorophenyl)-4-oxobutanoic acid (4.8 kg, 14.7 mol), prepared as in Reference Example 6, and activated carbon, Darco®, (0.4 kg) in 5 L of acetic acid was stirred at room temperature for 1 hour. The mixture was filtered on to Pearlman's catalyst (0.5 kg, 50% wet) and washed in with 15 L of glacial acetic acid. Sulfuric acid (1.2 L, 21.8 mol) in 1 L of glacial acetic acid was filtered into the mixture and washed in with 2.8 L of glacial acetic acid. The reaction vessel was vacuum/pressure purged 3 times with nitrogen and then 6 times with hydrogen to 10 psig. The mixture was stirred vigorously under hydrogen at atmospheric pressure at room temperature, for 24 hours. The reaction vessel then was purged with nitrogen and the mixture was filtered onto 4.6 kg of sodium acetate trihydrate. The filter was washed with 10 L of glacial acetic acid. The glacial acetic acid was removed by distilling the mixture *in vacuo*.

The residue was partitioned between 20 L of methylene chloride and 40 L of water. The aqueous layer was extracted with 10 L of methylene chloride and the combined methylene chloride was washed with 10 L of water. The methylene chloride mixture then was dried over sodium sulfate (10 kg) and filtered. The solvent was removed *in vacuo* and the residue was dissolved in 5 L of methylene chloride. The solution was added under nitrogen to 15 L of hexane at a rate such that the temperature of the hexane mixture remained between 0 and 5°C. The mixture was allowed to stand for 1 hour giving a crystalline material. The material was isolated by filtration and the filter residue was washed with 10 L of hexane. Drying to constant weight *in vacuo* at 25°C with a nitrogen bleed gave (*S*)-2-[(trifluoroacetyl)amino]-4-(2,4-difluorophenyl)butanoic acid (3.3 kg, 10.4 mol), m.p. 62-83.5°C. An analytically pure sample had a melting point of 86-89°C. [α]_{D} +6.8° (c = 0.995, CH₃OH).

### Reference EXAMPLE 8

### (S)-5,7-Difluoro-2-[(trifluoroacetyl)amino]-3,4-dihydro-1(2H)-naphthalenone

The following is the preparation of a compound of Formula 29 in which, t is 2 and R¹ is fluoro at the 5- and 7-position.

A suspension of phosphorus pentachloride (2.2 kg, 10.6 mol) in 12 L of methylene chloride was cooled to 5°C and (*S*)-4-(2,4-difluorophenyl)-2-[(trifluoroacetyl)amino]butanoic acid (3.1 kg, 9.9 mol), prepared as in Reference Example 7, in 12 L of methylene chloride was added over 20 minutes. Thin layer chromatography of a methanol-quenched aliquot confirmed that the butanoic acid had converted to the corresponding acid chloride.

The mixture was stirred for 30 minutes and then was added to a slurry of aluminum chloride (4.3 kg) in 38.8 L of methylene chloride at a rate such that the temperature of the slurry remained between 1 and 5°C. The reaction mixture was stirred for 1 hour and then added to 28 kg of ice and 5.3 kg of concentrated hydrochloric acid. The mixture was stirred for 1 hour and the temperature allowed to rise to 20°C.

The aqueous layer was separated and extracted methylene chloride (2 x 15 L). The methylene chloride layer was washed once with water and combined with the methylene chloride extracts. The combined methylene chloride then was washed with water. The pH of the aqueous phase was adjusted to 6 by addition of aqueous sodium bicarbonate solution. The methylene chloride layer was washed with water and then brine. The methylene chloride was dried over sodium sulfate and filtered. The mixture was concentrated by evaporation at atmospheric pressure and the residue was dissolved in 15 L of methanol. The methanol solution was distilled to remove residual methylene chloride and then 9.9 L of water was added. The mixture was warmed to 56°C, allowed to cool to room temperature and then stirred for approximately 12 hours. A crystalline material was obtained and isolated by filtration. The filter residue was washed with 15 L of water. The isolated material was dried to constant weight *in vacuo* at room temperature with a nitrogen bleed.

The material was dissolved in 5 L of toluene at a temperature of 90°C and combined with 10 L of heptane at a temperature of 80°C. The temperature of the mixture gradually was decreased over 1.5 hours. The mixture then was stirred at 5°C for approximately 12 hours giving a crystalline material. The material was isolated by filtration and the filter residue was washed with 15 L of heptane. Drying to constant weight *in vacuo* at room temperature with a nitrogen bleed gave (*S*)-5,7-difluoro-2-[(trifluoroacetyl)amino]-3,4-dihydro-(2*H*)-naphthalen-1-one (2.0 kg, 6.8 mol), m.p. 142.4-144.6°C. [α]_{D} -59.4°
(c = 0.934, CH₃OH).

### Reference EXAMPLE 9

### (S)-5,7-Difluoro-2-[(trifluoroacetyl)amino]-1,2,3,4-tetrahydronaphthalene

The following is the preparation of a compound of Formula 28 in which t is 2 and R¹ is fluoro at the 5- and 7-position.

A reaction vessel containing a mixture of (*S*)-5,7-difluoro-2-[(trifluoroacetyl)amino]-3,4-dihydro-(2*H*)-naphthalen-1-one (1.1 kg, 3.8 mol), prepared as in Reference Example 8, and Pearlman's catalyst (0.55 kg, 50% wet) in 11 L of TFA was vacuum/pressure purged 8 times with nitrogen and then 8 times with hydrogen to 11 psig. The mixture was stirred vigorously under hydrogen (125 psig) at room temperature for 24 hours. Thin layer chromatography confirmed that the naphthalen-1-one had converted to (*S*)-1-hydroxy-5,7-difluoro-2-[(trifluoroacetyl)amino]-3,4-dihydro-(2*H*)-naphthalene.

Sulfuric acid (1.1 L, 19.4 mol) in 1 L of TFA then was added and the mixture stirred under hydrogen (125 psig) at room temperature for an additional 24 hours. The reaction vessel then was purged with nitrogen and the mixture was filtered over celite and washed through with 11 L of TFA. The filtrate was combined with 2.8 kg sodium acetate trihydrate and 80 L of water. The mixture was cooled to 10°C giving a crystalline material. The material was isolated by filtration and the filter residue was washed with 10 L of ice water. Drying gave (*S*)-5,7-difluoro-2-[(trifluoroacetyl)amino]-1,2,3,4-tetrahydronaphthalene (0.8 kg, 2.9 mol), m.p. 159.9 160.9°C. [α]_{D} -56.0° (c= 1.01, CH₃OH).

### EXAMPLE 43

### (S)-5,7-Difluoro-1,2,3,4-tetrahydronaphthalen-2-ylamine Hydrochloride

The following is the preparation of a compound of Formula 3 in which t is 2 and R¹ is fluoro at the 5- and 7-position.

Lithium hydroxide monohydrate (7.8 g, 0.2 mol) was added to a solution of (*S*)-5,7-difluoro-2-[(trifluoroacetyl)amino]-1,2,3,4-tetrahydronaphthalene (20.8 g, 74.5 mmol), prepared as in Reference Example 9 , in 187 mL of methanol and 21 mL of water. The mixture was stirred at reflux for 30 minutes and diluted with 200 mL of methanol. The diluted mixture then was combined with 60 mL of water, 24.8 mL of concentrated hydrochloric acid and 4.2 g of activated carbon, Darco®. The mixture was stirred for 30 minutes and then filtered through celite. The filtrate was distilled until the head temperature reached 75°C. The remaining mixture was allowed to cool and let stand for approximately 60 hours. The mixture then was cooled in an ice bath giving a crystalline material. The material was isolated by filtration and the filter residue was washed with water. Drying to constant weight *in vacuo* at room temperature under a nitrogen stream gave (*S*)-5,7-difluoro-1,2,3,4-tetrahydronaphthalen-2-ylamine hydrochloride (14.8 g, 67.6 mol), m.p. > 280°C. [α]_{D} -66.2° (c = 0.162, CH₃OH).

### EXAMPLE 44

### (S)-5-Hydroxymethyl-1-(5,7-difluoro-1,2,3,4-tetrahydronaphthalen-2-yl)-1,3-dihydroimidazole-2-thione

The following is the preparation of a compound of Formula 27 in which t is 2 and R¹ is fluoro at the 5- and 7-position.

Potassium thiocyanate (15.9 g, 162.6 mmol) was dried by heating to 175°C under nitrogen and then cooled to 35°C under vacuum with several nitrogen purges. A mixture of dihydroxyacetone (15.9 g, 176.7 mmol) and (*S*)-5,7-difluoro-1,2,3,4-tetrahydronaphthalen-2-ylamine hydrochloride (30.0 g, 137.0 mmol), prepared as in Example 43, in 540 mL of ethyl acetate was added to the dry potassium thiocyanate. The reaction vessel was purged with nitrogen and 40.83 g of glacial acetic acid was added. The reaction mixture was stirred at 35°C for 2 hours and 100 mL of 1.0 M sulfuric acid was added. The mixture was stirred for 15 minutes, then cooled in an ice bath and 2.5 M sodium hydroxide was added until the mixture was pH 7. The organic layer was washed with 50 mL of saturated aqueous sodium bicarbonate and then 50 mL of brine. The organic layer was concentrated to 480 mL by distillation and the mixture was cooled to 6°C and allowed to stand for 12 hours giving a crystalline material. The material was isolated by filtration, the filter residue was washed with cold ethyl acetate and the isolated material dried.

The material was dissolved in 650 mL of ethyl acetate and 25 mL of water. The mixture was distilled until 500 mL of volatiles were removed. The mixture was cooled to room temperature and stirred for 45 minutes giving a crystalline material. The material was isolated by filtration and the filter residue was washed with cold ethyl acetate. Drying *in vacuo* with a nitrogen bleed gave (*S*)-5-hydroxymethyl-1-(5,7-difluoro-1,2,3,4-tetrahydronaphthalen-2-yl)-1,3-dihydroimidazole-2-thione (30.4 g, 107.4 mol), m.p. 206-207°C. [α]_{D} -40° (c = 0.682, CH₃OH).

### EXAMPLE 45

### (S)-N-[3-(5,7-Difluoro-1,2,3,4-tetrahydronaphthalen-2-yl)-2-thioxo-2,3-dihydro-1H-imidazol-4-ylmethyl] formamide

The following is the preparation of a compound of Formula 26 in which t is 2, R¹ is fluoro at the 5- and 7-position and R³ is hydrogen.

Formamide (250 mL, 6.3 mol) was heated to 175°C and (*S*)-5-hydroxymethyl-1-(5,7-difluoro-1,2,3,4-tetrahydronaphthalen-2-yl)-1,3-dihydroimidazole-2-thione (25.0 g, 88.3 mmol), prepared as in Example 44 was added in portions over 30 minutes and the reaction mixture was stirred for 1 hour under a nitrogen sweep. The mixture was cooled to 50°C and 2.5 g of activated carbon, Darco®, was added. The mixture was cooled to 30°C, filtered through celite and washed in with 25 mL of formamide. The filtrate was heated to 95°C and then 1 L of water was added dropwise. The mixture was allowed to cool and then stirred at room temperature for 12 hours. The mixture was cooled to 0°C giving a crystalline material. The material was isolated by filtration and dried.

The material was stirred with approximately 5 times by weight of 70% THF/30% hexanes for five minutes. The material was isolated by filtration and the filter residue was washed with 50% THF/50% hexanes. Drying to constant weight gave (*S*)-*N*-[3-(5,7-difluoro-1,2,3,4-tetrahydronaphthalen-2-yl)-2-thioxo-2,3-dihydro-1*H*-imidazol-4-ylmethyl]formamide (19.5 g, 62.7 mmol), m.p. 245-246°C. [α]_{D} +48.9° (c = 0.613, DMSO).

Proceeding similarly as in Example 45, but subsituting urea for formamide (*S*)-5-ureidomethyl-1-(5,7-difluoro-1,2,3,4-tetrahydronaphthalen-2-yl)-1,3-dihydroimidazole-2-thione, m.p. 258-260°C, [α]_{D} +34.3° (c = 0.574, DMSO).

### EXAMPLE 46

### (S)-N-[3-(5,7-Difluoro-1,2,3,4-tetrahydronaphthalen-2-yl)-2-thioxo-2,3-dihydro-1H-imidazol-4-ylmethyl]formamide

The following is the preparation of a compound of Formula 26 in which t is 2, R¹ is fluoro at the 5- and 7-position and R³ is hydrogen.

A mixture of (*S*)-5-hydroxymethyl-1-(5,7-difluoro-1,2,3,4-tetrahydronaphthalen-2-yl)-1,3-dihydroimidazole-2-thione (1.0 g, 3.5 mmol), prepared as in Example 44, and ammonium formate (10 g, 158.6 mmol) stirred at approximately 125°C for 1 hour. The mixture was then heated to approximately 138°C and stirred for an additional 35 minutes. The mixture was diluted with 25 mL of water and allowed to cool to room temperature. The mixture was aged for approximately 18 hours giving a crystalline material. The material was isolated by filtration and the filter residue was washed with water. Drying to constant weight gave (*S*)-*N*-[3-(5,7-difluoro-1,2,3,4-tetrahydronaphthalen-2-yl)-2-thioxo-2,3-dihydro-1*H*-imidazol-4-ylmethyl]formamide (0.92 g, 2.56 mmol).

Proceeding similarly as in Example 46, but substituting ammonium acetate for ammonium formate gave (*S*)-*N*-[3-(5,7-difluoro-1,2,3,4-tetrahydro-naphthalen-2-yl)-2-thioxo-2,3-dihydro-1*H*-imidazol-4-ylmethylacetamide, m.p. 275.5-276(dec)°C. [α]_{D} +41.3° (c = 1.00, DMSO).

### EXAMPLE 47

### (S)-5-Aminomethyl-1-(5,7-difluoro-1,2,3,4-tetrahydronaphthalen-2-yl)-2,3-dihydro-2-thioxo-1H-imidazole Hydrochloride

The following is the preparation of a compound of Formula 25 in which t is 2 and R¹ is fluoro at the 5- and 7-position.

A mixture of (*S*)-*N*-[3-(5,7-difluoro-1,2,3,4-tetrahydronaphthalen-2-yl)-2-thioxo-2,3-dihydro-1*H*-imidazol-4-ylmethyl]formamide (19.1 g, 59.0 mmol), prepared as in Reference Exemple 9, and 25 mL of concentrated hydrochloric acid (12.0 M, 25 mL, 300 mmol) in 400 mL of isopropanol was heated to reflux over 12 minutes and stirred for 1 hour 40 minutes. The mixture was distilled removing 150 mL of isopropanol. The mixture was gradually cooled to room temperature and stirred for 3 hours 45 minutes. The material was isolated by filtration and the filter residue was washed with 75 mL of isopropanol. Drying *in vacuo* at 110 to 125°C with a nitrogen bleed gave (*S*)-5-aminomethyl-1-(5,7-difluoro-1,2,3,4-tetrahydro-naphthalen-2-yl)-1,3-dihydroimidazole-2-thione hydrochloride (15.6 g, 47.1 mmol), m.p. 251.9°C. [α]_{D} +10.2° (c = 0.500, DMSO).

### Example 48

### IN VITRO, DOPAMINE β-HYDROXYLASE INHIBITION

The following describes an *in vitro* assay to identify compounds that inhibit dopamine β-hydroylase (DBH). The assay relies upon the DBH-catalyzed conversion of tyramine to octopamine and the inhibition of DBH activity by test compounds.

A mixture comprising bovine adrenal DBH (13.8 mUnits/mL), Cu²⁺ (2 mM), ascorbic acid (10 mM), catalase (200 µg/mL), and test compound in 0.65 mL of 50 mM sodium acetate buffer (pH 4.5) was incubated at 37°C for 10 minutes. Tyramine was added and the reaction mixture was incubated at 37°C for 10 minutes. The reaction was quenched with 0.1 mL of concentrated ammonium hydroxide. The octopamine product was oxidized to *p*-hydroxybenzaldehyde by adding 0.2 mL of 2% sodium metaperiodate and incubating for an additional 4 minutes. Excess sodium metaperiodate was reduced with 0.2 mL of 10% sodium bisulfite and the *p*-hydroxybenzaldehyde concentration was measured by spectrophotometry at a wave length of 330 nanometers.

Alternatively, a mixture comprising bovine adrenal DBH (0.02 mUnits/mL), 0.125 M sodium acetate, 10 mM fumarate, 0.5 µM CuSO₄, 100 µg/mL catalase and 10 mM tyramine Cu²⁺ (2 mM) was incubated at 30°C for 5 minutes and then *N,N*-dimethyl-1,4-phenylenediamine (DMPD) was added to initiate the reaction. The absorbance was monitored continously by spectrophotometry at a wave length of 515 nanometers.

The test compounds were assayed over a wide range of concentrations and the concentration of test compound necessary to produce 50% inhibition of DBH activity was interpolated (ID₅₀).

Proceeding as in Example 48 the compounds of the invention were tested and found to possess DBH inhibitory activity. For example, 5-(S)-aminomethyl-1-(5,7-difluoro-1,2,3,4-tetrahydronaphthalen-2-yl)-1,3-dihydroimidazole-2-thione hydrochloride has an ID₅₀ of 8.5nM.

### Example 49

### IN VIVO DOPAMINE β-HYDROXYLASE INHIBITION

The following describes an *in vivo* assay to identify compounds that inhibit dopamine β-hydroylase (DBH). The assay relies upon dopamine and norepinephrine tissue concentrations and the affect of the test compounds thereon.

Male, normotensive or spontaneously hypertensive rats were dosed with vehicle (1 to 10 mL/kg) or test compound (0.3 to 100 mg/kg) by oral or intravenous administration. Some rats were dosed 1 to 2 times daily for up to 24 days. At 2 to 12 hours post final dose, the rats were anesthetized with halothane and decapitated. Selected tissues (e.g., cerebral cortical, medullary, mesenteric arterial and left ventricular) were rapidly harvested, weighed and placed in 0.4 mL of cold perchloric acid. Tissue concentrations of dopamine and norepinephrine were measured by HPLC and electrochemical detection methods.

The test compounds were assayed over a wide range of doses and their effects compared to those of controls. DBH inhibition was defined as a statistically significant (p≤0.05) decrease in norepinephrine concentration, a concomitant increase in dopamine concentration and an increase in the dopamine to norepinephrine ratio.

Proceeding as in Example 49 the compounds of the invention were tested and found to possess DBH inhibitory activity. The effect of 5-(S)-aminomethyl-1-(5,7-difluoro-1,2,3,4-tetrahydronaphthalen-2-yl)-1,3-dihydroimidazole-2-thione hydrochloride on dopamine and norepinephrine (g/g of tissue) and the dopamine/norepinephrine ratio (g/g) in the left ventricle of spontaneously hypertensive rats following three oral doses given 12 hours part, is summarized in the following table. Values are presented as the mean (9 rats / group) ± standard deviation.

| Dose (mg/Kg, po) | Norepinephrine | Dopamine | Dopamine/Norepinephrine |
|---|---|---|---|
| 0 | 1.30 ± 0.18 | 0.02 ± 0.01 | 0.02 ± 0.00 |
| 3 | 1.26 ± 0.64 | 0.06 ± 0.01 | 0.05 ± 0.02 |
| 10 | 1.04 ± 0.37 | 0.09 ± 0.02 | 0.09 ± 0.02 |
| 30 | 0.95 ± 0.21 | 0.14 ± 0.03 | 0.15 ± 0.03 |
| 100 | 0.85 ± 0.17 | 0.18 ± 0.02 | 0.22 ± 0.04 |

### Example 50

### BLOOD PRESSURE LOWERING EFFECTS

The following describes an assay to identify compounds that lower blood pressure.

Male, spontaneously hypertensive rats were anesthetized and a femoral or carotid artery was cannulated for continuous blood pressure monitoring. The rats were allowed 30 to 60 minutes to recover from the anesthesia and then basal blood pressure levels were obtained. The rats were dosed with vehicle (10 mL/kg) or test compound (0.3 to 30 mg/kg) by oral or intravenous administration and followed for 4 to 6 hours.

The test compounds were assayed over a wide range of doses and blood pressure lowering activity was defined as a statistically significant (p≤0.05) lowering of blood pressure as compared to the vehicle treated rats.

Proceeding is in Example 50 the compounds of the invention were tested and found to possess blood pressure lowering activity. For example, effect of 5-(S)-aminomethyl-1-(5,7-difluoro-1,2,3,4-tetrahydronaphthalen-2-yl)-1,3-dihydroimidazole-2-thione hydrochloride on the change in mean arterial blood pressure from pre-dose to 4 hours after administration of a single oral dose (mg/Kg) given to restrained spontaneously hypertensive rats is summarized in the following table.

| Dose (mg/Kg) | Change in Mean Arterial Pressure |
|---|---|
| 0 | 20 mmHg |
| 0.3 | 27 mmHg |
| 1.0 | 32 mmHg |
| 3.0 | 41 mmHg |
| 10 | 53 mmHg |

### Example 51

### Toxicity

5-(S)-Aminomethyl-1-(5,7-difluoro-1,2,3,4-tetrahydronaphthalen-2-yl)-1,3-dihydroimidazole-2-thione hydrochloride was not mutagenic in a battery of assays. The lethal oral dose of the compound was 1000 mg/Kg in mice, and greater than 2500 and 400 mg/Kg, respectively in rats and dogs.

### Example 52

The following are representative pharmaceutical formulations containing a compound of Formula I.

### ORAL FORMULATION

A representative solution for oral administration contains:

| | |
|---|---|
| Compound of Formula I | 70-700 mg |
| Citric Acid Monohydrate | 105 mg |
| Sodium Hydroxide | 18 mg |
| Flavoring | |
| Water | q.s. to 100 mL |

### INTRAVENOUS FORMULATION

A representative solution for intravenous administration contains:

| | |
|---|---|
| Compound of Formula I | 7-70 mg |
| Dextrose Monohydrate | q.s to make isotonic |
| Citric Acid Monohydrate | 1.05 mg |
| Sodium Hydroxide | 0.18 mg |
| Water for Injection | q.s. to 1.0 mL |

### TABLET FORMULATION

A representative tablet form of a compound of Formula I may contain:

| | |
|---|---|
| Compound of Formula I | 25 % |
| Microcrystalline cellulose | 54 % |
| Stearic Acid | 20 % |
| Colloidal Silica | 1 % |

Proceeding as in Example 52 resentative pharmaceutical formulations containing a compound of Formula II or III can be prepared.

## Claims

1. Compounds of Formula I, II or III 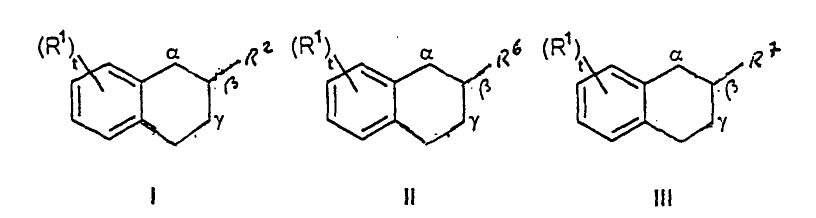 in which:
**t** is 0, 1, 2 or 3;
**R**^{**1**} is independently halo, hydroxy or (C₁₋₄)alkyloxy; and
**R**^{**2**} is a group of Formula (a): 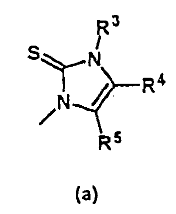 in which:
**R**^{**3**} is hydro, **R**^{**4**} is hydro, and **R**^{**5**} is NHR¹⁰; or
**R**^{**3**} is -(CH₂)_{q}R⁹, **R**^{**4**} is hydro and **R**^{**5**} is hydro; or
**R**^{**3**} is -(CH₂)_{q}R⁹, **R**^{**4**} is hydro and **R**^{**5**} is NHR¹⁰; or
**R**^{**3**} is -NHR¹⁰ and **R**^{**4**} and **R**^{**5**} are each hydro; or
**R**^{**3**} is hydro or -(CH₂)_{q}R⁹, **R**^{**4**} is (C₁₋₄)alkyl, di(C₁₋₄)alkylaminomethyl, piperidin-1-ylmethyl, morpholin-4-ylmethyl, formyl, 1-hydroxy(C₁₋₄)alkyl or-CH₂NHR¹³ and **R**^{**5**} is hydro; or
**R**^{**3**} is hydro or -(CH₂)_{q}R⁹, **R**^{**4**} is hydro, (C₁₋₄)alkyl or -C(O)R¹⁴ and **R**^{**5**} is cyano, hydroxymethyl, 1H-tetrazol-5-yl, 4,5-dihydroimidazol-2-yl, pyrrolidin-1-ylmethyl, piperidin-1-ylmethyl, morpholin-4-ylmethyl, piperazin-1-ylmethyl, 4-(C₁₋₄)alkylpiperazin-1-ylmethyl, -C(O)R¹⁴, -C(NH)NR¹⁵R¹⁶ or -CH₂NR¹⁰R¹⁷; or
**R**^{**3**} is hydro or -(CH₂)_{q}R⁹ and **R**^{**4**} and **R**^{**5**} are dependently di(C₁₋₄)alkylaminomethyl, piperidin-1-ylmethyl, morpholin-4-ylmethyl or hydroxymethyl;
**R**^{**6**} is a group of Formula (d) : 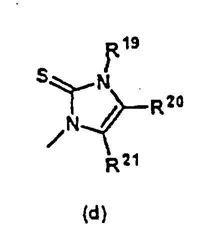 in which:
**R**^{**19**} is hydro or -(CH₂)_{q}R⁹, **R**^{**20**} is hydro and **R**^{**21**} is -NR²⁵R²⁶; or
**R**^{**19**} is -NR²⁵R²⁶, **R**^{**20**} and **R**^{**21**} are each hydro: or
**R**^{**19**} is hydro or -(CH₂)_{q}R⁹, **R**^{**20**} is -CH₂NR²⁵R²⁶ and **R**^{**21**} is hydro; or
**R**^{**19**} is hydro or -(CH₂)_{q}R⁹, **R**^{**20**} is hydro, (C₁₋₄)alkyl or -C(O)R¹⁴ and **R**^{**21**} is -CH₂NR²⁵R²⁶;
**R**^{**7**} is a group of Formula (g): 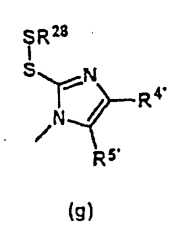 in which:
**R**^{**4**}**'** is hydro and **R**^{**5**}**'** is -NHR¹⁰; or
**R**^{**4**}' is (C₁₋₄)alkyl, di(C₁₋₄)alkylaminomethyl, piperidin-1-ylmethyl, morpholin-4-ylmethyl, 1-hydroxy(C₁₋₄)alkyl or -CH₂NHR¹³ and **R**^{**5**}**'** is hydro; or
**R**^{**4**}**'** is hydro, (C₁₋₄)alkyl or -C(O)R¹⁴ and **R**^{**5**}**'** is hydroxymethyl, 1H-tetrazol-5-yl, 4,5-dihydroimidazol-2-yl, pyrrolidin-1-ylmethyl, piperidin-1-ylmethyl, morpholin-4-ylmethyl, piperazin-1-ylmethyl, 4-(C₁₋₄)alkylpiperazin-1-ylmethyl, -C(O)R¹⁴, -C(NH)NR¹⁵R¹⁶ or -CH₂NR¹⁰R¹⁷; or
**R**^{**4**}**'** and **R**^{**5**}**'** are dependently di(C₁₋₄)alkylaminomethyl, piperidin-1-ylmethyl, morpholin-4-ylmethyl or hydroxymethyl;
and
**R**^{**28**} is (C₂₋₆)alkyl {which alkyl is further substituted by one to two substituents independently selected from -N(R²⁹)₂, -C(O)OR³⁰, -PO(OR³⁰)₂, -SO₃R³⁰, -SO₂NHR³⁰ and -OR³⁰};
**q** is 0, 1, 2, 3 or 4;
**R**^{**9**} is carboxy, (C₁₋₄)alkyloxycarbonyl, carbamoyl or a group selected from aryl and heteroaryl (which group is optionally further substituted with one to two substituents independently selected from hydroxy, (C₁₋₄)alkyloxy, cyano, 1H-tetrazo-5-yl, carboxy and (C₁₋₄)alkyloxycarbonyl);
**R**^{**10**} is hydro, (C₁₋₄)alkanoyl, trifluoro(C₁₋₄)alkanoyl, carbamoyl, (C₁₋₄)alkyloxycarbonyl, (C₁₋₄)alkylcarbamoyl, di(C₁₋₄)alkylcarbamoyl, amino(C₁₋₄)alkanoyl, (C₁₋₄)alkylamino(C₁₋₄)-alkanoyl, di(C₁₋₄)alkylamino(C₁₋₄)alkanoyl, a group selected from aroyl and heteroaroyl (which aroyl and heteroaroyl are optionally further substituted with one to two substituents independently selected from hydroxy, (C₁₋₄)alkyloxy, cyano, 1H-tetrazol-5-yl, carboxy and (C₁₋₄)alkyloxycarbonyl) or -C(NR¹¹)NHR¹²;
**R**^{**11**} and **R**^{**12**} are independently hydro, acetyl or tert-butoxycarbonyl;
**R**^{**13**} is hydro, (C₁₋₄)alkyl, (C₁₋₄)alkanoyl, trifluoro(C₁₋₄)alkanoyl, carbamoyl, (C₁₋₄)alkyloxycarbonyl, (C₁₋₄)alkylcarbamoyl, di(C₁₋₄)alkylcarbamoyl, amino(C₁₋₄)alkanoyl, (C₁₋₄)alkylamino(C₁₋₄)alkanoyl, di(C₁₋₄)alkylamino(C₁₋₄)alkanoyl, carboxy(C₁₋₄)alkyl, (C₁₋₄)alkyloxycarbonyl(C₁₋₄)alkyl, carbamoyl(C₁₋₄)alkyl, a group selected from aroyl, heteroaroyl, aryl(C₁₋₄)alkyl and heteroaryl(C₁₋₄)alkyl (which aroyl, heteroaroyl, aryl and heteroaryl are optionally further substituted with one to two substituents independently selected from hydroxy, (C₁₋₄)alkyloxy, cyano, 1H-tetrazol-5-yl, carboxy and (C₁₋₄)alkyloxycarbonyl) or -C(NR¹¹)NHR¹²);
**R**^{**14**} is amino, hydroxy, (C₁₋₄)alkyloxy, 2-(dimethylamino)ethylamino, 4-methylpiperazin-1-yl, 2-(dimethylamino)-ethylmercapto, 4-(methylsulfonylamino)anilino or 1H-tetrazol-5-ylamino;
**R**^{**15**} and **R**^{**16**} are independently hydro, (C₁₋₄)alkyl or trifluoro(C₁₋₄)alkyl;
**R**^{**17**} is hydro or (C₁₋₄)alkyl;
**R**^{**25**} is hydro or (C₁₋₄)alkyl;
**R**^{**26**} is L-alanyl, L-arginyl, L-asparaginyl, L-α-aspartyl; L-β-aspartyl, L-cysteinyl, L-glutaminyl, L-α-glutamyl, L-γ-glutamyl, N-(C₁₋₄)-alkanoyl-L-α-glutamyl, N-(C₁₋₄)alkanoyl-L-γ-glutamyl, glycyl, L-histidyl, L-isoleucyl, L-leucyl, L-lysyl, L-methionyl, L-ornithinyl, L-phenylalanyl, L-prolyl, L-seryl, L-threonyl, L-tryptophyl, L-tyrosyl, L-valyl, 1-amino-cyclopropylcarbonyl, 1-aminocyclobutylcarbonyl, 1-aminocyclo-pentylcarbonyl or 1-aminocyclohexylcarbonyl;
**R**^{**29**} is independently hydro, acetyl or trifluoroacetyl;
**R**^{**30**} is independently hydro or (C₁₋₅)alkyl;
**aryl** means an organic radical derived from an aromatic hydrocarbon containing 6 to 14 carbon atoms and includes monocyclic or condensed carbocyclic aromatic rings; the aryl residue is optionally further substituted with one to two substituents independently selected from halo and cyano;
**aroyl** means the radical -C(O)R, wherein R is aryl as defined above;
**heteroaryl** means an organic radical derived from an aromatic hydrocarbon containing 5 to 14 atoms, 1 to 5 of which are hetero atoms chosen from N, O, or S, and includes monocyclic, condensed heterocyclic and condensed carbocyclic and heterocyclic aromatic rings; the heteroaryl residue is optionally further substituted with one to two substituents independently selected from halo and cyano;
**heteroaroyl** means the radical -C(O)R, wherein R is heteroaryl as defined above;
and the pharmaceutically acceptable salts, individual isomers, and mixtures of isomers thereof.

2. Compounds of formula I according to claim 1, wherein R⁵ is di(C₁₋₄)alkylaminomethyl, pyrrolidin-1-ylmethyl, piperidin-1-ylmethyl, morpholin-4-ylmethyl, piperazin-1-ylmethyl, 4-(C₁₋₄)alkylpiperazin-1-ylmethyl or -CH₂NHR¹⁰.

3. Compounds according to claim 2, wherein R⁵ is ureidomethyl, aminomethyl or acetylaminomethyl.

4. Compounds according to claim 3, wherein t is 2 and each R¹ is fluoro.

5. Compounds according to claim 4, wherein R¹ is fluoro at the 5- and 7-position.

6. Compound of formula I according to claim 1, selected from the group consisting of 5-aminomethyl-1-(5,7-difluoro-1,2,3,4-tetrahydronaphthalen-2-yl)-1,3-dihydroimidazole-2-thione or a pharmaceutically acceptable salt thereof, (S)-5-aminomethyl-1-(5,7-difluoro-1,2,3,4-tetrahydronaphthalen-2-yl)-1,3-dihydroimidazole-2-thione hydrochloride, 5-ureidomethyl-1-(5,7-difluoro-1,2,3,4-tetrahydronaphthalen-2-yl)-1,3-dihydroimidazole-2-thione, (S)-5-ureidomethyl-1-(5,7-difluoro-1,23,4-tetrahydronaphthalen-2-yl)-1,3-dihydroimidazole-2-thione, N-[3-(5,7-difluoro-1,2,3,4-tetrahydronaphthalen-2-yl)-2-thioxo-2,3-dihydro-1H-imidazol-4-ylmethyl]acetamide, (S)-N-[3-(5,7-difluoro-1,2,3,4-tetrahydronaphthalene-2-yl)-2-thioxo-2,3-dihydro-1H-imidazol-4-ylmethyl]acetamide.

7. Compounds of formula II according to claim 1, wherein t is 2, each R¹ is fluoro and R²¹ is -CH₂NHR²⁶.

8. Compounds according to claim 7, wherein R¹ is fluoro at the 5- and 7-position and R²⁶ is L-arginyl, L-α-aspartyl, L-β-aspartyl, L-histidyl or L-ornithyl.

9. Compound of formula II according to claim 1, selected from the group consisting of 3S-amino-N-[3-(57-difluoro-1,2,3,4-tetrahydronaphthalen-2-yl)-2-thioxo-2,3-dihydro-1H-imidazol-4-yl-methyl]succinamic acid and the pharmaceutically acceptable salts thereof, 3S-amino-N-[3-(5,7-difluoro-1,2,3,4-tetrahydronaphthalen-2S-yl)-2-thioxo-2,3-dihydro-1H-imidazol-4-yl-methyl]succinamic acid hydrochloride, 2S-amino-3-(3H-imidazol-4-yl)-N-[3-(1,2,3,4-tetrahydronaphthalen-2-yl)-2-thioxo-2,3-dihydro-1H-imidazol-1-yl]propionamide and the pharmaceutically acceptable salts thereof, 2S-amino-3-(3H-imidazol-4-yl)-N-[3-(1,2,3,4-tetrahydronaphthalen-2S-yl)-2-thioxo-2,3-dihydro-1H-imidazol-1-yl]propionamide dihydrochloride, 2S,5-diamino-N-[3-(5,7-difluoro-1,2,3,4-tetrahydronaphthalen-2-yl)-2-thioxo-2,3-dihydro-1H-imidazol-4-ylmethyl]valeramide and the pharmaceutically acceptable salts thereof and 2S,5-diamino-N-[3-(5,7-difluoro-1,2,3,4-tetrahydronaphthalen-2S-yl)-2-thioxo-2,3-dihydro-1H-imidazol-4-yl-methyl]valeramide dihydrochloride.

10. Compounds of formula III according to claim 1, wherein t is 2, each R¹ is fluoro.

11. Compounds according to claim 10, wherein R¹ is fluoro at the 5- and 7-position.

12. Compounds according to claim 11, wherein R²⁸ is a group selected from ethyl, 1,1-dimethylethyl and propyl (which group is further sustituted with one to two substituents independently selected from carboxy, methoxycarbonyl, amino and trifluoroacetylamino).

13. Compounds according to claim 12, wherein R²⁸ is (R)-2-amino-2-methoxycarbonylethyl, (R)-2-amino-2-carboxyethyl, (R)-2-trifluoracetylamino-2-methoxycarbonylethyl, 2-aminoethyl, (S)-2-amino-2-carboxy-1,1-dimethylethyl or 3-amino-3-carboxyprop-1-yl.

14. Pharmaceutical composition comprising a compound according to any of claims 1 to 13.

15. Compound according to any of claims 1 to 13 as a therapeutic substance.

16. Use of a compound according to any of claims 1 to 13 for the preparation of a medicament for treating a condition which can be ameliorated by a drug which inhibits dopamine β-hydroxylase in an animal, namely for treating Parkinson's disease, hypertension or congestive heart failure.

17. (*S*)-5,7-difluoro-1,2,3,4-tetrahydronaphthalen-2-ylamine:

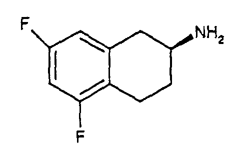

18. A process for the preparation of (*S*)-5,7-difluoro-1,2,3,4-tetrahydronaphthalen-2-ylamine: 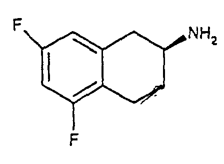 which process comprises:
(a) reducing, in the presence of 2*S*-dimethylamino-1*R*-phenylpropanol and 2-ethylaminopyridine, the compound of Formula: 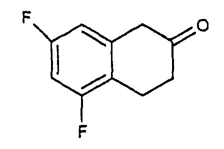 to give the (*R*)-enantiomer of Formula: 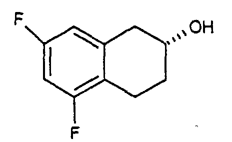
(b) treating the (*R*)-enantiomer with methanesulfonyl chloride and then reacting with an azide salt to give the (*S*)-enantiomer of Formula: 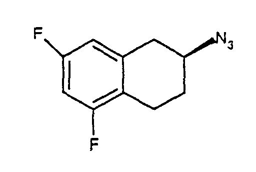 and
(c) reducing.

## Patentansprüche

1. Verbindungen der Formeln I, II oder III 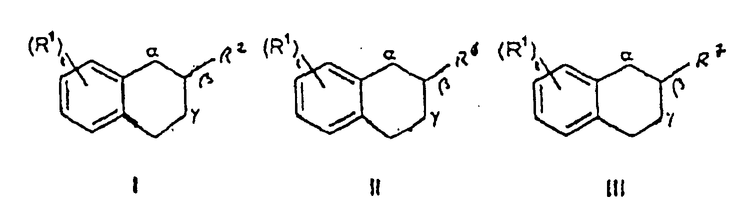 worin
t 0, 1, 2 oder 3 ist;
R¹ unabhängig Halogen, ein Hydroxy- oder (C₁-C₄)-Alkyloxyrest ist und
R² eine Gruppe der Formel (a) ist, 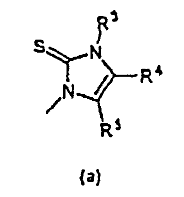 worin
R³ Wasserstoff ist, R⁴ Wasserstoff ist und R⁵ NHR¹⁰ ist oder
R³ -(CH₂)_{q}R⁹ ist, R⁴ Wasserstoff ist und R⁵ Wasserstoff ist oder
R³ -(CH₂)_{q}R⁹ ist, R⁴ Wasserstoff ist und R⁵ -NHR¹⁰ ist oder
R³ -NHR¹⁰ ist und R⁴ und R⁵ jeweils Wasserstoff sind oder R³ Wasserstoff oder -(CH₂)_{q}R⁹ ist, R⁴ ein (C₁-C₄)-Alkyl-, Di-(C₁-C₄)-alkylaminomethyl-, Piperidin-1-ylmethyl-, Morpholin-4-ylmethyl-, Formyl-, 1-Hydroxy-(C₁-C₄)-alkylrest oder -CH₂NHR¹³ ist und R⁵ Wasserstoff ist oder R³ Wasserstoff oder -(CH₂)_{q}R⁹ ist, R⁴ Wasserstoff, ein (C₁-C₄)-Alkylrest oder -C(O)R¹⁴ ist und R⁵ ein Cyano-, Hydroxymethyl-, 1H-Tetrazol-5-yl-, 4,5-Dihydroimidazol-2-yl-, Pyrrolidin-1-ylmethyl-, Piperidin-1-ylmethyl-, Morpholin-4-ylmethyl-, Piperazin-1-ylmethyl-, 4-(C₁-C₄)-Alkylpiperazin-1-ylmethylrest, -C(O)R¹⁴, -C(NH)NR¹⁵R¹⁶ oder -CH₂NR¹⁰R¹⁷ ist oder
R³ Wasserstoff oder -(CH₂)_{q}R⁹ ist und R⁴ und R⁵ abhängig Di-(C₁-C₄)-alkylaminomethyl-, Piperidin-1-ylmethyl-, Morpholin-4-ylmethyl- oder Hydroxymethylreste sind;
R⁶ eine Gruppe ist mit der Formel (d) 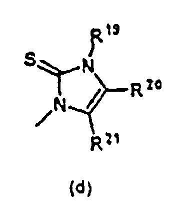 worin
R¹⁹ Wasserstoff oder -(CH₂)_{q}R⁹ ist, R²⁰ Wasserstoff ist und R²¹ -NR²⁵R²⁶ ist oder
R¹⁹ -NR²⁵R²⁶ ist, R²⁰ und R²¹ jeweils Wasserstoff sind oder R¹⁹ Wasserstoff oder -(CH₂)_{q}R⁹ ist, R²⁰ -CH₂NR²⁵R²⁶ ist und R²¹ Wasserstoff ist oder
R¹⁹ Wasserstoff oder -(CH₂)_{q}R⁹ ist, R²⁰ Wasserstoff, ein (C₁-C₄)-Alkylrest oder -C(O)R¹⁴ ist und R²¹ -CH₂NR²⁵R²⁶ ist;
R⁷ eine Gruppe der Formel (g) 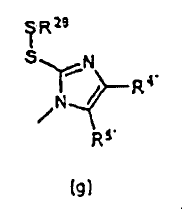 ist, worin
R^{4'} Wasserstoff ist und R^{5'} -NHR¹⁰ ist oder
R^{4'} ein (C₁-C₄) -Alkyl-, Di- (C₁-C₄) -alkylaminomethyl-, Piperidin-1-ylmethyl-, Morpholin-4-ylmethyl-, 1-Hydroxy-(C₁-C₄)-alkylrest oder -CH₂NHR¹³ ist und R^{5'} Wasserstoff ist oder
R^{4'} Wasserstoff, ein (C₁-C₄)-Alkylrest oder -C(O)R¹⁴ ist und R^{5'} ein Hydroxymethyl-, 1H-Tetrazol-5-yl-, 4,5-Dihydroimidazol-2-yl-, Pyrrolidin-1-ylmethyl-, Piperidin-1-ylmethyl-, Morpholin-4-ylmethyl-, Piperazin-1-ylmethyl-, 4-(C₁-C₄)-Alkylpiperazin-1-ylmethylrest, -C(O)R¹⁴, -C(NH)NR¹⁵R¹⁶ oder -CH₂NR¹⁰R¹⁷ ist oder
R^{4'} und R^{5'} abhängig Di- (C₁-C₄)-alkylaminomethyl-, Piperidin-1-ylmethyl-, Morpholin-4-ylmethyl- oder Hydroxymethylreste sind und
R²⁸ ein (C₂-C₆)-Alkylrest ist (wobei der Alkylrest weiterhin mit 1 oder 2 Substituenten substituiert ist, die unabhängig ausgewählt sind aus -N(R²⁹)₂, -C(O)OR³⁰, -PO(OR³⁰)₂, -SO₃R³⁰, -SO₂NHR³⁰ und -OR³⁰);
q 0, 1, 2, 3 oder 4 ist;
R⁹ ein Carboxy-, (C₁-C₄)-Alkyloxycarbonyl-, Carbamoylrest oder eine Gruppe ist ausgewählt aus Aryl- und Heteroarylgruppen (wobei die Gruppe gegebenenfalls weiter mit 1 oder 2 Substituenten substituiert ist, die unabhängig ausgewählt sind aus Hydroxy-, (C₁-C₄)-Alkyloxy-, Cyano-, 1H-Tetrazo-5-yl-, Carboxy- und (C₁-C₄)-Alkyloxycarbonylresten) ;
R¹⁰ Wasserstoff, ein (C₁-C₄)-Alkanoyl-, Trifluor- (C₁-C₄)-alkanoyl-, Carbamoyl-, (C₁-C₄)-Alkyloxycarbonyl-, (C₁-C₄)-Alkylcarbamoyl-, Di-(C₁-C₄)-Alkylcarbamoyl-, Amino-(C₁-C₄)-alkanoyl-, (C₁-C₄)-Alkylamino-(C₁-C₄)-alkanoyl-, Di-(C₁-C₄)-alkylamino-(C₁-C₄)-alkanoylrest, eine Gruppe ausgewählt aus Aroyl- und Heteroaroylgruppen (wobei die Aroyl- und Heteroaroylgruppen gegebenenfalls weiter mit 1 oder 2 Substituenten substituiert sind, die unabhängig ausgewählt sind aus Hydroxy-, (C₁-C₄)-Alkyloxy-, Cyano-, 1H-Tetrazol-5-yl-, Carboxy- und (C₁-C₄)-Alkyloxycarbonylresten) oder -C(NR¹¹)NHR¹² ist;
R¹¹ und R¹² unabhängig Wasserstoff, Acetyl- oder tert.-Butoxycarbonylreste sind;
R¹³ Wasserstoff, ein (C₁-C₄)-Alkyl-, (C₁-C₄)-Alkanoyl-, Trifluor- (C₁-C₄)-alkanoyl-, Carbamoyl-, (C₁-C₄)-Alkyloxycarbonyl-, (C₁-C₄)-Alkylcarbamoyl-, Di-(C₁-C₄)-alkylcarbamoyl-, Amino-(C₁-C₄)-alkanoyl-, (C₁-C₄)-Alkylamino-(C₁-C₄)-alkanoyl-, Di-(C₁-C₄)-alkylamino-(C₁-C₄)-alkanoyl-, Carboxy-(C₁-C₄)-alkyl-, (C₁-C₄)-Alkyloxycarbonyl-(C₁-C₄)-alkyl-, Carbamoyl-(C₁-C₄)-alkylrest, eine Gruppe ausgewählt aus Aroyl-, Heteroaroyl-, Aryl-(C₁-C₄)-alkylund Heteroaryl-(C₁-C₄)-alkylgruppen (wobei die Aroyl-, Heteroaroyl-, Aryl- und Heteroarylgruppen gegebenenfalls weiter mit 1 oder 2 Substituenten substituiert sind, die unabhängig ausgewählt sind aus Hydroxy-, (C₁-C₄)-Alkyloxy-, Cyano-, 1H-Tetrazol-5-yl-, Carboxy- und (C₁-C₄)-Alkyloxycarbonylresten) oder -C(NR¹¹)NHR¹² ist;
R¹⁴ ein Amino-, Hydroxy-, (C₁-C₄)-Alkyloxy-, 2-(Dimethylamino)ethylamino-, 4-Methylpiperazin-1-yl-, 2-(Dimethylamino)ethylmercapto-, 4-(Methylsulfonylamino)anilinooder 1H-Tetrazol-5-ylaminorest ist;
R¹⁵ und R¹⁶ unabhängig Wasserstoff, (C₁-C₄)-Alkyl- oder Trifluor- (C₁-C₄)-alkylreste sind;
R¹⁷ Wasserstoff oder ein (C₁-C₄)-Alkylrest ist;
R²⁵ Wasserstoff oder ein (C₁-C₄)-Alkylrest ist;
R²⁶ ein L-Alanyl-, L-Arginyl-, L-Asparaginyl-, L-α-Aspartyl-, L-β-Aspartyl-, L-Cysteinyl-, L-Glutaminyl-, L-α-Glutamyl-, L-γ-Glutamyl-, N-(C₁-C₄)-Alkanoyl-L-α-glutamyl-, N-(C₁-C₄)-Alkanoyl-L-γ-glutamyl-, Glycyl-, L-Histidyl-, L-Isoleucyl-, L-Leucyl-, L-Lysyl-, L-Methionyl-, L-Ornithinyl-, L-Phenylalanyl-, L-Prolyl-, L-Seryl-, L-Threonyl-, L-Tryptophyl-, L-Tyrosyl-, L-Valyl-, 1-Aminocyclopropylcarbonyl-, 1-Aminocyclobutylcarbonyl-, 1-Aminocyclopentylcarbonyl- oder 1-Aminocyclohexylcarbonylrest ist;
R²⁹ unabhängig Wasserstoff, ein Acetyl- oder Trifluoracetylrest ist;
R³⁰ unabhängig Wasserstoff oder ein (C₁-C₅)-Alkylrest ist;
wobei ein Arylrest einen organischen Rest bedeutet, der von einem aromatischen Kohlenwasserstoff mit 6 bis 14 Kohlenstoffatomen abgeleitet ist und monocyclische oder kondensierte carbocyclische aromatische Ringe einschließt, wobei der Arylrest gegebenenfalls weiter mit 1 bis 2 Substituenten substituiert ist, die unabhängig ausgewählt sind aus Halogen- und Cyanoresten; ein Aroylrest den Rest -C(O)R bedeutet, worin R ein Arylrest wie oben definiert ist;
ein Heteroarylrest einen organischen Rest bedeutet, der von einem aromatischen Kohlenwasserstoff mit 5 bis 14 Atomen, von denen 1 bis 5 Heteroatome ausgewählt aus N, O oder S sind, abgeleitet ist und monocyclische kondensierte heterocyclische und kondensierte carbocyclische und heterocyclische aromatische Ringe einschließt, wobei der Heteroarylrest gegebenenfalls weiter mit 1 bis 2 Substituenten substituiert ist, die unabhängig ausgewählt sind aus Halogen- und Cyanoresten; ein Heteroaroylrest den Rest -C(O)R bedeutet, worin R ein Heteroarylrest ist, wie oben definiert;
und die pharmazeutisch annehmbaren Salze, einzelnen Isomeren und Mischungen von Isomeren davon.

2. Verbindungen der Formel I gemäß Anspruch 1, worin R⁵ ein Di-(C₁-C₄)-alkylaminomethyl-, Pyrrolidin-1-ylmethyl-, Piperidin-1-ylmethyl-, Morpholin-4-ylmethyl-, Piperazin-1-ylmethyl-, 4-(C₁-C₄)-Alkylpiperazin-1-ylmethylrest oder -CH₂NHR¹⁰ ist.

3. Verbindungen nach Anspruch 2, worin R⁵ ein Ureidomethyl-, Aminomethyl- oder Acetylaminomethylrest ist.

4. Verbindungen nach Anspruch 3, worin t 2 ist und jeder Rest R¹ Fluor ist.

5. Verbindungen nach Anspruch 4, worin R¹ Fluor an Position 5 und 7 ist.

6. Verbindung der Formel I nach Anspruch 1, ausgewählt aus der Gruppe bestehend aus 5-Aminomethyl-1-(5,7-difluor-1,2,3,4-tetrahydronaphthalin-2-yl)-1,3-dihydroimidazol-2-thion oder einem pharmazeutisch annehmbaren Salz davon, (S)-5-Aminomethyl-1-(5,7-difluor-1,2,3,4-tetrahydronaphthalin-2-yl)-1,3-dihydroimidazol-2-thionhydrochlorid, 5-Ureidomethyl-1-(5,7-difluor-1,2,3,4-tetrahydronaphthalin-2-yl)-1,3-dihydroimidazol-2-thion, (S)-5-Ureidomethyl-1-(5,7-difluor-1,2,3,4-tetrahydronaphthalin-2-yl)-1,3-dihydroimidazol-2-thion, N-[3-(5,7-Difluor-1,2,3,4-tetrahydronaphthalin-2-yl)-2-thioxo-2,3-dihydro-1H-imidazol-4-ylmethyl]acetamid, (S)- N-[3-(5,7-Difluor-1,2,3,4-tetrahydronaphthalin-2-yl)-2-thioxo-2,3-dihydro-1H-imidazol-4-ylmethyl]acetamid.

7. Verbindungen der Formel II nach Anspruch 1, worin t 2 ist, jeder Rest R¹ Fluor ist und R²¹ -CH₂NHR²⁶ ist.

8. Verbindungen nach Anspruch 7, worin R¹ Fluor an Position 5 und 7 ist und R²⁶ L-Arginyl, L-α-Aspartyl, L-β-Aspartyl, L-Histidyl oder L-Ornithyl ist.

9. Verbindung der Formel II nach Anspruch 1 ausgewählt aus der Gruppe bestehend aus 3S-Amino-N-[3-(5,7-difluor-1,2,3,4-tetrahydronaphthalin-2-yl)-2-thioxo-2,3-dihydro-1H-imidazol-4-ylmethyl]succinamidsäure und den pharmazeutisch annehmbaren Salzen davon, 3S-Amino-N-[3-(5,7-difluor-1,2,3,4-tetrahydronaphthalin-2S-yl)-2-thioxo-2,3-dihydro-1H-imidazol-4-ylmethyl]succinamidsäurehydrochlorid, 2S-Amino-3-(3H-imidazol-4-yl)-N-[3-(1,2,3,4-tetrahydronaphthalin-2-yl)-2-thioxo-2,3-dihydro-lH-imidazol-1-yl]propionamid und den pharmazeutisch annehmbaren Salzen davon, 2S-Amino-3-[3H-imidazol-4-yl)-N-[3-(1,2,3,4-tetrahydronaphthalin-2S-yl)-2-thioxo-2,3-dihydro-1H-imidazol-1-yl]propionamiddihydrochlorid, 2S, 5-Diamino-N-[3-(5,7-difluor-1,2,3,4-tetrahydronaphthalin-2-yl)-2-thioxo-2,3-dihydro-1H-imidazol-4-ylmethyl]valeramid und den pharmazeutisch annehmbaren Salzen davon und 2S,5-Diamino-N-[3-(5,7-difluor-1,2,3,4-tetrahydronaphthalin-2S-yl)-2-thioxo-2,3-dihydro-1H-imidazol-4-ylmethyl]valeramiddihydrochlorid.

10. Verbindungen der Formel III nach Anspruch 1, worin t 2 ist, jeder Rest R¹ Fluor ist.

11. Verbindungen nach Anspruch 10, worin R¹ Fluor an Position 5 und 7 ist.

12. Verbindungen nach Anspruch 11, worin R²⁸ eine Gruppe ist ausgewählt aus Ethyl-, 1,1-Dimethylethyl- und Propylresten (wobei die Gruppe weiterhin mit 1 oder 2 Substituenten substituiert ist, die unabhängig ausgewählt sind aus Carboxy-, Methoxycarbonyl-, Aminound Trifluoracetylaminoresten).

13. Verbindungen nach Anspruch 12, worin R²⁸ (R)-2-Amino-2-methoxycarbonylethyl, (R)-2-Amino-2-carboxyethyl, (R)-2-Trifluoracetylamino-2-methoxycarbonylethyl, 2-Aminoethyl, (S) -2-Amino-2-carboxy-1,1-dimethylethyl oder 3-Amino-3-carboxyprop-1-yl ist.

14. Pharmazeutische Zusammensetzung enthaltend eine Verbindung nach einem der Ansprüche 1 bis 13.

15. Verbindung nach einem der Ansprüche 1 bis 13 als therapeutische Substanz.

16. Verwendung einer Verbindung nach einem der Ansprüche 1 bis 13 zur Herstellung eines Arzneimittels zur Behandlung eines Zustandes, der durch ein Arzneimittel verbessert werden kann, das Dopamin-β-hydroxylase bei einem Tier hemmt, nämlich zur Behandlung von Parkinson-Krankheit, Bluthochdruck oder Stauungsherzinsuffizienz.

17. (S)-5,7-Difluor-1,2,3,4-tetrahydronaphthalin-2-ylamin:

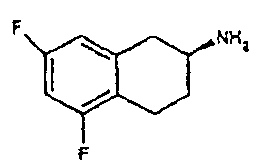

18. Verfahren zur Herstellung von (S)-5,7-Difluor-1,2,3,4-tetrahydronaphthalin-2-ylamin 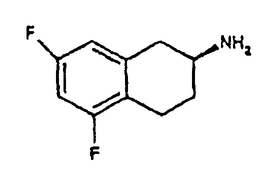 wobei das Verfahren umfasst
(a) dass die Verbindung der Formel 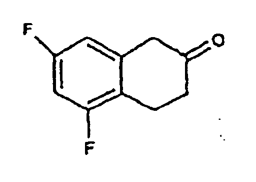 in Gegenwart von 2S-Dimethylamino-1R-phenylpropanol und 2-Ethylaminopyridin reduziert wird, was das (R)-Enantiomer der Formel 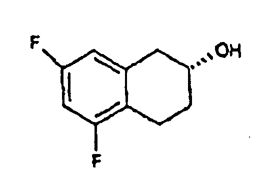 ergibt,
(b) das (R)-Enantiomer mit Methansulfonylchlorid behandelt wird und dann mit einem Azidsalz umgesetzt wird, was das (S)-Enantiomer der Formel 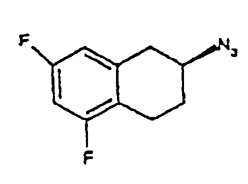 ergibt und
(c) reduziert wird.

## Revendications

1. Composés de la formule I, II ou III : 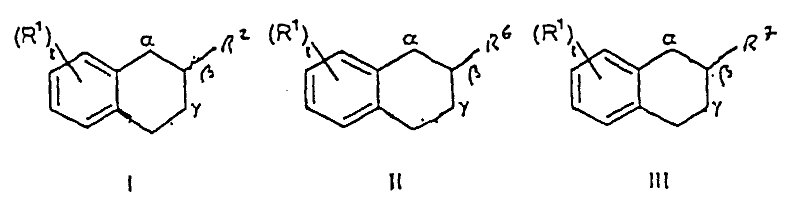 dans lesquelles t vaut 0, 1, 2 ou 3,
R¹ est indépendamment un groupe halo, hydroxy ou alkyloxy en C₁-C₄, et
R² est un groupe de formule (a) : 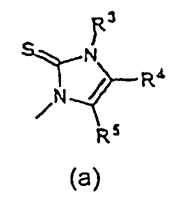 où
R³ est un groupe hydro, R⁴ est un groupe hydro et R⁵ est -NHR¹⁰, ou
R³ est -(CH₂)_{q}R⁹, R⁴ est un groupe hydro et R⁵ est un groupe hydro, ou
R³ est -(CH₂)_{q}R⁹, R⁴ est un groupe hydro et R⁵ est -NHR¹⁰, ou
R³ est -NHR¹⁰ et R⁴ et R⁵ sont chacun un groupe hydro, ou
R³ est un groupe hydro ou -(CH₂)_{q}R⁹, R⁴ est un groupe alkyle en C₁-C₄, di(C₁-C₄)alkylaminométhyle, pipéridin-1-ylméthyle, morpholin-4-ylméthyle, formyle, 1-hydroxyalkyle en C₁-C₄ ou -CH₂NHR¹³ et R⁵ est un groupe hydro, ou
R³ est un groupe hydro ou -(CH₂)_{q}R⁹, R⁴ est un groupe hydro, alkyle en C₁-C₄ ou -C(O)R¹⁴ et R⁵ est un groupe cyano, hydroxyméthyle, 1H-tétrazol-5-yle, 4,5-dihydroimidazol-2-yle, pyrrolidin-1-ylméthyle, pipéridin-1-ylméthyle, morpholin-4-ylméthyle, pipérazin-1-yl-méthyle, 4-(C₁-C₄)alkylpipérazin-1-ylméthyle, -C(O)R¹⁴, -C(NH)NR¹⁵R¹⁶ ou -CH₂NR¹⁰R¹⁷, ou
R³ est un groupe hydro ou -(CH₂)_{q}R⁹ et R⁴ et R⁵ sont d'une manière dépendante du di(C₁-C₄)alkylaminométhyle, du pipéridin-1-ylméthyle, du morpholin-4-ylméthyle ou de l'hydroxyméthyle,
R⁶ est un groupe de formule (d): 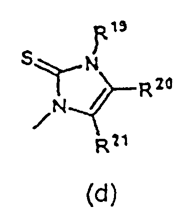 où
R¹⁹ est un groupe hydro ou -(CH₂)_{q}R⁹, R²⁰ est un groupe hydro et R²¹ est -NR²⁵R²⁶, ou
R¹⁹ est -NR²⁵R²⁶, R²⁰ et R²¹ sont chacun un groupe hydro, ou
R¹⁹ est un groupe hydro ou -(CH₂)_{q}R⁹, R²⁰ est -CH₂NR²⁵R²⁶ et R²¹ est un groupe hydro, ou
R¹⁹ est un groupe hydro ou -(CH₂)_{q}R⁹, R²⁰ est un groupe hydro, alkyle en C₁-C₄ ou -C(O)R¹⁴ et R²¹ est -CH₂NR²⁵R²⁶,
R⁷ est un groupe de formule (g) : 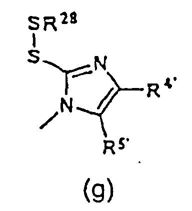 où
R^{4'} est un groupe hydro et R^{5'} est -NHR¹⁰, ou
R^{4'} est un groupe alkyle en C₁-C₄, di(C₁-C₄)alkylaminométhyle, pipéridin-1-ylméthyle, morpholin-4-ylméthyle 1-hydroxyalkyle en C₁-C₄ ou -CH₂NHR¹³ et R^{5'} est un groupe hydro, ou
R^{4'} est un groupe hydro, alkyle en C₁-C₄ ou -C(O)R¹⁴ et R^{5'} est un groupe hydroxyméthyle, 1H-tétrazol-5-yle, 4,5-dihydroimidazol-2-yle, pyrrolidin-1-ylméthyle, pipéridin-1-ylméthyle, morpholin-4-ylméthyle, pipérazin-1-ylméthyle, 4-(C₁-C₄)alkylpipérazin-1-ylméthyle, -C(O)R¹⁴, -C(NH)NR¹⁵R¹⁶ ou -CH₂NR¹⁰R¹⁷, ou
R^{4'} et R^{5'} sont de manière dépendante du di(C₁-C₄)alkylaminométhyle, du pipéridin-1-ylméthyle, du morpholin-4-ylméthyle ou de l'hydroxyméthyle, et
R²⁸ est un groupe alkyle en C₂-C₆ (cet alkyle étant en outre substitué par un ou deux substituants indépendamment choisis parmi -N(R²⁹)₂, -C(O)OR³⁰, -PO(OR³⁰)₂, -SO₃R³⁰, -SO₂NHR³⁰ et -OR³⁰),
q vaut 0, 1, 2, 3 ou 4,
R⁹ est un groupe carboxy, (C₁-C₄)alkyloxycarbonyle, carbamoyle ou un groupe choisi parmi de l'aryle et de l'hétéroaryle [ce groupe étant éventuellement en outre substitué par un à deux substituants indépendamment choisis parmi de l'hydroxy, de l'alkyloxy en C₁-C₄, du cyano, du 1H-tétrazol-5-yle, du carboxy et du (C₁-C₄)alkyloxycarbonyle],
R¹⁰ est un groupe hydro, alcanoyle en C₁-C₄, trifluoroalcanoyle en C₁-C₄, carbamoyle, (C₁-C₄)alkyloxycarbonyle, (C₁-C₄)-alkylcarbamoyle, di(C₁-C₄)alkylcarbamoyle, aminoalcanoyle en C₁-C₄, (C₁-C₄)alkylamino(C₁-C₄)alcanoyle, di(C₁-C₄)alkylamino(C₁-C₄)alcanoyle, un groupe choisi parmi de l'aroyle et de l'hétéroaroyle [ces aroyle et hétéroaroyle étant éventuellement en outre substitués par un à deux substituants indépendamment choisis parmi les groupes hydroxy, (C₁-C₄)alkyloxy, cyano, 1H-tétrazol-5-yle, carboxy et (C₁-C₄)alkyloxycarbonyle] ou -C(NR¹¹)NHR¹²,
R¹¹ et R¹² sont indépendamment des groupes hydro, acétyle ou tert-butoxycarbonyle,
R¹³ est un groupe hydro, alkyle en C₁-C₄, alcanoyle en C₁-C₄, trifluoroalcanoyle en C₁-C₄, carbamoyle, (C₁-C₄)alkyloxycarbonyle, (C₁-C₄)alkylcarbamoyle, di(C₁-C₄)alkylcarbamoyle, amino(C₁-C₄)alcanoyle, (C₁-C₄)alkylamino(C₁-C₄)alcanoyle, di(C₁-C₄)alkylamino(C₁-C₄)-alcanoyle, carboxy(C₁-C₄)alkyle, (C₁-C₄)alkyloxycarbonyl(C₁-C₄)alkyle, carbamoyl(C₁-C₄)alkyle, un groupe choisi parmi de l'aroyle, du hétéroaroyle, de l'aryl(C₁-C₄)alkyle et de l'hétéroaryl(C₁-C₄)alkyle [ces aroyle, hétéroaryle, aryle et hétéroaryle étant éventuellement en outre substitués par un à deux substituants indépendamment choisis parmi de l'hydroxy, de l'alkyloxy en C₁-C₄, du cyano, du 1H-tétrazol-5-yle, du carboxy et du (C₁-C₄)alkyloxycarbonyle] ou du -C(NR¹¹)NHR¹²);
R¹⁴ est un groupe amino, hydroxy, (C₁-C₄)alkyloxy, 2-(diméthylamino)éthylamino, 4-méthylpipérazin-1-yle, 2-(diméthylamino)-éthylmercapto, 4-(méthylsulfonylamino)anilino ou 1H-tétrazol-5-ylamino,
R¹⁵ et R¹⁶ sont indépendamment un groupe hydro, alkyle en C₁-C₄ ou trifluoroalkyle en C₁-C₄,
R¹⁷ est un groupe hydro ou alkyle en C₁-C₄,
R²⁵ est un groupe hydro ou alkyle en C₁-C₄,
R²⁶ est un groupe L-alanyle, L-arginyle, L-asparaginyle, L-α-aspartyle, L-β-aspartyle, L-cystéinyle, L-glutaminyle, L-α-glutamyle, L-γ-glutamyle, N-(C₁-C₄)alcanoyl-L-α-glutamyle, N-(C₁-C₄)alcanoyl-L-γ-glutamyle, glycyle, L-histidyle, L-isoleucyle, L-leucyle, L-lysyle, L-méthionyle, L-ornithinyle, L-phénylalanyle, L-prolyle, L-séryle, L-thréonyle, L-tryptophyle, L-tyrosyle, L-valyle, 1-aminocyclopropylcarbonyle, 1-aminocyclobutylcarbonyle, 1-aminocyclopentylcarbonyle ou 1-aminocyclohexylcarbonyle,
R²⁹ est indépendamment un groupe hydro, acétyle ou trifluoroacétyle,
R³⁰ est indépendamment hydro ou alkyle en C₁-C₅,
aryle signifiant un radical organique dérivé d'un hydrocarbure aromatique contenant 6 à 14 atomes de carbone et incluant des noyaux aromatiques monocycliques ou carbocycliques condensés, le radical aryle étant éventuellement en outre substitué par un à deux substituants indépendamment choisis parmi les groupes halo et cyano,
aroyle signifiant le radical -C(O)R, où R est un aryle comme défini ci-dessus,
hétéroaryle signifiant un radical organique dérivé d'un hydrocarbure aromatique contenant 5 à 14 atomes, dont 1 à 5 sont des hétéroatomes choisis parmi N, O ou S, et incluant des noyaux aromatiques monocycliques, hétérocycliques condensés et carbocycliques et hétérocycliques condensés, le radical hétéroaryle étant en outre éventuellement substitué par un à deux substituants indépendamment choisis parmi les groupes halo et cyano,
hétéroaryle signifiant le radical -C(O)R, où R est un hétéroaryle tel que défini ci-dessus,
et les sels, isomères individuels et mélanges d'isomères pharmaceutiquement acceptables de ces composés.

2. Composés de la formule I suivant la revendication 1, dans lesquels R⁵ est un groupe di(C₁-C₄)alkylaminométhyle, pyrrolidin-1-ylméthyle, pipéridin-1-ylméthyle, morpholin-4-ylméthyle, pipérazin-1-ylméthyle, 4-(C₁-C₄)alkylpipérazin-1-ylméthyle ou -CH₂NHR¹⁰.

3. Composés suivant la revendication 2, dans lesquels R⁵ est un groupe uréidométhyle, aminométhyle ou acétylaminométhyle.

4. Composés suivant la revendication 3, dans lesquels t vaut 2 et chaque R¹ est un fluoro.

5. Composés suivant la revendication 4, dans lesquels R¹ est du fluoro aux positions 5 et 7.

6. Composé de la formule I suivant la revendication 1, choisi parmi le groupe constitué de la 5-aminométhyl-1-(5,7-difluoro-1,2,3,4-tétrahydronaphtalén-2-yl)-1,3-dihydroimidazole-2-thione ou d'un sel pharmaceutiquement acceptable de celle-ci, du chlorhydrate de (S)-5-aminométhyl-1-(5,7-difluoro-1,2,3,4-tétrahydronaphtalén-2-yl)-1,3-dihydroimidazole-2-thione, de la 5-uréidométhyl-1-(5,7-difluoro-1,2,3,4-tétrahydronaphtalén-2-yl)-1,3-dihydroimidazole-2-thione, de la (S)-5-uréido-méthyl-1-(5,7-difluoro-1,2,3,4-tétrahydronaphtalén-2-yl)-1,3-dihydroimidazole-2-thione, du N-[3-(5,7-difluoro-1,2,3,4-tétrahydronaphtalén-2-yl)-2-thioxo-2,3-dihydro-1H-imidazol-4-ylméthyl]acétamide, du (S)-N-[3-(5,7-difluoro-1,2,3,4-tétrahydronaphtalén-2-yl)-2-thioxo-2,3-dihydro-1H-imidazol-4-yiméthyi]acétamide.

7. Composés de la formule II suivant la revendication 1, dans lesquels t vaut 2, chaque R¹ est du fluoro et R²¹ est -CH₂NR²⁶.

8. Composés suivant la revendication 7, dans lesquels R¹ est du fluoro aux positions 5 et 7 et R²⁶ est un groupe L-arginyle, L-α-aspartyle, L-β-aspartyle, L-histidyle ou L-ornithyle.

9. Composé de la formule II suivant la revendication 1, choisi parmi le groupe constitué de l'acide 3S-amino-N-[3-(5,7-difluoro-1,2,3,4-tétrahydronaphtalén-2-yl)-2-thioxo-2,3-dihydro-1H-imidazol-4-ylméthyl]succinamique et de ses sels pharmaceutiquement acceptables, du chlorhydrate d'acide 3S-amino-N-[3-(5,7-difluoro-1,2,3,4-tétrahydronaphtalén-2S-yl)-2-thioxo-2,3-dihydro-1H-imidazol-4-yl-méthyl)succinamique, du 2S-amino-3-(3H-imidazol-4-yl)-N-[3-(1,2,3,4-tétrahydronaphtalén-2-yl)-2-thioxo-2,3-dihydro-1H-imidazol-1-yl]propionamide et de ses sels pharmaceutiquement acceptables, du dichlorhydrate de 2S-amino-3-(3H-imidazol-4-yl)-N-[3-(1,2,3,4-tétrahydronaphtalén-2S-yl)-2-thioxo-2,3-dihydro-1H-imidazol-1-yl]propionamide, du 2S,5-diamino-N-[3-(5,7-difluoro-1,2,3,4-tétrahydronaphtalén-2-yl)-2-thioxo-2,3-dihydro-1H-imidazol-4-ylméthyl]valéramide et de ses sels pharmaceutiquement acceptables, et du dichlorhydrate de 2S,5-diamino-N-[3-(5,7-difluoro-1,2,3,4-tétrahydronaphtalén-2S-yl)-2-thioxo-2,3-dihydro-1H-imidazol-4-ylméthyl]-valéramide.

10. Composés de la formule III suivant la revendication 1, dans lesquels t vaut 2 et chaque R¹ est du fluoro.

11. Composés suivant la revendication 10, dans lesquels R¹ est du fluoro aux positions 5 et 7.

12. Composés suivant la revendication 11, dans lesquels R²⁸ est un groupe choisi parmi de l'éthyle, du 1,1-diméthyléthyle et du propyle (ce groupe étant en outre substitué par un à deux substituants indépendamment choisis parmi du carboxy, du méthoxycarbonyle, de l'amino et du trifluoroacétylamino).

13. Composés suivant la revendication 12, dans lesquels R²⁸ est un groupe (R)-2-amino-2-méthoxycarbonyléthyle, (R)-2-amino-2-carboxyéthyle, (R)-2-trifluoroacétylamino-2-méthoxycarbonyléthyle, 2-aminoéthyle, (S)-2-amino-2-carboxy-1,1-diméthyléthyle ou 3-amino-3-carboxyprop-1-yle.

14. Composition pharmaceutique comprenant un composé suivant l'une quelconque des revendications 1 à 13.

15. Composé suivant l'une quelconque des revendications 1 à 13, en tant que substance thérapeutique.

16. Utilisation d'un composé suivant l'une quelconque des revendications 1 à 13, pour la préparation d'un médicament pour le traitement d'un état qui peut être amélioré par un médicament qui inhibe la dopamine β-hydroxylase chez un animal, à savoir pour le traitement de la maladie de Parkinson, de l'hypertension ou d'une défaillance du coeur par congestion.

17. (S)-5,7-difluoro-1,2,3,4-tétrahydronaphtalén-2-ylamine :

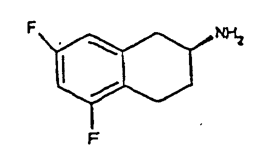

18. Procédé de préparation de (S)-5,7-difluoro-1,2,3,4-tétrahydronaphtalén-2-ylamine : 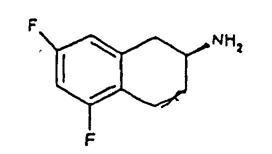 ce procédé comprenant :
(a) une réduction, en présence de 2S-diméthylamino-1R-phénylpropanol et de 2-éthylaminopyridine, du composé de la formule : 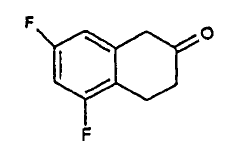 ce qui donne le (R)-énantiomère de la formule : 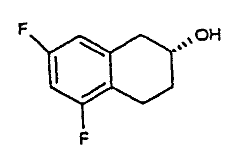
(b) un traitement du (R)-énantiomère par du chlorure de méthanesulfonyle et ensuite une réaction avec un sel azide pour donner le (S)-énantiomère de formule : 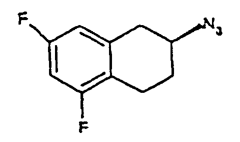 et
(c) une réduction.
